(19) Europäisches Patentamt
European Patent Office
Office européen des brevets

(11) EP 4 357 437 A1

(12) **EUROPEAN PATENT APPLICATION**
published in accordance with Art. 153(4) EPC

(43) Date of publication:
**24.04.2024 Bulletin 2024/17**

(21) Application number: **22825031.2**

(22) Date of filing: **15.06.2022**

(51) International Patent Classification (IPC):
***C09K 11/06** (2006.01)* ***C09B 23/01** (2006.01)*
***C09B 23/14** (2006.01)* ***G01N 33/533** (2006.01)*
***G01N 33/58** (2006.01)* ***G01N 21/64** (2006.01)*

(52) Cooperative Patent Classification (CPC):
**C09K 11/06; C09B 23/0066; C09B 23/086; C09B 23/107; G01N 33/533; G01N 33/582**

(86) International application number:
**PCT/JP2022/023976**

(87) International publication number:
**WO 2022/265043 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2021 JP 2021101305**
**18.03.2022 JP 2022044427**

(71) Applicant: **FUJIFILM Corporation**
**Tokyo 106-8620 (JP)**

(72) Inventors:
- **KANAZAWA, Yoshinori**
**Ashigarakami-gun, Kanagawa 258-8577 (JP)**
- **WATANABE, Kousuke**
**Ashigarakami-gun, Kanagawa 258-8577 (JP)**
- **TANAKA, Hiroaki**
**Ashigarakami-gun, Kanagawa 258-8577 (JP)**
- **NAKATA, Hiyoku**
**Ashigarakami-gun, Kanagawa 258-8577 (JP)**
- **HAMADA, Naoka**
**Ashigarakami-gun, Kanagawa 258-8577 (JP)**
- **KOMIYAMA, Kazuoki**
**Ashigarakami-gun, Kanagawa 258-8577 (JP)**
- **KAWAI, Kazuki**
**Ashigarakami-gun, Kanagawa 258-8577 (JP)**

(74) Representative: **HGF**
**HGF Limited**
**1 City Walk**
**Leeds LS11 9DX (GB)**

# (54) COMPOUND AND LABELED BIOMATERIAL USING SAME

(57) There are provided a compound of Formula (1) and a labeled biological substance having the compound.

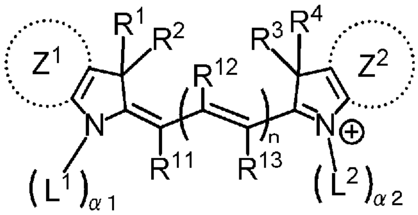

General Formula (1)

A ring $Z^1$ and a ring $Z^2$ represent a 6-membered ring formed of a ring-constituting atom selected from a carbon atom and a nitrogen atom.

$R^1$ to $R^4$, $R^{11}$ to $R^{13}$, $L^1$, and $L^2$ represents specific groups, and n, $\alpha 1$, and $\alpha 2$ represent specific numbers.

At least one of $R^{11}$, $R^{12}$, or $R^{13}$ contains a carboxy group or a substituent capable of being bonded to a biological substance. the compound represented by Formula (1) has at least one structure represented by $-(CH_2-CH_2-O)_m-R^{21}$, where $R^{21}$ represents a specific group, and m represents a specific number.

The compound represented by Formula (1) is a neutral compound.

EP 4 357 437 A1

## Description

## BACKGROUND OF THE INVENTION

1. Field of the Invention

The present invention relates to a compound and a labeled biological substance using the compound.

2. Description of the Related Art

In order to observe in vivo changes in response to various stimuli (diseases, environmental changes, and the like), fluorescently labeled biological substances obtained by labeling a biological molecule (an antibody or the like) having a binding property to a target substance to be detected, with a fluorescent compound (a dye), are often used.

For example, also in Western blotting (hereinafter, also abbreviated as WB) that detects a specific protein from a protein mixture, a fluorescence method in which the presence or absence or the abundance of the specific protein is detected using a fluorescently labeled antibody having a binding property to this protein is used.

In addition, in bioimaging technology for analyzing the dynamics and functions of biological molecules, cells, tissues, and the like in a living body, in vivo fluorescence imaging in which a specific portion of a living body visualized by fluorescence labeling is observed is used as one of the techniques for the living body observation.

As a fluorescent dye that is used for fluorescence labeling, a cyanine dye is known, for example, as described in US2021/0093737A. However, in a case where a cyanine dye is used for fluorescence labeling, interactions such as self-association between the dyes after labeling easily occur, and the fluorescence quantum yield tends to decrease.

As a technique for coping with this problem, for example, WO2009/078970A describes a cyanine dye into which a water-soluble polyethylene glycol (PEG) group has been introduced. According to WO2009/078970A, the cyanine dyes described in the US2021/0093737A and WO2009/078970A are said to exhibit a high fluorescence intensity as compared with the cyanine dyes in the related art by suppressing self- association between the dyes after labeling, due to the PEG group contained in the dyes.

## SUMMARY OF THE INVENTION

However, from the studies by the inventors of the present invention, it was found that the fluorescence intensity obtained by the fluorescence labeling using the cyanine dye described in US2021/0093737A is low, and thus a sufficient fluorescence intensity cannot be obtained. In addition, it was found that in the fluorescent labeling using the cyanine dye described in WO2009/078970A, the binding property of the cyanine dye to a biological molecule such as an antibody is low in the first place, the fluorescence intensity itself is also low, and thus a sufficient fluorescence intensity cannot be obtained.

An object of the present invention is to provide a compound that exhibits an excellent fluorescence intensity of a labeled biological substance to be obtained. In addition, another object of the present invention is to provide a labeled biological substance obtained by bonding the compound to a biological substance.

That is, the above objects of the present invention have been achieved by the following means.

[1] A compound represented by General Formula (1),

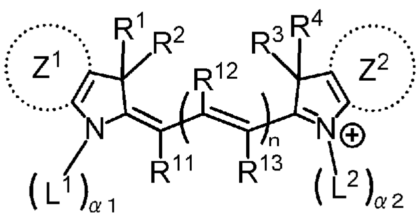

General Formula (1)

in the formula, $R^1$ to $R^4$ represent an alkyl group, an aryl group, a heteroaryl group, or $-(CH_2-CH_2-O)_m-R^{21}$, where m is 1 to 10, and $R^{21}$ represents an alkyl group,

$R^{11}$ to $R^{13}$ represent a hydrogen atom, an alkyl group, an aryl group, a heteroaryl group, an alkoxy group, an aryloxy group, an alkylthio group, an arylthio group, an amino group, an amide group, or a halogen atom, where adjacent groups may be bonded to each other to form a 5- or 6-membered ring, provided that at least one of $R^{11}$, $R^{12}$, or $R^{13}$ contains a carboxy group or a substituent capable of being bonded to a biological substance,

n is an integer of 1 to 3,

$L^1$ and $L^2$ represent an alkyl group or $-(CH_2-CH_2-O)_m-R^{21}$, where $R^{21}$ and m respectively have the same meanings as $R^{21}$ and m described above, and $L^1$ and $L^2$ may be bonded to each other to form a ring,
$\alpha 1$ and $\alpha 2$ are 0 or 1,
a ring $Z^1$ and a ring $Z^2$ represent a 6-membered ring formed of a ring-constituting atom selected from a carbon atom and a nitrogen atom, may have a substituent, and may form a fused ring,
in a case where the ring $Z^1$ and the ring $Z^2$ form a 6-membered ring having a ring-constituting nitrogen atom, the ring-constituting nitrogen atom may be substituted with a substituent, and
the compound represented by Formula (1) has at least one structure represented by $-(CH_2-CH_2-O)_m-R^{21}$, where $R^{21}$ and m respectively have the same meanings as $R^{21}$ and m described above, and
provided that the compound represented by Formula (1) is a neutral compound.

[2] The compound according to [1], in which the compound is represented by any one of General Formulae (2-1) to (2-4),

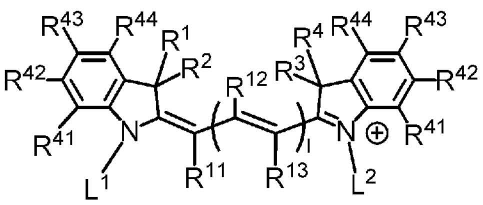
General Formula (2-1)

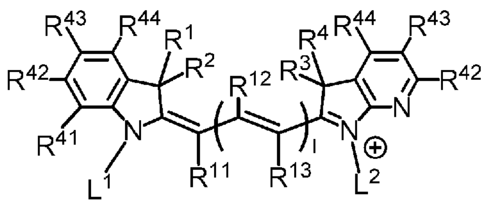
General Formula (2-2)

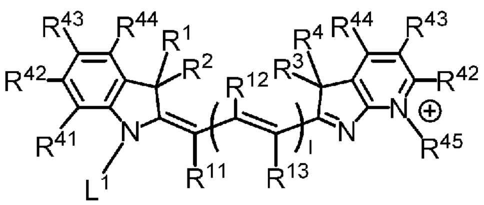
General Formula (2-3)

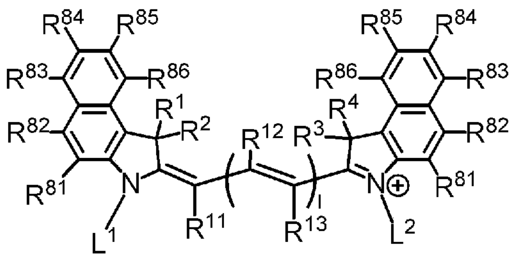
General Formula (2-4)

in the formulae, $R^{45}$ represent an alkyl group or $-(CH_2-CH_2-O)_m-R^{21}$,
$R^{41}$ to $R^{44}$ and $R^{81}$ to $R^{86}$ represent a hydrogen atom, an alkyl group, an alkoxy group, an aryl group, a sulfo group, a sulfonamide group, a nitro group, a carboxy group, an amide group, or a halogen atom, where adjacent groups may be bonded to each other to form a fused ring,
$R^1$ to $R^4$, $R^{11}$ to $R^{13}$, $L^1$, $L^2$, and m respectively have the same meanings as $R^1$ to $R^4$, $R^{11}$ to $R^{13}$, $L^1$, $L^2$, and m described above,
at least one of $R^1$ to $R^4$, $L^1$, $L^2$, or $R^{45}$ is $-(CH_2-CH_2-O)_m-R^{21}$, where $R^{21}$ and m respectively have the same meanings as $R^{21}$ and m described above, and
l represents an integer of 2 or 3,
provided that the compound represented by any one of Formulae (2-1) to (2-4) is a neutral compound.

[3] The compound according to [1] or [2],
in which at least one of $R^1$ or $R^2$ described above and at least one of $R^3$ or $R^4$ described above includes a structure represented by $-(CH_2-CH_2-O)_m-$, where m is 1 to 10.
[4] The compound according to [3], in which the compound includes four structures, each of which is represented by $-(CH_2-CH_2-O)_m-$, where m is 1 to 10.
[5] The compound according to any one of [1] to [4], in which at least one of $L^1$ or $L^2$ described above is an alkyl group having, as a substituent, at least one of an alkoxy group, a carboxy group, a sulfo group, or a phosphono group.
[6] The compound according to [5], in which $L^1$ and $L^2$ described above are an alkyl group having, as a substituent, at least one of an alkoxy group, a carboxy group, a sulfo group, or a phosphono group.
[7] The compound according to any one of [1] to [6], in which one among $R^{11}$ to $R^{13}$ described above, which has a substituent having a carboxy group or a substituent capable of being bonded to a biological substance, is a substituent represented by any one of General Formulae (I) to (V),

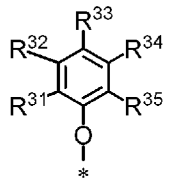 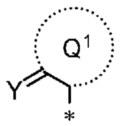 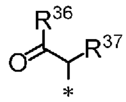 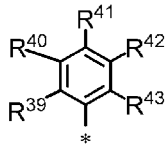 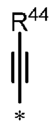

General Formula (I) General Formula (II) General Formula (III) General Formula (IV) General Formula (V)

in the formulae, Y represents an oxygen atom or $=NR^{38}$, and a ring $Q^1$ represents a heterocyclic ring or hydrocarbon ring which is 5-membered or 6-membered,

$R^{31}$ to $R^{44}$ represent a hydrogen atom, an alkyl group, an aryl group, a heteroaryl group, an alkoxy group, an aryloxy group, an alkylthio group, an arylthio group, an amino group, an amide group, a halogen atom, a sulfo group, a nitro group, a cyano group, a carboxy group, or a substituent capable of being bonded to a biological substance,

provided that a group represented by any one of General Formulae (I) to (V) has at least one carboxy group or one substituent capable of being bonded to a biological substance, and

* represents a bonding site.

[8] The compound according to [7], in which the substituent represented by General Formula (II) is a substituent represented by any one of Formulae (E-1) to (E-8),

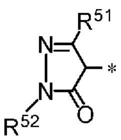 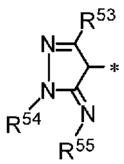 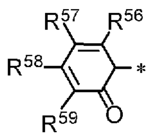 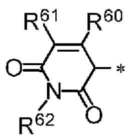 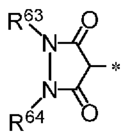 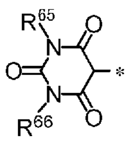 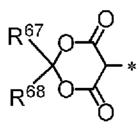 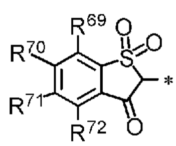

(E-1) (E-2) (E-3) (E-4) (E-5) (E-6) (E-7) (E-8)

in the formulae, $R^{51}$ to $R^{72}$ represent a hydrogen atom, an alkyl group, an aryl group, a heteroaryl group, an alkoxy group, an aryloxy group, an alkylthio group, an arylthio group, an amino group, a halogen atom, a sulfo group, a nitro group, a cyano group, a carboxy group, or a substituent capable of being bonded to a biological substance,

provided that a substituent represented by any one of General Formulae (E-1) to (E-8) has at least one carboxy group or one substituent capable of being bonded to a biological substance, and

* represents a bonding site.

[9] The compound according to [7],
in which one among $R^{11}$ to $R^{13}$ described above, which has a substituent having a carboxy group or a substituent capable of being bonded to a biological substance, is a substituent represented by General Formula (IV).

[10] The compound according to [9], in which at least one of $R^{39}$, ..., or $R^{43}$ described above is a carboxy group, an alkyloxy group having a carboxy group, an alkylcarbamoyl group having a carboxy group, or a poly(oxyalkylene) carbamoyl group having a carboxy group.

[11] A labeled biological substance that is obtained by bonding the compound according to any one of [1] to [10] to a biological substance.

[12] The labeled biological substance according to [11], in which the biological substance is any one of a protein, an amino acid, a nucleic acid, a sugar chain, or a phospholipid.

The compound according to the aspect of the present invention makes it possible to obtain a labeled biological substance that exhibits an excellent fluorescence intensity. In addition, the labeled biological substance according to the aspect of the present invention exhibits an excellent fluorescence intensity.

## DESCRIPTION OF THE PREFERRED EMBODIMENTS

In the present invention, in a case of a plurality of substituents, linking groups, and the like (hereinafter, referred to as a substituent and the like) represented by a specific reference or formula, or in a case of simultaneously defining a plurality of the substituent and the like, unless otherwise specified, the substituent and the like may be the same or

different from each other (regardless of the presence or absence of an expression "each independently", the substituent and the like may be the same or different from each other). The same also applies to the regulation of the number of substituents or the like. In addition, in a case where a plurality of substituents and the like are close to each other (particularly in a case where the substituents and the like are adjacent to each other) unless otherwise specified, the substituents and the like may be linked to each other to form a ring, where the ring to be formed may have an unsaturated bond or may not have an unsaturated bond. In addition, unless otherwise specified, rings such as an alicyclic ring, an aromatic ring, and a heterocyclic ring may be fused to form a fused ring.

In the present specification, in a case where the E type and the Z type of the double bond are present in the molecule, any one of the E type or the Z type, or a mixture thereof may be used unless otherwise specified. In addition, in a case where a compound has diastereomers and enantiomers, any one of the diastereomers or the enantiomers may be used, or a mixture thereof may be used unless otherwise specified.

In the present invention, the denotation of a compound or substituent is meant to include not only the compound itself but also a salt thereof, and an ion thereof. For example, the carboxy group, the sulfo group, and the phosphono group ($-P(=O)(OH)_2$) may have an ionic structure by a hydrogen atom being dissociated therefrom, or they may have a salt structure. That is, in the present invention, the "carboxy group" is meant to include an ion or salt of a carboxylic acid, the "sulfo group" is meant to include an ion or salt of a sulfonic acid, and the "phosphono group" is meant to include an ion or salt of a phosphonic acid. The monovalent or polyvalent cation in forming the salt structure is not particularly limited. Examples thereof include an inorganic cation and an organic cation, and specific examples thereof include alkali metal cations such as $Na^+$, $Li^+$, and $K^+$, alkaline earth metal cations such as $Mg^{2+}$, $Ca^{2+}$, and $Ba^{2+}$, and organic ammonium cations such as a trialkylammonium cation and a tetraalkylammonium cation.

In a case of a salt structure, the kind of the salt may be one kind, two or more kinds thereof may be mixed, a salt-type group and a group having a free acid structure may be mixed in a compound, and a compound having a salt structure and a compound having a free acid structure compound may be mixed.

Any compound according to the embodiment of the present invention is a neutral compound. In the present invention, the fact that the compound is neutral means that the compound is electrically neutral. Specifically, the charge of the compound as a whole is adjusted to be 0 by a group having a charge or by a counterion in the compound. For example, in the compound represented by General Formula (1), the formal charge of the nitrogen atom to which $L^2$ is bonded is +1 except for a case of $\alpha 2 = 0$. In order to be paired with this formal charge, a dissociable group such as a sulfo group in the compound has an ionic structure such as a sulfonate ion, and thus the compound according to the embodiment of the present invention is a compound having a charge of 0 as a whole. In a case of $\alpha 2 = 0$, the formal charge of the nitrogen atom to which $L^2$ is bonded is 0.

It is noted that in the compound represented by General Formula (1), the formal charge of the nitrogen atom to which $L^1$ is bonded is 0 in a case of $\alpha 1 = 1$. In a case of $\alpha 1 = 0$, a conjugated structure, in which the bonding site between the nitrogen atom to which $L^1$ is bonded and the ring $Z^1$ becomes a double bond instead of the double bond of the fused portion between the 5-membered ring containing the nitrogen atom to which $L^1$ is bonded in General Formula (1) and the ring $Z^1$, is adopted so that the formal charge of the nitrogen atom to which the $L^1$ is bonded is 0.

In each general formula defined in the present invention, the positive charge possessed by the compound is specified and indicated, for convenience, as a structure of a specific nitrogen atom. However, since the compound according to the embodiment of the present invention has a conjugated system, another atom other than the nitrogen atom actually may be capable of being positively charged, and thus any compound capable of adopting a structure represented by each general formula as one of the chemical structures is included in the compound represented by each general formula. This also applies to the negative charge.

In addition, it is meant to include those in which a part of the structure is changed within the scope that does not impair the effect of the present invention. Further, it is meant that a compound, which is not specified to be substituted or unsubstituted, may have any substituent within the scope that does not impair the effect of the present invention. The same also applies to a substituent (for example, a group denoted as "alkyl group", "methyl group", or "methyl") and a linking group (for example, a group denoted as "alkylene group", "methylene group", or "methylene") and a ring (for example, a ring denoted as "heterocyclic ring" or "hydrocarbon ring"). Among such substituents, a preferred substituent in the present invention is a substituent selected from a substituent group T described later.

In the present invention, in a case where the number of carbon atoms of a certain group is specified, this number of carbon atoms means the number of carbon atoms of the entire group thereof unless otherwise specified in the present invention or the present specification. That is, in a case where this group has a form of further having a substituent, it means the total number of carbon atoms, to which the number of carbon atoms of this substituent is included.

In addition, in the present invention, the numerical range represented by using "to" means a range including the numerical values before and after "to" as the lower limit value and the upper limit value, respectively.

The compound according to the embodiment of the present invention is represented by General Formula (1). Although the details of the reason why the compound according to the embodiment of the present invention makes it possible to obtain a labeled biological substance that exhibits an excellent fluorescence intensity are not clear, it is

conceived as follows.

As represented by General Formula (1), the compound according to the embodiment of the present invention has a polymethine chain having a heterocyclic ring which contains a nitrogen atom as a ring-constituting atom and in which a ring $Z^1$ or a ring $Z^2$ is fused at both terminals (in the present invention, the polymethine chain means a hydrocarbon chain having a plurality of double bonds in the chain, which is an entirely conjugated chain, where the hydrocarbon chain has substituents $R^{11}$ to $R^{13}$; and it is noted that the number of carbon atoms constituting this hydrocarbon chain is 2n + 3, and the polymethine chain consists of carbon atoms constituting a hydrocarbon chain enclosed in $( )_n$, a carbon atom substituted with $R^{11}$, and ring-constituting carbon atoms constituting a heterocyclic ring located on both sides of these carbon atoms, and the same applies hereinafter), and furthermore, the nitrogen atom of the heterocyclic ring in which the ring Z1 is fused has a tertiary amine structure, and the nitrogen atom of the heterocyclic ring in which the ring $Z^2$ is fused has a quaternary ammonium structure, which causes absorption due to charge movement through the polymethine skeleton. The compound according to the embodiment of the present invention as described above is classified into a compound referred to as a polymethine dye (broadly, a cyanine dye).

In addition to having the above-described structure, the compound according to the embodiment of the present invention further has at least one structure in which at least one of $R^{11}$, $R^{12}$, or $R^{13}$ which is a substituent on the methine chain having a repetition number of 2n + 3 contains a carboxy group or a substituent capable of being bonded to a biological substance, and which includes, in the compound, a polyethylene glycol (PEG) group represented by-$(CH_2-CH_2-O)_m-R^{21}$, where m is 1 to 10. Due to having a carboxy group or a substituent capable of being bonded to a biological substance at at least any position of $R^{11}$, $R^{12}$, or $R^{13}$ in the compound as described above, It is conceived that it is possible to make the compound have a chemical structure that is more likely to exhibit the excluded volume effect due to the PEG group represented by $-(CH_2-CH_2-O)_m-R^{21}$, and as a result of effectively suppressing the interaction between the compounds, it is possible to suppress a decrease in the fluorescence intensity due to the self-association of the compound. In addition, in a case where the repetition number m of the PEG group is set to 10 or less, the decrease in the binding property to a biological substance due to the large excluded volume effect, which occurs in a case of a PEG group having a m of more than 10, is suppressed, and it is possible to exhibit a binding property to a biological substance at a level of no problem in practical use.

Depending on the length of the methine chain having a repetition number of 2n + 3, the compounds according to the embodiment of the present invention respectively have an excitation absorption wavelength in a wavelength range of 520 to 600 nm (in the vicinity of 585 nm) in a case of n = 1, in a wavelength range of 620 to 700 nm (in the vicinity of 685 nm) in a case of n = 2, and in a wavelength range of 740 to 830 nm (in the vicinity of 785 nm) in a case of n = 3. As a result, each of the compounds represented by General Formula (1) can be used as a compound from which a labeled biological substance exhibiting an excellent fluorescence intensity is obtained, in the fluorescence labeling in which a light source having any wavelength in a wavelength range of about 500 to 800 nm (for example, in the vicinity of 600 nm, in the vicinity of 700 nm, or in the vicinity of 800 nm) matching with the absorption excitation wavelength of the compound is used as an excitation light source.

In multicolor WB, a plurality of luminescence colors are detected in the range from the visible range to the near infrared range. As a result, it is necessary to select wavelengths so that the absorption and luminescence waveforms of a plurality of dyes have a suitable wavelength relationship so that crosstalk does not occur due to mutual interference in a case where the dyes are excited to emit light. Ideally, it should be adjusted so that only one dye emits light at one excitation light and the other dyes do not emit light. From this point of view, two kinds of excitation light sources having wavelengths separated to some extent, for example, in the vicinity of 700 nm and in the vicinity of 800 nm, are used for luminescence in the near infrared range of the multicolor WB.

As compared with the detection by visible light excitation, the fluorescence detection by near-infrared light excitation can suppress the autofluorescence of the membrane, that is, the background fluorescence, and thus it is easy to increase the signal to noise ratio (the S/N ratio) and it is possible to detect a target protein with high sensitivity. As a result, in recent years, there has been an increasing need for fluorescence detection WB using luminescence in the near infrared range in the analytical research on the trace amount of proteins.

However, in the near infrared range, the fluorescence quantum yield of the fluorescent dye is generally low, and thus it is difficult to obtain a high signal amount. Among the compounds according to the embodiment of the present invention, the compound in which n = 2 or 3 can be used as a compound from which a labeled biological substance exhibiting an excellent fluorescence intensity can be obtained, even in the multicolor WB having the above-described two kinds of excitation light sources in the vicinity of 700 nm and in the vicinity of 800 nm, and in particular, it can exhibit an excellent fluorescence intensity even with respect to a request for observing and detecting proteins with higher sensitivity, as compared with the fluorescence labeling using cyanine dyes in the related art including the cyanine dyes described in US2021/0093737A and WO2009/078970A.

Hereinafter, the compound according to the embodiment of the present invention, which is represented by General Formula (1), will be described in detail.

<Compound represented by General Formula (1)>

The compound according to the embodiment of the present invention, which is represented by General Formula (1), is as follows.

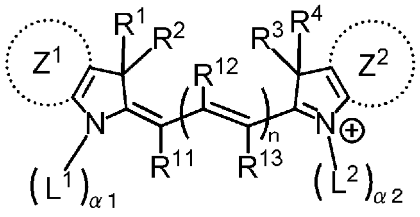

General Formula (1)

In the formula, $R^1$ to $R^4$ represent an alkyl group, an aryl group, a heteroaryl group, or $-(CH_2-CH_2-O)_m-R^{21}$. Here, m is 1 to 10, and $R^{21}$ represents an alkyl group.

$R^{11}$ to $R^{13}$ represent a hydrogen atom, an alkyl group, an aryl group, a heteroaryl group, an alkoxy group, an aryloxy group, an alkylthio group, an arylthio group, an amino group, an amide group, or a halogen atom, where adjacent groups may be bonded to each other to form a 5- or 6-membered ring. However, at least one of $R^{11}$, $R^{12}$, or $R^{13}$ contains a carboxy group or a substituent capable of being bonded to a biological substance.
n is an integer of 1 to 3.
$L^1$ and $L^2$ represent an alkyl group or $-(CH_2-CH_2-O)_m-R^{21}$. Here, $R^{21}$ and m respectively have the same meanings as $R^{21}$ and m described above. $L^1$ and $L^2$ may be bonded to each other to form a ring.
$\alpha 1$ and $\alpha 2$ are 0 or 1.
A ring $Z^1$ and a ring $Z^2$ represent a 6-membered ring formed of a ring-constituting atom selected from a carbon atom and a nitrogen atom, may have a substituent, and may form a fused ring. In a case where the ring $Z^1$ and the ring $Z^2$ form a 6-membered ring having a ring-constituting nitrogen atom, the ring-constituting nitrogen atom may be substituted with a substituent.

The compound represented by Formula (1) has at least one structure represented by $-(CH_2-CH_2-O)_m-R^{21}$. Here, $R^{21}$ and m respectively have the same meanings as $R^{21}$ and m described above.
However, the compound represented by Formula (1) is a neutral compound.
Hereinafter, the substituent and the like in General Formula (1) will be described in detail.

(1) $R^1$ to $R^4$

$R^1$ to $R^4$ represent an alkyl group, an aryl group, a heteroaryl group, or $-(CH_2-CH_2-O)_m-R^{21}$. $R^1$ and $R^2$ may be linked to each other to form a ring, and $R^3$ and $R^4$ may be linked to each other to form a ring.
The alkyl group, the aryl group, and the heteroaryl group, which can be adopted as $R^1$ to $R^4$ respectively have the same meanings as the alkyl group, the aryl group, and the heteroaryl group in the substituent group T described later.
The unsubstituted alkyl group preferably has 1 to 6 carbon atoms, more preferably 1 to 4 carbon atoms, and still more preferably 1 or 2 carbon atoms.
The alkyl group moiety of the alkyl group having a substituent preferably has 1 to 10 carbon atoms, more preferably 1 to 8 carbon atoms, still more preferably 2 to 6 carbon atoms, and even still more preferably 3 to 5 carbon atoms. In addition, the number of atoms constituting the longest chain of the alkyl group having a substituent is preferably 3 to 35, more preferably 3 to 30, and still more preferably 3 to 25.
In the present invention, the "number of carbon atoms of the alkyl group moiety of the alkyl group having a substituent" means the number of carbon atoms excluding the substituent moiety contained in the alkyl group.
In the present invention, the "number of atoms constituting the longest chain of the alkyl group having a substituent" means the number of atoms including the substituent moiety (that is, the number of atoms obtained by subtracting the number of atoms of the molecular chain that does not constitute the longest chain, from the number of total atoms). It is noted that in a case where a substituent having a dissociative hydrogen atom such as a sulfo group or a carboxy group constitutes the longest chain, the calculation is carried out including the hydrogen atom regardless of the presence or absence of dissociation. In addition, the number of atoms in the substituent moiety capable of being bonded to a biological substance described later is not included.
Examples of the substituent which may be contained in the alkyl group which can be adopted as $R^1$ to $R^4$ include an alkoxy group, a carboxy group, an alkoxycarbonyl group, an acyloxy group, a carbamoyl group, an acylamino group, a sulfo group, a phosphono group, $-(CH_2-CH_2-O)_m-R^{21}$, and $-(C_3H_6-O)_m-R^{21}$, as well as a group consisting of a

combination of these substituents. In addition, examples thereof include a substituent capable of being bonded to a biological substance described later. It is noted that the alkyl group moiety in the alkoxy group, the carboxy group, the alkoxycarbonyl group, the acyloxy group, the carbamoyl group, the acylamino group, the sulfo group, and the phosphono group, as well as the group consisting of a combination of these substituents may have a substituent capable of being bonded to a biological substance described later.

The alkyl group having a substituent, which can be adopted as $R^1$ to $R^4$, is not particularly limited as long as it is the above-described alkyl group having a substituent. However, from the viewpoint of suppressing the interaction between molecules, it is preferably an alkyl group having $-(CH_2-CH_2-O)_m-R^{21}$ as a substituent. In this case, the alkyl group may be directly substituted with $-(CH_2-CH_2-O)_m-R^{21}$, or it may be substituted with a group consisting of a combination of a carbamoyl group and$-(CH_2-CH_2-O)_m-R^{21}$.

$(-(CH_2-CH_2-O)_m-R^{21})$

In $-(CH_2-CH_2-O)_m-R^{21}$ which can be adopted as $R^1$ to $R^4$, m is 1 to 10, and $R^{21}$ represents an alkyl group.

m means an average repetition number (simply also referred to as a repetition number), and it is preferably 4 to 10 and more preferably 4 to 8.

The average repetition number can be calculated from the average integrated value obtained by subjecting a compound to $^1H$-NMR measurement. The average repetition number defined in the present invention means a number obtained by rounding off the first decimal place of the average repetition number calculated according to the above method.

To the alkyl group as $R^{21}$, the description for the alkyl group which can be adopted as $R^1$ to $R^4$ described above can be applied.

The $-(CH_2-CH_2-O)_m-R^{21}$ which can be adopted as $R^1$ to $R^4$ and $-(CH_2-CH_2-O)_m-R^{21}$ contained in $R^1$ to $R^4$ are preferably an alkyl group of $-(CH_2-CH_2-O)_m$-unsubstituted.

$(-(C_3H_6-O)_m-R^{21})$

m and $R^{21}$ in $-(C_3H_6-O)_m-R^{21}$, which can be adopted as $R^1$ to $R^4$, respectively have the same meanings as m and $R^{21}$ in $-(CH_2-CH_2-O)_m-R^{21}$.

$-C_3H_5-$ in $-(C_3H_6-O)_m-R^{21}$, which can be adopted as $R^1$ to $R^4$, may have any structure of $-CH_2-CH_2-CH_2-$, $-CH_2-CH(CH_3)-$, or $-CH(CH_3)-CH_2-$.

It is preferable that at least one of $R^1$, ..., or $R^4$ includes a structure represented by $-(CH_2-CH_2-O)_m-$, and from the viewpoint of further improving the fluorescence intensity, it is more preferable that at least one of $R^1$ or $R^2$ and at least one of $R^3$ or $R^4$ include a structure represented by $-(CH_2-CH_2-O)_m-$, and it is still more preferable that at least one of $R^1$ or $R^2$ and at least one of $R^3$ or $R^4$ includes a structure represented by $-(CH_2-CH_2-O)_m-$ and includes a total of four structures represented by $-(CH_2-CH_2-O)_m-$ in the compound. Among the above, it is particularly preferable that at least one of $R^1$ or $R^2$, at least one of $R^3$ or $R^4$, $L^1$, and $L^2$ include a structure represented by $-(CH_2-CH_2-O)_m-$. The structure represented by $-(CH_2-CH_2-O)_m-$ is preferably directly bonded, as $-(CH_2-CH_2-O)_m-R^{21}$, to a ring-constituting atom of a heterocyclic ring directly bonded to the methine chain.

The m in $-(CH_2-CH_2-O)_m-$ described above has the same meaning as the m in $-(CH_2-CH_2-O)_m-R^{21}$ described above.

Since the substituent of each of $R^1$ to $R^4$ is present at a position distant from $R^{11}$ to $R^{13}$ having at least one carboxy group or substituent capable of being bonded to a biological substance and moreover, protrudes in a direction perpendicular to the cyanine dye skeleton (plane), it is presumed that in a case of including a structure represented by $-(CH_2-CH_2-O)_m-$as this substituent, the excluded volume effect due to the structure represented by $-(CH_2-CH_2-O)_m-$ is more effectively exhibited, the fused ring portion is difficult to undergo the $\pi$-$\pi$ interaction (the effect of suppressing the association is strengthened), and the decrease in the fluorescence intensity due to the association can be further suppressed.

(2) $R^{11}$ to $R^{13}$

$R^{11}$ to $R^{13}$ represent a hydrogen atom, an alkyl group, an aryl group, a heteroaryl group, an alkoxy group, an aryloxy group, an alkylthio group, an arylthio group, an amino group, an amide group, or a halogen atom. Adjacent groups may be bonded to each other to form a 5- or 6-membered ring. However, at least one of $R^{11}$, $R^{12}$, or $R^{13}$ has a carboxy group or a substituent capable of being bonded to a biological substance, as will be described later.

The alkyl group, the aryl group, the heteroaryl group, the alkoxy group, the aryloxy group, the alkylthio group, the arylthio group, the amino group, and the halogen atom, which can be adopted as $R^{11}$ to $R^{13}$ respectively have the same meanings as the alkyl group, the aryl group, the heteroaryl group, the alkoxy group, the aryloxy group, the alkylthio group, the arylthio group, the amino group, and the halogen atom in the substituent group T described later, and the

same applies to the preferred range thereof.

In addition, examples of the amide group which can be adopted as $R^{11}$ to $R^{13}$ include, in addition to the amide group in the substituent group T described later, an acylamino group or a carbamoyl group, which is a monovalent substituent derived from carboxylic acid amide, or a sulfonylamino group or a sulfamoyl group, which is a monovalent substituent derived from sulfonic acid amide. These groups respectively have the same meanings as the acylamino group, the carbamoyl group, the sulfonylamino group, and the sulfamoyl group in the substituent group T described later, and the same applies to the preferred ranges thereof. Among these, an acylamino group is preferable as the amide group that can be adopted as $R^{11}$ to $R^{13}$.

The alkyl group, the aryl group, the heteroaryl group, the alkoxy group, the aryloxy group, the alkylthio group, the arylthio group, the amino group, and the amide group, which can be adopted as $R^{11}$ to $R^{13}$, may be unsubstituted or may have a substituent.

Examples of the substituent which may be contained in the alkyl group, the aryl group, the heteroaryl group, the alkoxy group, the aryloxy group, the alkylthio group, the arylthio group, the amino group, and the amide group, as $R^{11}$ to $R^{13}$, include substituents in the substituent group T described later, where the preferred one is, for example, an alkyl group, an alkoxy group, an aryloxy group, an alkylthio group, an arylthio group, an aryl group, a heteroaryl group, an acyl group, an alkoxycarbonyl group, an alkylsulfonyl group, an amino group, an amide group, a nitro group, a cyano group, a sulfo group, or a halogen atom. In addition, examples thereof also include an oxo group (=O) or an imino group (=NH; here, a hydrogen atom may be substituted with a substituent). In addition, preferred examples thereof also include a carboxy group or a substituent capable of being bonded to a biological substance described later, where the substituent which may be contained in the alkyl group, the aryl group, the heteroaryl group, the alkoxy group, the aryloxy group, the alkylthio group, the arylthio group, the amino group, or the amide group, as $R^{11}$ to $R^{13}$, may be a group obtained by combining two or more of the groups described above.

In a case where adjacent groups among $R^{11}$ to $R^{13}$ are bonded to each other to form a 5- or 6-membered ring, the 5- or 6-membered ring to be formed may be either aromatic or aliphatic and is preferably aliphatic. The number of the above-described 5- or 6-membered rings in the compound is not particularly limited; however, it is preferably 1 or 2 and more preferably 1.

In a case of taking a case of n = 3 as an example, preferred examples of the structure having a ring formed by bonding adjacent groups among $R^{11}$ to $R^{13}$ include the following structures. It is noted that in the following examples, although $R^{11}$ to $R^{13}$ that do not form a ring structure are a hydrogen atom, and the ring structure is described as a structure having no substituent, at least one of $R^{11}$, $R^{12}$, or $R^{13}$ has a carboxy group or a substituent capable of being bonded to a biological substance as described above, and the other $R^{11}$ to $R^{13}$ are not limited to the following structures either.

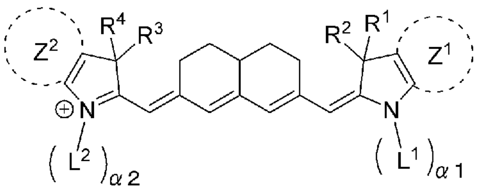
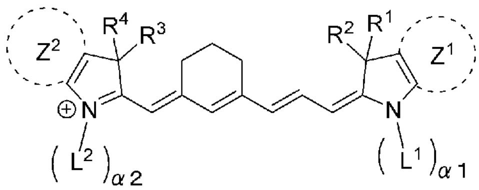

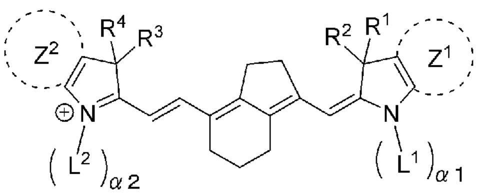
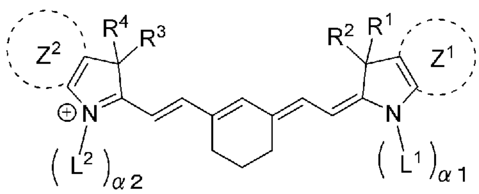

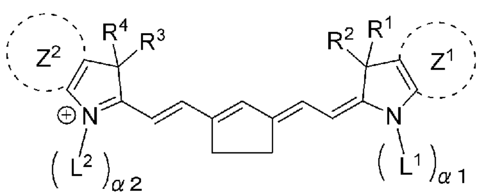

The one among $R^{11}$ to $R^{13}$, which has a substituent having a carboxy group or a substituent capable of being

bonded to a biological substance, is preferably a substituent represented by any one of General Formulae (I) to (V) and is more preferably a substituent represented by General Formula (IV).

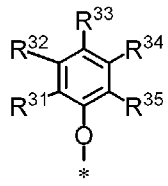

General Formula (I)

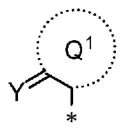

General Formula (II)

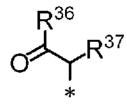

General Formula (III)

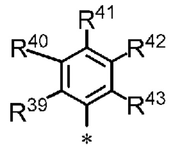

General Formula (IV)

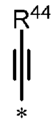

General Formula (V)

In the formulae, Y represents an oxygen atom or $=NR^{38}$, and a ring $Q^1$ represents a heterocyclic ring or hydrocarbon ring which is 5-membered or 6-membered.

$R^{31}$ to $R^{44}$ represent a hydrogen atom, an alkyl group, an aryl group, a heteroaryl group, an alkoxy group, an aryloxy group, an alkylthio group, an arylthio group, an amino group, an amide group, a halogen atom, a sulfo group, a nitro group, a cyano group, a carboxy group, or a substituent capable of being bonded to a biological substance.

It is noted that $R^{36}$ and $R^{37}$ are not bonded to each other to form a 5- or 6-membered ring and the substituent represented by General Formula (II) is different from the substituent represented by General Formula (III).

However, a group represented by any one of General Formulae (I) to (V) has at least one carboxy group or one substituent capable of being bonded to a biological substance.

* represents a bonding site.

Adjacent groups in $R^{31}$ to $R^{35}$ may be linked to each other to form a ring, and adjacent groups in $R^{39}$ to $R^{43}$ may be linked to each other to form a ring.

The alkyl group, the aryl group, the heteroaryl group, the alkoxy group, the aryloxy group, the alkylthio group, the arylthio group, the amino group, and the halogen atom, which can be adopted as $R^{31}$ to $R^{44}$ respectively have the same meanings as the alkyl group, the aryl group, the heteroaryl group, the alkoxy group, the aryloxy group, the alkylthio group, the arylthio group, the amino group, and the halogen atom in the substituent group T described later, and the same applies to the preferred range thereof.

The alkyl group, the aryl group, the heteroaryl group, the alkoxy group, the aryloxy group, the alkylthio group, the arylthio group, the amino group, and the halogen atom, which can be adopted as $R^{31}$ to $R^{44}$ may have a substituent, and preferred examples thereof include a sulfo group and a halogen atom (preferably a fluorine atom).

To the amide group that can be adopted as $R^{31}$ to $R^{44}$, the description for the amide group that can be adopted as $R^{11}$ to $R^{13}$ described above can be applied. Among these, preferred examples of the amide group that can be adopted as $R^{31}$ to $R^{44}$ include a carbamoyl group and a sulfamoyl group.

The substituent capable of being bonded to a biological substance, which can be adopted as $R^{31}$ to $R^{44}$, has the same meaning as the substituent capable of being bonded to a biological substance described later, and the same applies to the preferred range thereof.

In the substituent represented by General Formula (I), $R^{31}$ to $R^{35}$ are preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance and is more preferably a hydrogen atom, an alkyl group, an alkoxy group, an amide group, a halogen atom, a sulfo group, a nitro group, a carboxy group, or a substituent capable of being bonded to a biological substance.

Regarding the substituent represented by General Formula (I), it is preferable that at least one of $R^{32}$, $R^{33}$, or $R^{34}$ has a carboxy group or a substituent capable of being bonded to a biological substance, and it is more preferable that at least one (preferably any one) of $R^{32}$, $R^{33}$, or $R^{34}$ has a carboxy group or a substituent capable of being bonded to a biological substance. A form in which any substituent of $R^{31}$ to $R^{35}$ has a carboxy group or a substituent capable of being bonded to a biological substance may be such that $R^{31}$ to $R^{35}$ may be a carboxy group or a substituent capable of being bonded to a biological substance, and it may be a form in which the substituent that can be adopted as $R^{31}$ to $R^{35}$ is substituted with a carboxy group or a substituent capable of being bonded to a biological substance.

In the substituent represented by General Formula (II), Y is preferably an oxygen atom. The ring $Q^1$ represents a 5- or 6-membered heterocyclic ring or hydrocarbon ring.

The ring $Q^1$ may be an aromatic ring or may be an aliphatic ring; however, it is preferably an aliphatic ring.

In addition, examples of the atom, other than the carbon atom to which Y is bonded and the carbon atom having a bonding site * among the ring-constituting atoms constituting the ring $Q^1$, include a carbon atom, a nitrogen atom, an oxygen atom, or a sulfur atom. The kind of the ring-constituting atom may be one kind or may be two or more kinds;

however, it is preferably one kind or two kinds.

The ring $Q^1$ may have a substituent and may form a fused ring.

The substituent represented by General Formula (II) is preferably a substituent represented by any one of Formulae (E-1) to (E-8).

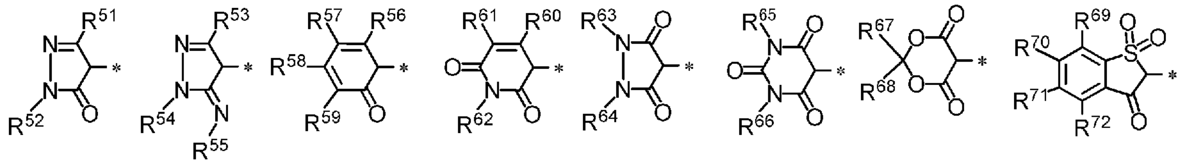

(E-1) (E-2) (E-3) (E-4) (E-5) (E-6) (E-7) (E-8)

In the formulae, $R^{51}$ to $R^{72}$ represent a hydrogen atom, an alkyl group, an aryl group, a heteroaryl group, an alkoxy group, an aryloxy group, an alkylthio group, an arylthio group, an amino group, a halogen atom, a sulfo group, a nitro group, a cyano group, a carboxy group, or a substituent capable of being bonded to a biological substance.

However, a substituent represented by any one of General Formulae (E-1) to (E-8) has at least one carboxy group or one substituent capable of being bonded to a biological substance.

* represents a bonding site.

The alkyl group, the aryl group, the heteroaryl group, the alkoxy group, the aryloxy group, the alkylthio group, the arylthio group, the amino group, and the halogen atom, which can be adopted as $R^{51}$ to $R^{72}$ respectively have the same meanings as the alkyl group, the aryl group, the heteroaryl group, the alkoxy group, the aryloxy group, the alkylthio group, the arylthio group, the amino group, and the halogen atom in the substituent group T described later, and the same applies to the preferred range thereof.

The substituent capable of being bonded to a biological substance, which can be adopted as $R^{51}$ to $R^{72}$, has the same meaning as the substituent capable of being bonded to a biological substance described later, and the same applies to the preferred range thereof.

Among the groups described above, $R^{51}$ to $R^{72}$ are preferably a hydrogen atom, an alkyl group, a cyano group, a carboxy group, or a substituent capable of being bonded to a biological substance.

Regarding the substituent represented by any one of General Formulae (E-1) to (E-8), it suffices that at least one of $R^{51}$, ..., or $R^{72}$ in each of the substituents has a carboxy group or a substituent capable of being bonded to a biological substance. Among them, $R^{52}$ in General Formula (E-1), $R^{54}$ in General Formula (E-2), at least one of $R^{57}$, ..., or $R^{59}$ in General Formula (E-3), at least one of $R^{61}$ or $R^{62}$ in General Formula (E-4), at least one of $R^{63}$ or $R^{64}$ in General Formula (E-5), at least one of $R^{65}$ or $R^{66}$ in General Formula (E-6), at least one of $R^{67}$ or $R^{68}$ in General Formula (E-7), and at least one of $R^{69}$, ..., or $R^{72}$ in General Formula (E-8) preferably have a carboxy group or a substituent capable of being bonded to a biological substance. A form in which any substituent of $R^{51}$ to $R^{72}$ has a carboxy group or a substituent capable of being bonded to a biological substance may be such that $R^{51}$ to $R^{72}$ may be a carboxy group or a substituent capable of being bonded to a biological substance, and it may be a form in which the substituent that can be adopted as $R^{51}$ to $R^{72}$ is substituted with a carboxy group or a substituent capable of being bonded to a biological substance.

In the substituent represented by General Formula (III), $R^{36}$ to $R^{37}$ are preferably, among the groups described above, a hydrogen atom, an alkyl group, an amino group, a cyano group, a carboxy group, or a substituent capable of being bonded to a biological substance.

It suffices that regarding the substituent represented by General Formula (III), at least one of $R^{36}$ or $R^{37}$ has a carboxy group or a substituent capable of being bonded to a biological substance, and it is more preferable that $R^{36}$ has a carboxy group or a substituent capable of being bonded to a biological substance. A form in which any substituent of $R^{36}$ to $R^{37}$ has a carboxy group or a substituent capable of being bonded to a biological substance may be such that $R^{36}$ to $R^{37}$ may be a carboxy group or a substituent capable of being bonded to a biological substance, and it may be a form in which the substituent that can be adopted as $R^{36}$ to $R^{37}$ has a carboxy group or a substituent capable of being bonded to a biological substance.

In the present invention, at least one of $R^{12}$ or $R^{13}$ other than $R^{11}$ and $R^{13}$ possessed by the carbon atom bonded to the heterocyclic ring in which the ring $Z^2$ is fused preferably has a carboxy group or a substituent capable of being bonded to a biological substance and more preferably has a carboxy group or a substituent capable of being bonded to a biological substance, at the central portion of the bond connecting the heterocyclic ring in which the ring $Z^1$ is fused and the heterocyclic ring in which the ring $Z^2$ is fused.

$R^{11}$ and $R^{13}$ possessed by the carbon atom bonded to the heterocyclic ring in which the ring $Z^2$ is fused are

preferably a hydrogen atom.

Among $R^{12}$ and $R^{13}$ other than those described above, the group which does not have a carboxy group or a substituent capable of being bonded to a biological substance is preferably a hydrogen atom, an alkyl group, an aryloxy group, or an arylthio group, more preferably a hydrogen atom or an alkyl group, and still more preferably a hydrogen atom.

In addition, among $R^{11}$ to $R^{13}$, $R^{11}$ and adjacent groups in $R^{12}$ and $R^{13}$ other than the $R^{13}$ possessed by the carbon atom bonded to the heterocyclic ring in which the ring $Z^2$ is fused are preferably bonded to each other to form a 5- or 6-membered ring and more preferably to form a 6-membered ring. In addition, it is preferable that the 5- or 6-membered ring is formed at a central portion of a bond connecting the heterocyclic ring in which the ring $Z^1$ is fused and the heterocyclic ring in which the ring $Z^2$ is fused. The ring formed at the central portion of the bond connecting the heterocyclic ring in which the ring $Z^1$ is fused and the heterocyclic ring in which the ring $Z^2$ is fused means a ring containing a carbon atom as a ring-constituting atom, in which the numbers of bonded atoms from the two heterocyclic rings are the same.

It is noted that in a case where adjacent groups in $R^{11}$ to $R^{13}$ described above are bonded to each other to form a 5-membered or 6-membered ring, the carboxy group or the substituent capable of being bonded to a biological substance is preferably contained at a position other than the ring.

In the substituent represented by General Formula (IV), $R^{39}$ to $R^{43}$ is preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance, more preferably a hydrogen atom, an alkyl group, an alkoxy group, an amide group, a halogen atom, a sulfo group, a nitro group, a cyano group, a carboxy group, or a substituent capable of being bonded to a biological substance, and still more preferably a hydrogen atom, an alkyl group, an alkoxy group, an amide group, a halogen atom, a sulfo group, a nitro group, a carboxy group, or a substituent capable of being bonded to a biological substance.

Regarding the substituent represented by General Formula (IV), $R^{39}$ and $R^{43}$ are preferably an alkyl group, an alkoxy group, or a halogen atom from the viewpoint of further improving the fluorescence intensity and the viewpoint of obtaining excellent light resistance. In addition, it is preferable that at least one of $R^{40}$, $R^{41}$, or $R^{42}$ described above is a carboxy group, an alkyloxy group having a carboxy group, an alkylcarbamoyl group having a carboxy group, or a poly(oxyalkylene) carbamoyl group having a carboxy group or has a substituent capable of being bonded to a biological substance, it is more preferable that at least one of $R^{40}$, $R^{41}$, or $R^{42}$ described above is a carboxy group, an alkyloxy group having a carboxy group, an alkylcarbamoyl group having a carboxy group, or a poly(oxyalkylene) carbamoyl group having a carboxy group, it is still more preferable that $R^{41}$ is a carboxy group, an alkyloxy group having a carboxy group, an alkylcarbamoyl group having a carboxy group, or a poly(oxyalkylene) carbamoyl group having a carboxy group, and it is particularly preferable that $R^{41}$ is a carboxy group.

A form in which any substituent of $R^{39}$ to $R^{43}$ has a carboxy group or a substituent capable of being bonded to a biological substance may be such that $R^{39}$ to $R^{43}$ may be a carboxy group or a substituent capable of being bonded to a biological substance, and it may be a form in which the substituent that can be adopted as $R^{39}$ to $R^{43}$ is substituted with a carboxy group or a substituent capable of being bonded to a biological substance.

In the substituent represented by General Formula (V), among the groups described above, $R^{44}$ is preferably an aryl group which has a carboxy group as a substituent or a substituent capable of being bonded to a biological substance. This aryl group may have a substituent other than the carboxy group or the substituent capable of being bonded to a biological substance, and preferred examples thereof include an alkyl group, an alkoxy group, and a halogen atom.

(3) $L^1$ and $L^2$

$L^1$ and $L^2$ represent an alkyl group or $-(CH_2-CH_2-O)_m-R^{21}$. Here, $R^{21}$ and m respectively have the same meanings as $R^{21}$ and m described above. $L^1$ and $L^2$ may be bonded to each other to form a ring.

Examples of the substituent which may be contained in the alkyl groups as $L^1$ and $L^2$ include an alkoxy group, a carboxy group, an alkoxycarbonyl group, an acyloxy group, a carbamoyl group, an acylamino group, a sulfo group, and a phosphono group, as well as a group consisting of a combination of these substituents. In addition, examples thereof include $-(CH_2-CH_2-O)_m-R^{21}$ ($R^{21}$ and m respectively have the same meanings as $R^{21}$ and m described above) and the substituent capable of being bonded to a biological substance described later. It is noted that the alkyl group moiety in the alkoxy group, the carboxy group, the alkoxycarbonyl group, the acyloxy group, the carbamoyl group, the acylamino group, the sulfo group, and the phosphono group, as well as the group consisting of a combination of these substituents may have $-(CH_2-CH_2-O)_m-R^{21}$ or a substituent capable of being bonded to a biological substance described later.

The alkyl group which can be adopted as $L^1$ and $L^2$ has the same meaning as an alkyl group in the substituent group T described later.

The unsubstituted alkyl group preferably has 1 to 6 carbon atoms, more preferably 1 to 4 carbon atoms, and still more preferably 1 to 3 carbon atoms.

The alkyl group moiety of the alkyl group having a substituent preferably has 1 to 10 carbon atoms, more preferably 1 to 8 carbon atoms, still more preferably 1 to 7 carbon atoms, even still more preferably 1 to 6 carbon atoms, and even further still more preferably 1 to 5 carbon atoms. In addition, the number of atoms constituting the longest chain of the alkyl group having a substituent is preferably 3 to 14, more preferably 3 to 12, and still more preferably 3 to 10.

From the viewpoint of further improving water solubility, the alkyl group having a substituent, which can be adopted as $L^1$ and $L^2$ is preferably an alkyl group having, as a substituent, at least one of an alkoxy group, a carboxy group, a sulfo group, or a phosphono group, more preferably an alkyl group having, as a substituent, at least one of a carboxy group, a sulfo group, or a phosphono group, and still more preferably an alkyl group having, as a substituent, at least one of a carboxy group or a sulfo group. It is noted that it may be an alkyl group having a substituent consisting of a combination of the above-described preferred substituents (the alkoxy group, the carboxy group, the sulfo group, and the phosphono group) and a group other than these substituents.

In addition, the form of the alkyl group having a substituent, which can be adopted by $R^1$ to $R^4$, can be also preferably applied.

For $-(CH_2-CH_2-O)_m-R^{21}$ which can be adopted as $L^1$ and $L^2$, the description for $-(CH_2-CH_2-O)_m-R^{21}$ in $R^1$ to $R^4$ can be preferably applied.

The $-(CH_2-CH_2-O)_m-R^{21}$ which can be adopted as $L^1$ and $L^2$ is preferably an alkyl group in which $R^{21}$ does not have, at the terminal, a substituent capable of being bonded to a carboxy group or a biological substance, and it is more preferably an unsubstituted alkyl group.

From the viewpoint of suppressing the association between dyes due to a structure in a compound, where the structure exhibits high lipid solubility, it is preferable that at least one of $L^1$ or $L^2$ has an alkyl group having, as a substituent, at least one of an alkoxy group, a carboxy group, a sulfo group, or a phosphono group, and it is more preferable that both $L^1$ and $L^2$ are an alkyl group having, as a substituent, at least one of an alkoxy group, a carboxy group, a sulfo group, or a phosphono group.

Preferred examples of the combination of $R^1$ to $R^4$ and $L^1$ and $L^2$ include a combination as an alkyl group in which at least one of $R^1$ or $R^2$ and at least one of $R^3$ or $R^4$ includes a structure represented by $-(CH_2-CH_2-O)_m-$, and at least one of $L^1$ or $L^2$ has, as a substituent, at least one of an alkoxy group, a carboxy group, a sulfo group, or a phosphono group, where a combination as an alkyl group, in which at least one of $R^1$ or $R^2$ and at least one of $R^3$ or $R^4$ includes a structure represented by $-(CH_2-CH_2-O)_m-$ and $L^1$ and $L^2$ have, as a substituent, at least one of an alkoxy group, a carboxy group, a sulfo group, or a phosphono group, is more preferable, and an alkyl group, in which at least one of $R^1$ or $R^2$ and at least one of $R^3$ or $R^4$ includes a structure represented by $-(CH_2-CH_2-O)_m-$ and $L^1$ and $L^2$ have a sulfo group as a substituent, is still more preferable.

It is noted that The alkyl group having, as a substituent, at least one of an alkoxy group, a carboxy group, a sulfo group, or a phosphono group, which can be adopted as $L^1$ and $L^2$, is preferably an alkyl group having, as a substituent, at least one of a carboxy group, a sulfo group, or a phosphono group, more preferably an alkyl group having, as a substituent, at least one of a carboxy group or a sulfo group, and still more preferably an alkyl group having a sulfo group as a substituent.

(4) Ring $Z^1$ and ring $Z^2$

A ring $Z^1$ and a ring $Z^2$ represent a 6-membered ring formed of a ring-constituting atom selected from a carbon atom and a nitrogen atom, may have a substituent, and may form a fused ring.

In a case where the ring $Z^1$ and the ring $Z^2$ form a 6-membered fused ring that is formed of a ring-constituting atom selected from a carbon atom and a nitrogen atom, the entire fused ring is understood as the ring $Z^1$ and the ring $Z^2$.

In a case where the ring $Z^1$ and the ring $Z^2$ form a 6-membered ring having a ring-constituting nitrogen atom, the ring-constituting nitrogen atom may be substituted with a substituent. From the viewpoint of further improving the fluorescence intensity, the ring $Z^1$ and the ring $Z^2$ are a monocyclic ring.

The 6-membered ring formed of a ring-constituting atom selected from a carbon atom and a nitrogen atom, which can be adopted as the ring $Z^1$ and the ring $Z^2$ may be an aliphatic ring or an aromatic ring; however, it is preferably an aromatic ring.

Examples of the 6-membered ring include a hydrocarbon ring or a nitrogen-containing heterocyclic ring. The nitrogen-containing heterocyclic ring is preferably a nitrogen-containing heterocyclic ring in which the ring-constituting atom located at the ortho position with respect to the nitrogen atom to which $L^1$ or $L^2$ is bonded is a nitrogen atom, and it is more preferably a pyridine ring in which the ring-constituting atom located at the ortho position with respect to the nitrogen atom to which $L^1$ or $L^2$ is bonded is a nitrogen atom.

Examples of the 6-membered ring include a benzene ring, a pyridine ring, a pyrimidine ring, a pyridazine ring, a pyrazine ring, and a 1,2,3- or 1,2,4-triazine ring, where a benzene ring or a pyridine ring is preferable. In addition,

examples thereof also include an aliphatic ring having the same kind and position of the ring-constituting atom as these aromatic rings, depending on the method of describing the conjugated structure.

Examples of the substituent which may be contained in the 6-membered ring or the 6-membered fused ring include a substituent in the substituent group T described later, where an alkyl group, an alkoxy group, an aryl group, a carboxy group, a sulfo group, a phosphono group, a sulfonamide group, a nitro group, or a halogen atom is more preferable, and an alkyl group, a sulfo group, a sulfonamide group, a nitro group, or a halogen atom is more preferable.

From the viewpoint of improving water solubility and suppressing association, it is preferable that the ring $Z^1$ and the ring $Z^2$ each independently have one or more hydrophilic groups, and it is more preferable that they have at least one hydrophilic group per one ring constituting the ring $Z^1$ or the ring $Z^2$. For example, in a case where both the ring $Z^1$ and the ring $Z^2$ are naphthalene rings, it is meant to be more preferable that the number of rings constituting each of the ring $Z^1$ and the ring $Z^2$ is 2 and each of the ring $Z^1$ and the ring $Z^2$ has at least two hydrophilic groups. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited, and examples thereof include an alkoxy group having a substituent, a carboxy group, a sulfo group, and a phosphono group, where a sulfo group is preferable. These hydrophilic groups may be directly bonded to the ring $Z^1$ or the ring $Z^2$ or may be bonded through a linking group, where they are preferably directly bonded.

(5) n, $\alpha1$, and $\alpha2$

n is an integer of 1 to 3, where it is preferably an integer of 2 or 3.

$\alpha1$ and $\alpha2$ are 0 or 1. A case where $\alpha1$ is 0 means that $L^1$ is not contained, and a case where $\alpha1$ is 1 means that $L^1$ is contained. Similarly, a case where $\alpha2$ is 0 means that $L^2$ is not contained, and a case where $\alpha2$ is 1 means that $L^2$ is contained.

In a case where the ring $Z^1$ is a nitrogen-containing heterocyclic ring in which the ring-constituting atom located at the ortho position with respect to the nitrogen atom to which $L^1$ or $L^2$ is bonded is a nitrogen atom, 0 can be adopted as $\alpha1$. In addition, In a case where the ring $Z^2$ is a nitrogen-containing heterocyclic ring in which the ring-constituting atom located at the ortho position with respect to the nitrogen atom to which $L^1$ or $L^2$ is bonded is a nitrogen atom, 0 can be adopted as $\alpha2$.

At least one of $R^{11}$, $R^{12}$, or $R^{13}$ contains a carboxy group or a substituent capable of being bonded to a biological substance.

The compound represented by General Formula (1) can be bonded to a biological substance with the above-described carboxy group or a substituent capable of being bonded to a biological substance, whereby a targeted labeled biological substance can be obtained. It is noted that a substituent capable of being bonded to a biological substance can be easily derived from a carboxy group by a conventional method.

In the present invention, the "substituent capable of being bonded to a biological substance" includes a substituent capable of being bonded to a biological substance, which is derived from a carboxy group.

As described above, since the compound represented by General Formula (1) is bonded to a biological substance with a substituent (specifically, at least one of $R^{11}$, $R^{12}$, or $R^{13}$ which is a substituent on a polymethine chain having a repetition number of n + 1) contained in a specific position in the cyanine skeleton structure, a labeled biological substance to be obtained is conceived to exhibit excellent fluorescence intensity, as described above.

It suffices that the number of groups having a carboxy group or a substituent capable of being bonded to a biological substance in $R^{11}$ to $R^{13}$ is 1 or more in total, and it is preferably 1 to 3, more preferably 1 or 2, and still more preferably 1, from the viewpoint of the quantification of the substance to be detected.

In addition, from the viewpoint of imparting sufficient hydrophilicity as the compound, the compound represented by General Formula (1) preferably has two or more hydrophilic groups, more preferably has 2 to 8 hydrophilic groups, still more preferably has 2 to 6 hydrophilic groups, and particularly preferably has 3 to 6 hydrophilic groups, as the compound as a whole.

To the hydrophilic group, the description for the hydrophilic group which can be adopted by the ring $Z^1$ and ring $Z^2$ described above can be applied.

The position of the hydrophilic group is not particularly limited; however, preferred examples of the group having the hydrophilic group include $R^{11}$ to $R^{13}$, the ring $Z^1$, the ring $Z^2$, $L^1$, and $L^2$.

It is noted that in a case where the compound represented by General Formula (1) has the hydrophilic group as the above-described substituent capable of being bonded to a carboxy group or a biological substance, it is practical and preferable that the above-described substituent capable of being bonded to a carboxy group or a biological substance has one or more hydrophilic groups. Specific examples thereof include a carboxy group and a sulfo group, where a group in which an alkyl group, an alkoxy group, or the like has, as a substituent, a carboxy group, a sulfo group, or the like may be used, examples of which include a sulfoalkyl group and a sulfoalkoxy group.

<Compound represented by any one of General Formulae (2-1) to (2-4)>

The compound represented by General Formula (1) according to the embodiment of the present invention is preferably represented by any of the following General Formulae (2-1) to (2-4).

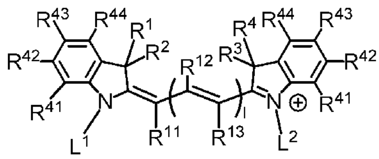

General Formula (2-1)

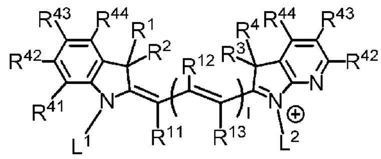

General Formula (2-2)

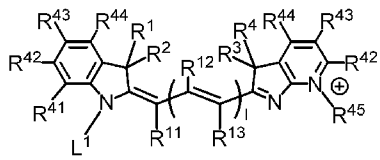

General Formula (2-3)

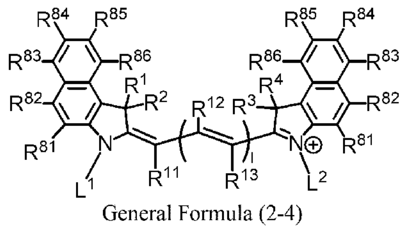

General Formula (2-4)

In the formulae, $R^{45}$ represent an alkyl group or $-(CH_2-CH_2-O)_m-R^{21}$.

$R^{41}$ to $R^{44}$ and $R^{81}$ to $R^{86}$ represent a hydrogen atom, an alkyl group, an alkoxy group, an aryl group, a sulfo group, a sulfonamide group, a nitro group, a carboxy group, an amide group, or a halogen atom, where adjacent groups may be bonded to each other to form a fused ring.

It is noted that a compound represented by any of General Formulae (2-1) or (2-4) is preferentially classified into the compound represented by General Formula (2-4). That is, $R^{43}$ and $R^{44}$ in General Formula (2-1) are not bonded to each other to form a benzene ring.

$R^1$ to $R^4$, $R^{11}$ to $R^{13}$, $L^1$, $L^2$, and m respectively have the same meanings as $R^1$ to $R^4$, $R^{11}$ to $R^{13}$, $L^1$, $L^2$, and m in General Formula (1), and the same applies to the preferred range thereof.

At least one of $R^1$ to $R^4$, $L^1$, $L^2$, or $R^{45}$ is $-(CH_2-CH_2-O)_m-R^{21}$. That is, it means that in General Formulae (2-1), (2-2), and (2-4), at least one of $R^1$ to $R^4$, $L^1$, or $L^2$ is $-(CH_2-CH_2-O)_m-R^{21}$, and in General Formula (2-3), at least one of $R^1$ to $R^4$, $L^1$, or $R^{45}$ is $-(CH_2-CH_2-O)_m-R^{21}$. $R^{21}$ and m respectively have the same meanings as $R^{21}$ and m in General Formula (1).

l represents an integer of 2 or 3.

Similar to the compound represented by General Formula (1), in the compound represented by any one of General Formulae (2-1) to (2-4) at least one of $R^{11}$, $R^{12}$, or $R^{13}$ contains a carboxy group or a substituent capable of being bonded to a biological substance.

However, the compound represented by any one of Formulae (2-1) to (2-4) is a neutral compound.

The alkyl group and $-(CH_2-CH_2-O)_m-R^{21}$, which can be adopted as $R^{45}$, respectively have the same meanings as the alkyl group and$-(CH_2-CH_2-O)_m-R^{21}$, which can be adopted as $L^1$ or $L^2$.

The alkyl group, the alkoxy group, the sulfo group, the sulfonamide group, the nitro group, the carboxy group, and the halogen atom as $R^{41}$ to $R^{44}$ and $R^{81}$ to $R^{86}$ respectively have the same meanings as the alkyl group, the aryl group, the alkoxy group, the aryl group, the sulfo group, the sulfonamide group, the nitro group, the carboxy group, and the halogen atom in the substituent group T described later.

To the amide group that can be adopted as $R^{41}$ to $R^{44}$ and $R^{81}$ to $R^{86}$, the description for the amide group that can be adopted as $R^{11}$ to $R^{13}$ in General Formula (1) can be applied.

$R^{41}$ to $R^{44}$ are preferably a hydrogen atom, an alkyl group, an alkoxy group, a sulfo group, or a carboxy group.

Among $R^{41}$ to $R^{44}$, adjacent groups may be linked to each other to form a ring. The ring to be formed may be a monocyclic ring or may be a fused ring. The ring is preferably composed of 5 or 6 ring members and may be an aliphatic ring or an aromatic ring. In a case being a 6-membered ring, it is preferably an aromatic ring.

In a case of being a fused ring, it is preferably a fused ring consisting of 2 to 5 rings including a benzene ring to which $R^{41}$ to $R^{44}$ are bonded, more preferably a fused ring consisting of 2 to 4 rings, and still more preferably a fused ring consisting of 2 or 3 rings.

The form in which a ring is formed is preferably such that adjacent groups in $R^{41}$ to $R^{44}$ are linked to each other to form at least a benzene ring, that is, the adjacent groups form at least a naphthalene ring structure, together with a benzene ring to which $R^{41}$ to $R^{44}$ are bonded.

Among the above, in the compound represented by General Formula (2-1) among the compounds represented by any of General Formulae (2-1) to (2-3), it is preferable that $R^{41}$ and $R^{42}$ form a ring, $R^{42}$ and $R^{43}$ form a ring, or $R^{43}$ and $R^{44}$ form a ring, and it is more preferable that $R^{42}$ and $R^{43}$ form a ring or $R^{42}$ and $R^{43}$ form a ring.

The ring composed of a benzene ring to which $R^{41}$ to $R^{44}$ are bonded and a ring that is formed by linking adjacent groups in $R^{41}$ to $R^{44}$ to each other may have a substituent. Here, examples of the substituent which may be contained include the alkyl group, the alkoxy group, the aryl group, the sulfo group, the sulfonamide group, the nitro group, the carboxy group, and the halogen atom, which can be adopted as $R^{41}$ to $R^{44}$ described above, where an alkyl group, an alkoxy group, a sulfo group, or a carboxy group is preferable.

From the viewpoint of improving water solubility and suppressing association, in the compound represented by any one of General Formulae (2-1) to (2-3), the rings corresponding to the ring $Z^1$ and the ring $Z^2$ in the compound represented by General Formula (1) each independently preferably have one or more hydrophilic groups and more preferably has at least one hydrophilic group per one ring constituting a ring corresponding to the ring $Z^1$ or the ring $Z^2$. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited, and examples thereof include an alkoxy group having a substituent, a carboxy group, a sulfo group, and a phosphono group, where a sulfo group is preferable. These hydrophilic groups may be directly bonded to a ring corresponding to the ring $Z^1$ or the ring $Z^2$ or may be bonded through a linking group, where they are preferably directly bonded.

The position having a hydrophilic group in the ring corresponding to the ring $Z^1$ or the ring $Z^2$ is not particularly limited. For example, it is preferable to have a hydrophilic group as at least $R^{43}$, and it is more preferable to have a hydrophilic group as $R^{43}$.

$R^{81}$ to $R^{86}$ are preferably a hydrogen atom, an alkyl group, an alkoxy group, an aryl group, a sulfo group, or a carboxy group.

The alkyl group, the alkoxy group, and the aryl group, which can be adopted as $R^{81}$ to $R^{86}$, may have a substituent. Here, examples of the substituent which may be contained include an alkyl group, an alkoxy group, a sulfo group, a sulfonamide group, a nitro group, a carboxy group, and a halogen atom, where an alkyl group, an alkoxy group, a sulfo group, or a carboxy group is preferable.

From the viewpoint of improving water solubility and suppressing association, in the compound represented by General Formula (2-4), the rings corresponding to the ring $Z^1$ and the ring $Z^2$ in the compound represented by General Formula (1) each independently preferably have one or more hydrophilic groups and more preferably has at least one hydrophilic group per one ring constituting a ring corresponding to the ring $Z^1$ or the ring $Z^2$. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited, and examples thereof include an alkoxy group having a substituent, a carboxy group, a sulfo group, and a phosphono group, where a sulfo group is preferable. These hydrophilic groups may be directly bonded to a ring corresponding to the ring $Z^1$ or the ring $Z^2$ or may be bonded through a linking group, where they are preferably directly bonded.

The position having the hydrophilic group in the ring corresponding to the ring $Z^1$ or the ring $Z^2$ is not particularly limited. For example, it is preferable to have a hydrophilic group in at least any one of $R^{83}$, $R^{84}$, or $R^{85}$, it is more preferable to have a hydrophilic group at least as $R^{83}$ or $R^{85}$ or have a hydrophilic group at least as $R^{84}$, it is still more preferable to have a hydrophilic group as $R^{83}$ or $R^{85}$ or have a hydrophilic group as $R^{84}$, and it is particularly preferable to have a hydrophilic group as $R^{83}$ and $R^{85}$.

Specific examples of the compound according to the embodiment of the present invention, which is represented by General Formula (1), will be shown; however, the present invention is not limited to these compounds. In the following specific examples, $X^+$ in $-SO_3X$ represents a hydrogen ion ($H^+$), a sodium ion, a potassium ion, ammonium ($NH_4^+$), or triethylammonium ($NH(CH_2CH_3)_3^+$).

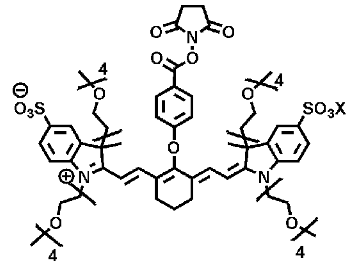
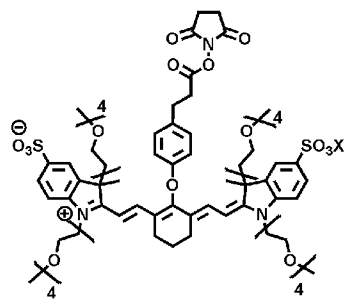
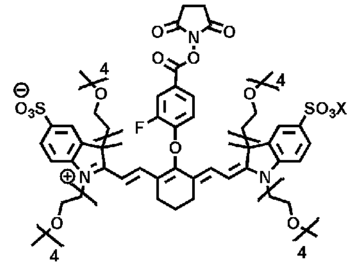

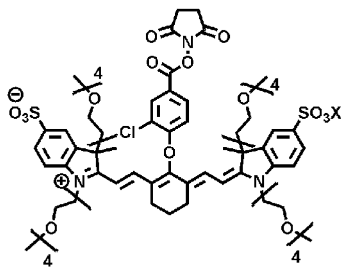

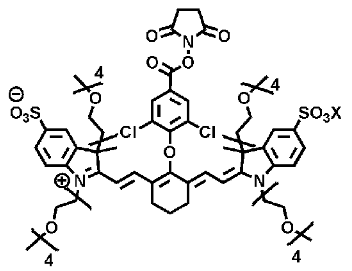

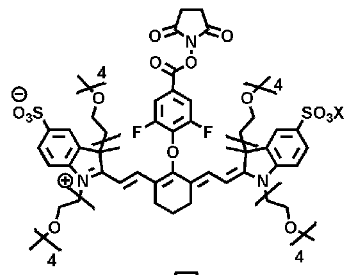

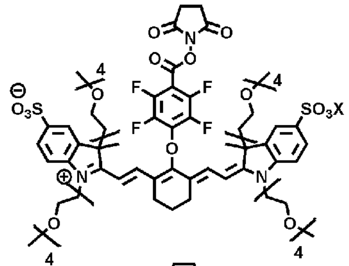

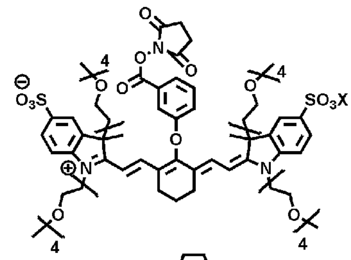

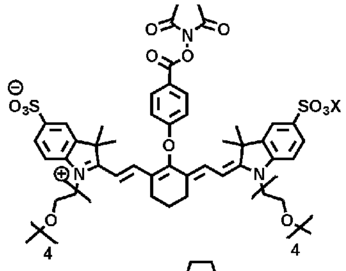

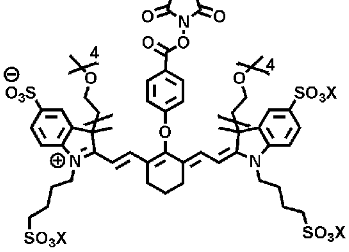

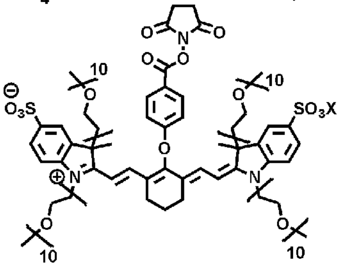

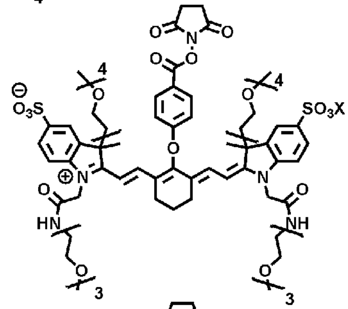

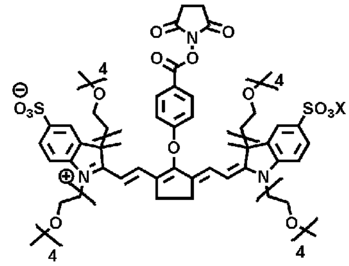

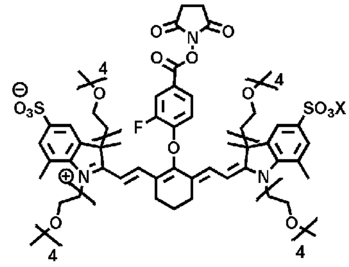

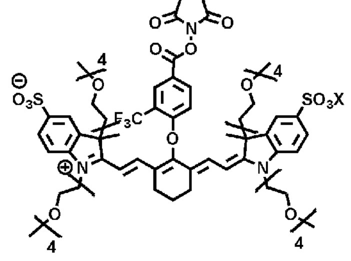

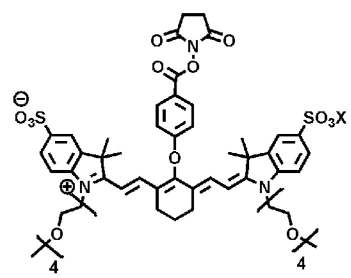

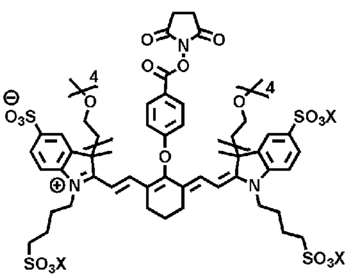

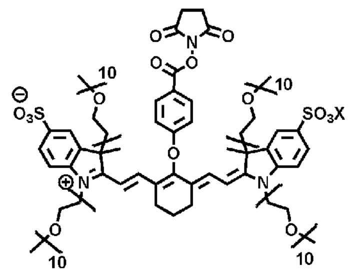

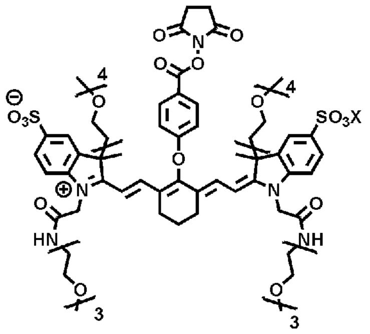

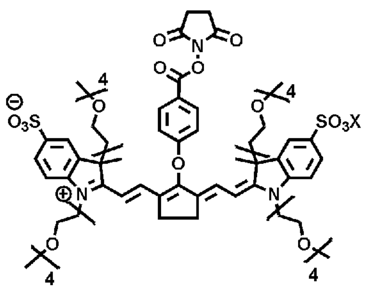

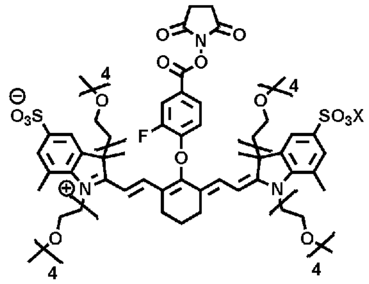

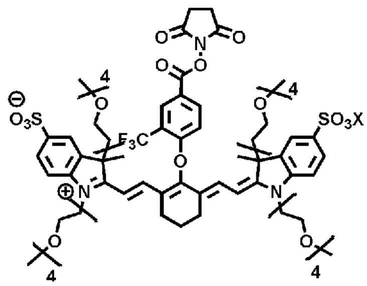

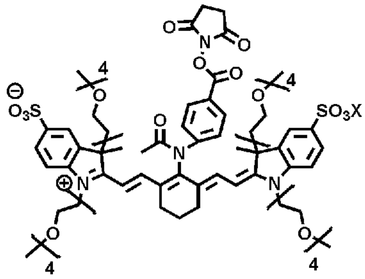

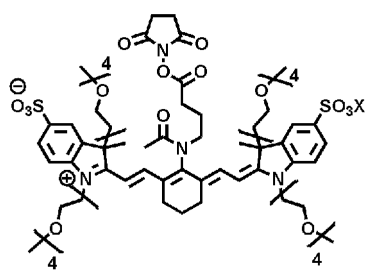

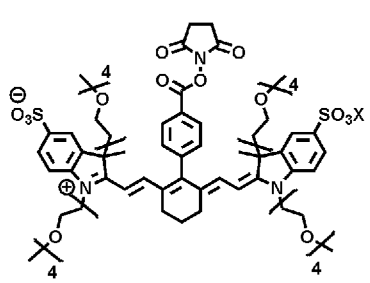

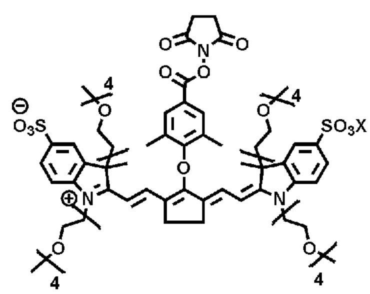

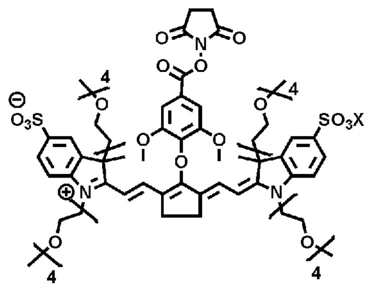

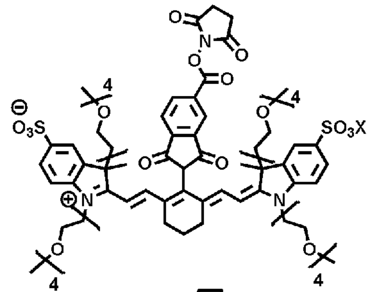

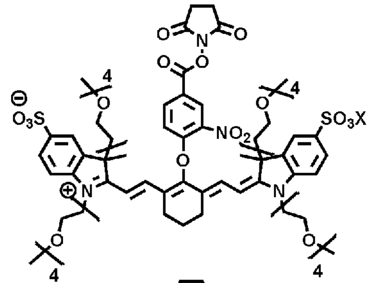

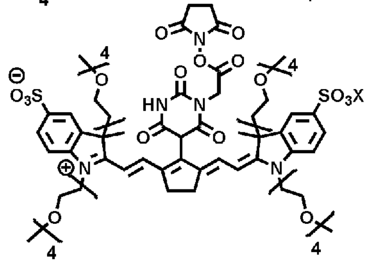

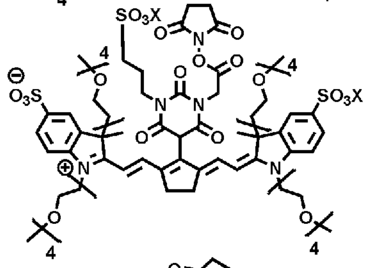

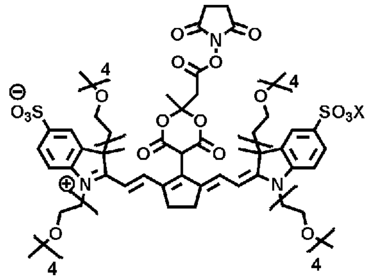

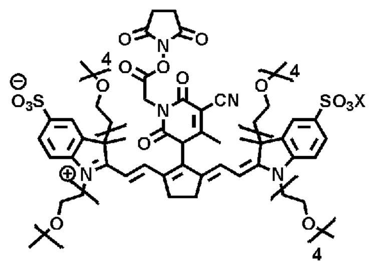

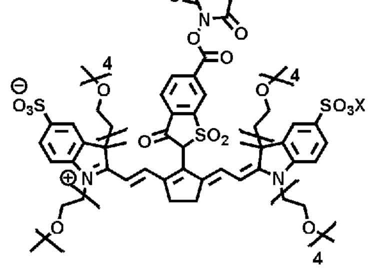

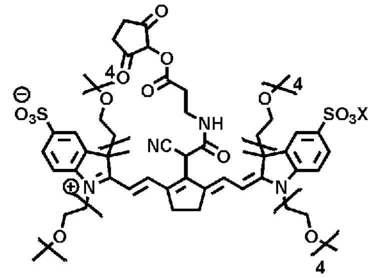

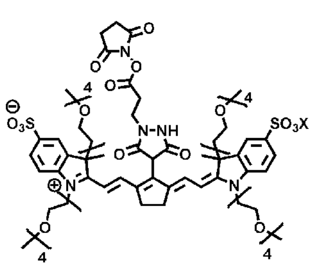

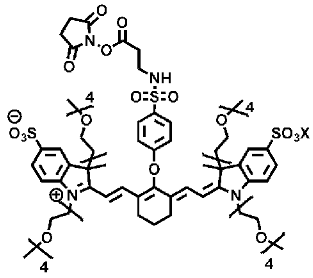

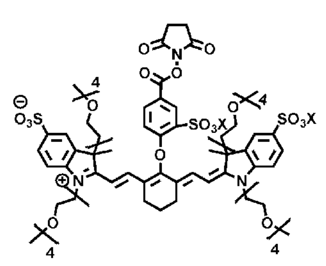

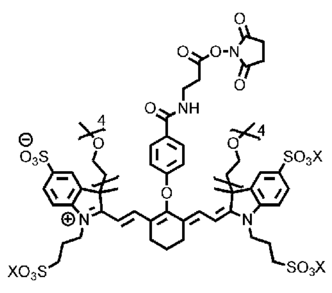

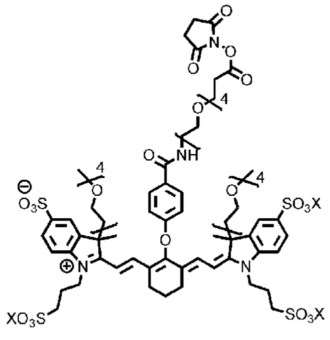

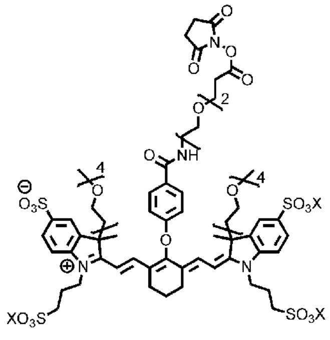

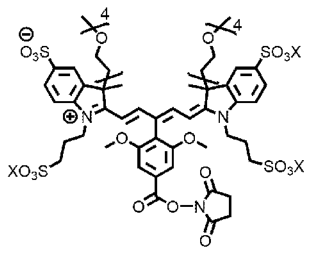

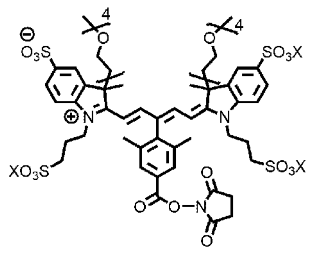

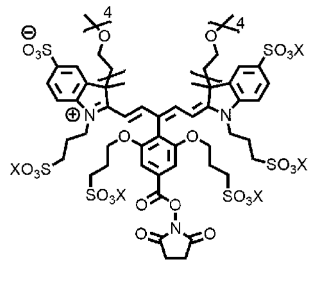

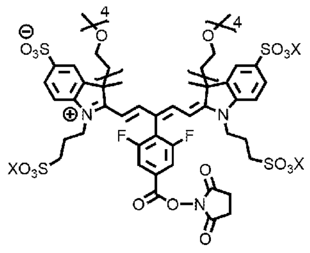

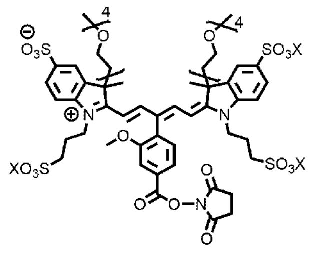

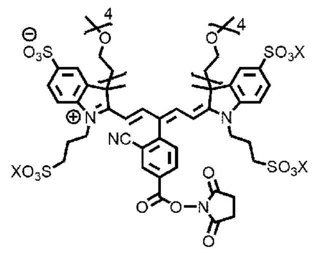

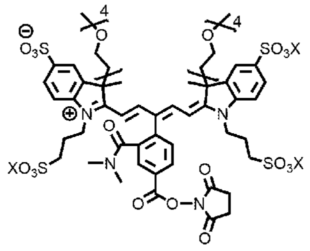

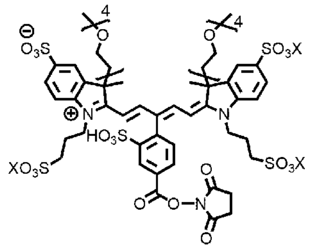

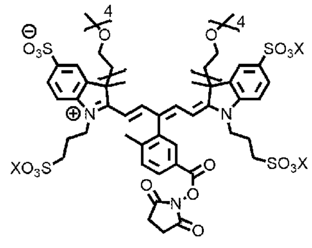

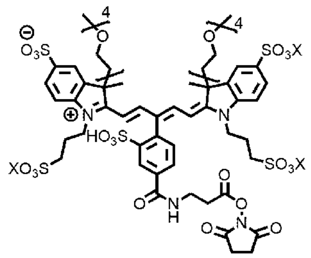

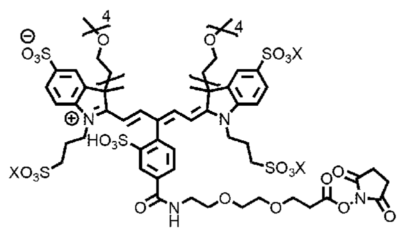

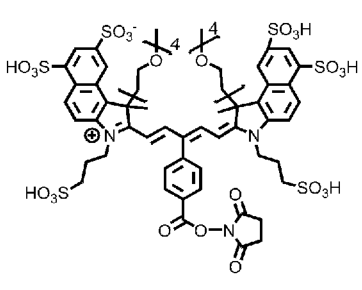

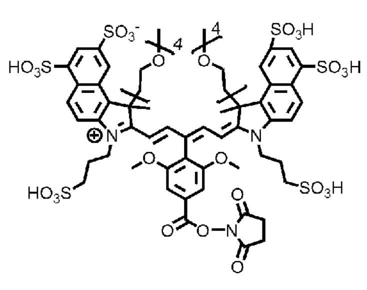

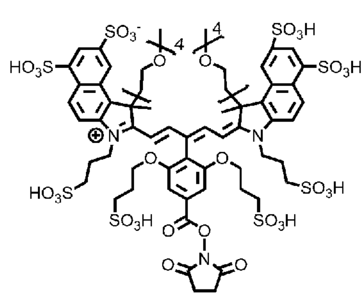

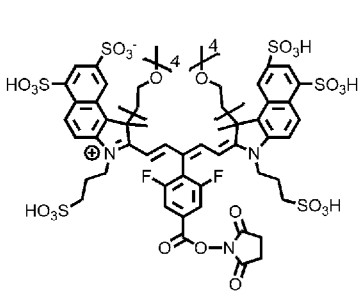

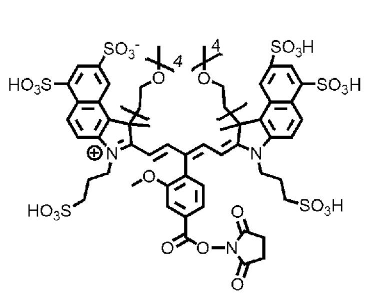

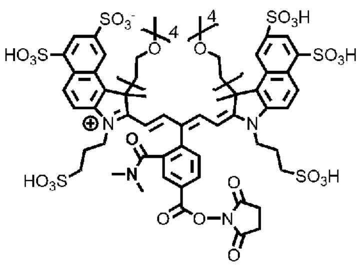
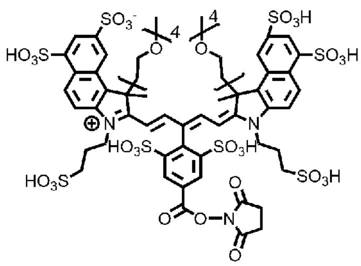
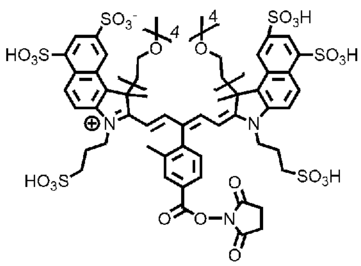
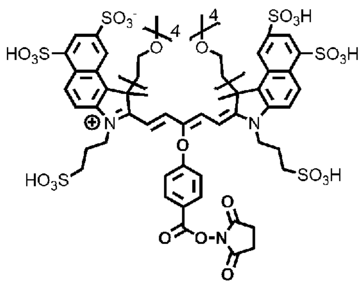
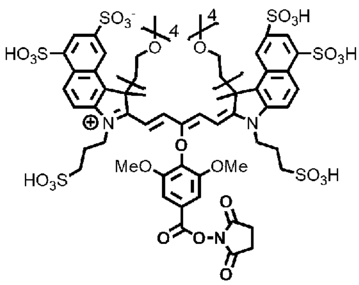
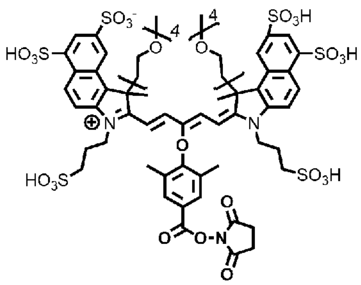
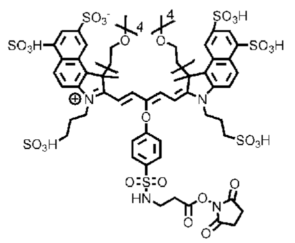
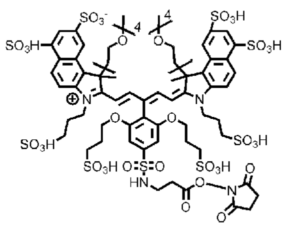
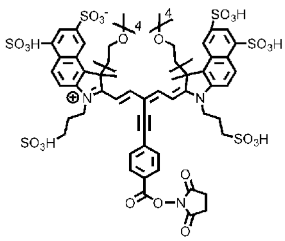
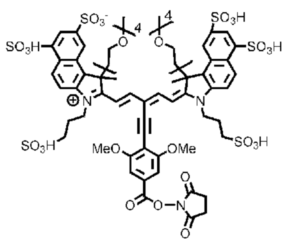
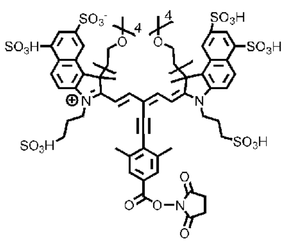
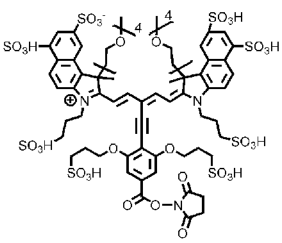
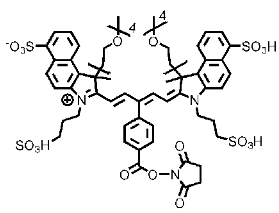
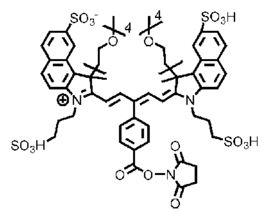
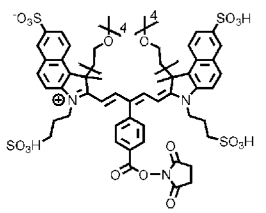
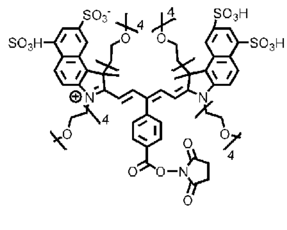
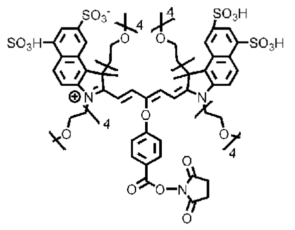
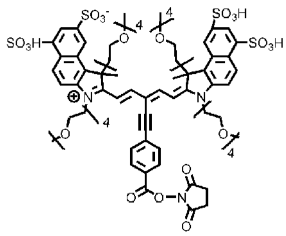

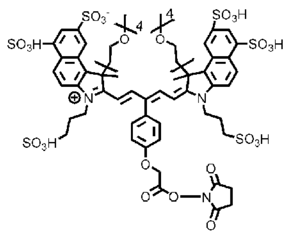
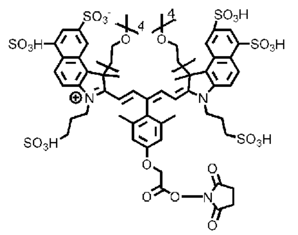
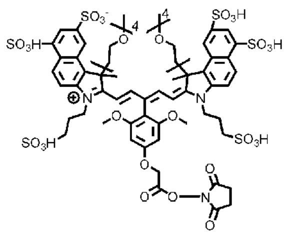
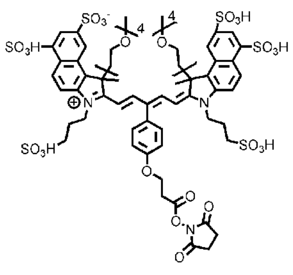
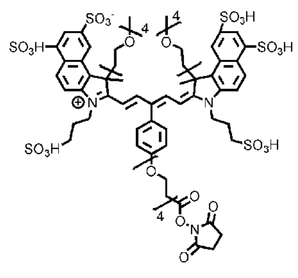
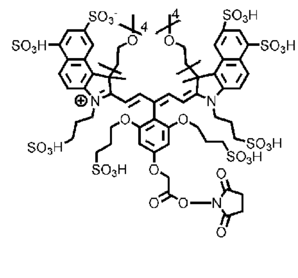
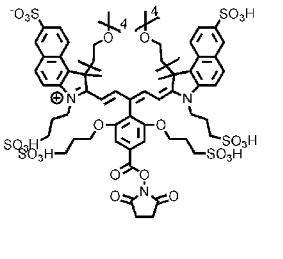
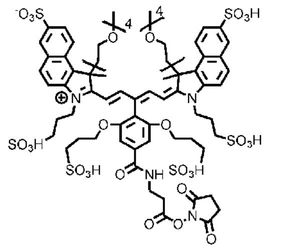
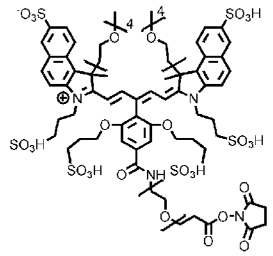
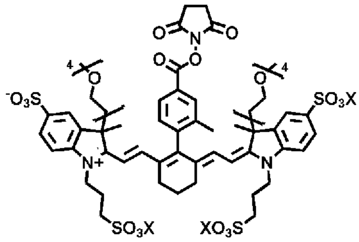
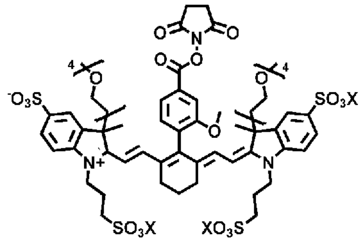
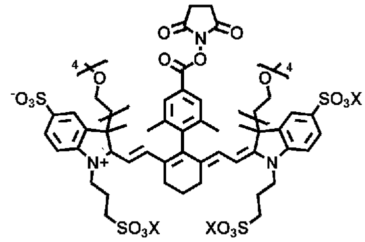
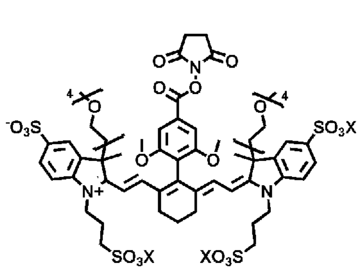
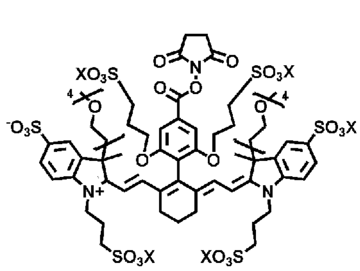
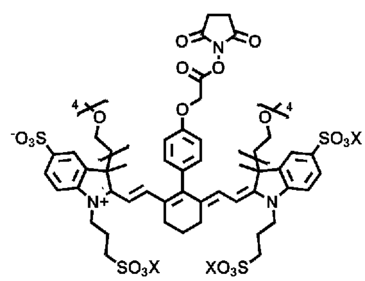
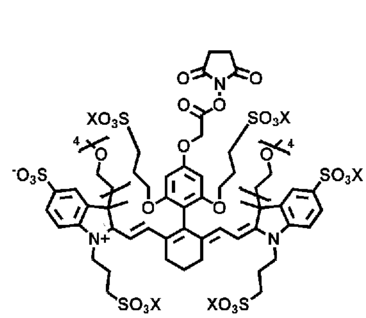
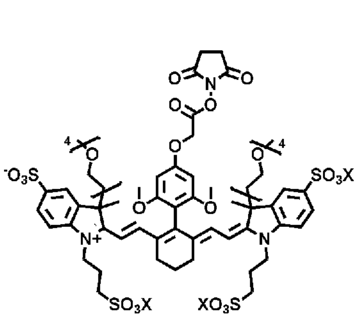
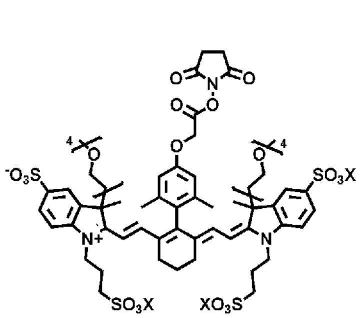

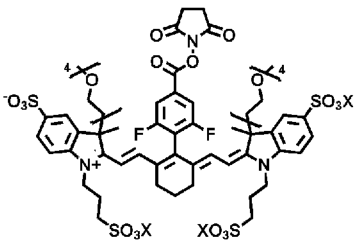
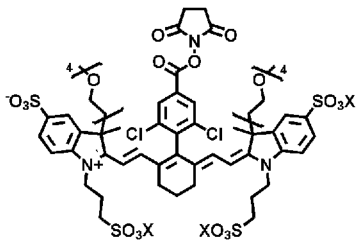
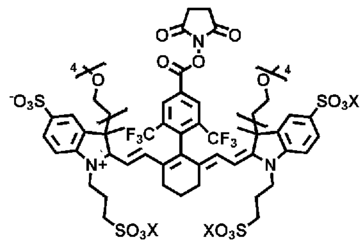

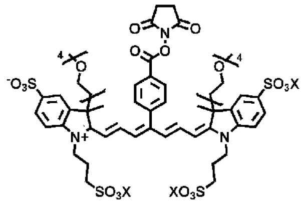
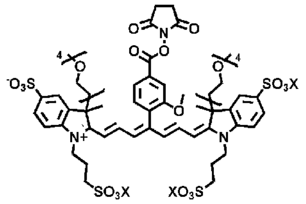
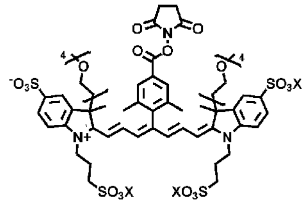

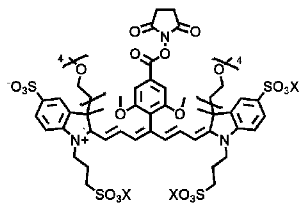
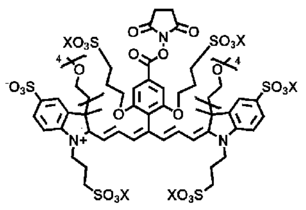
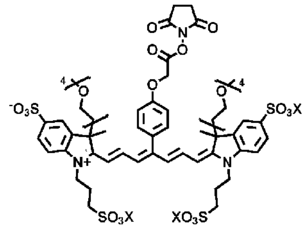

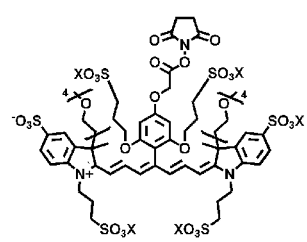
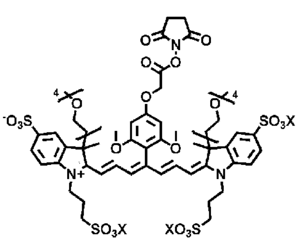
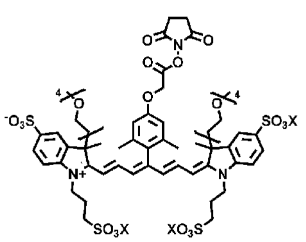

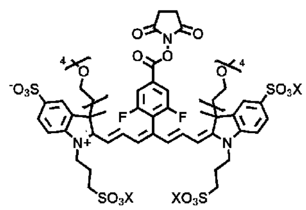
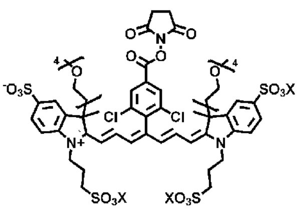
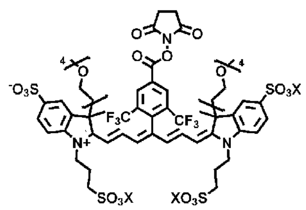

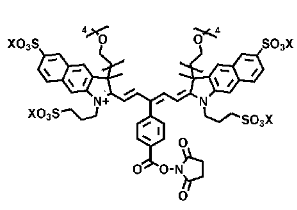
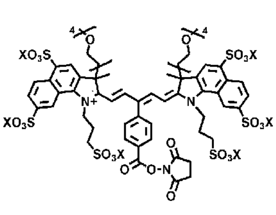
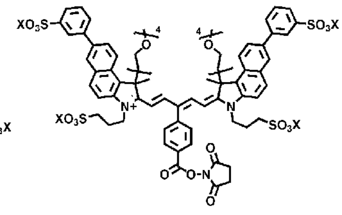

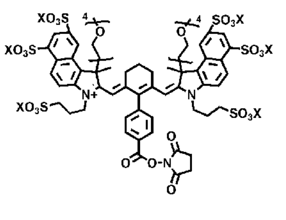
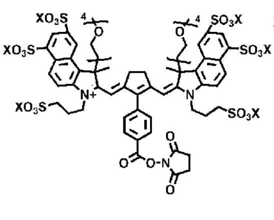
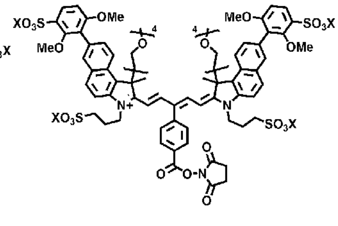

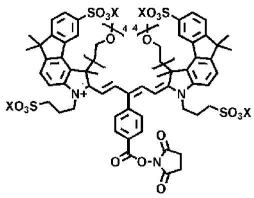

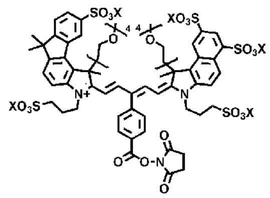

A first preferred form according to the present invention includes those having an excitation absorption wavelength at 740 to 830 nm among the compounds represented by General Formula (1). This includes such a substituent that one among $R^{11}$ to $R^{13}$ described above, which has a substituent having a carboxy group or a substituent capable of being bonded to a biological substance, is a substituent represented by any one of General Formulae (I) to (V), preferably a substituent represented by any one of General Formulae (I), (II), or (IV), and more preferably a substituent represented by General Formula (I) or (IV).

Examples of the compound in the first preferred form according to the present invention include a compound represented by General Formula (2-1) among the compounds represented by any of General Formulae (2-1) to (2-4) (provided that l is an integer of 3), and more preferred examples thereof include a compound in which each substituent in General Formula (2-1) satisfies any one of the following form I or II.

(Form I)

one among $R^{11}$ to $R^{13}$ described above, which has a substituent having a carboxy group or a substituent capable of being bonded to a biological substance, is a substituent represented by General Formulae (I) or (II), A form in which $L^1$ and $L^2$ are $-(CH_2-CH_2-O)_m-R^{21}$ described above or an alkyl group having, as a substituent, at least one of an alkoxy group, a carboxy group, a sulfo group, or a phosphono group, at least one of $R^1$ or $R^2$ and at least one of $R^3$ or $R^4$ includes a structure represented by $-(CH_2-CH_2-O)_m-$ described above, and at least one of $R^{41}$, ..., or $R^{44}$ (preferably $R^{43}$) is a sulfo group.

In this form, the substituents $R^{31}$ to $R^{35}$ in General Formula (I) are preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance and is more preferably a hydrogen atom, an alkyl group, an alkoxy group, an amide group, a halogen atom, a sulfo group, a nitro group, a carboxy group, or a substituent capable of being bonded to a biological substance. Among the substituents represented by General Formula (I), $R^{31}$ and $R^{35}$ are preferably an alkyl group, an alkoxy group, or a halogen atom from the viewpoint of further improving the fluorescence intensity and the viewpoint of obtaining excellent light resistance. In addition, it is particularly preferable that at least one of $R^{32}$, $R^{33}$, or $R^{34}$ has a carboxy group or a substituent capable of being bonded to a biological substance, and

In this form, the substituent represented by General Formula (II) is preferably the substituent represented by any one of Formulae (E-4) to (E-8) described above, and it is more preferably the group represented by Formula (E-6) described above. The description described above can be applied to the substituents in Formulae (E-4) to (E-8).

(Form II)

A form in which one among $R^{11}$ to $R^{13}$ described above, which has a substituent having a carboxy group or a substituent capable of being bonded to a biological substance, is a substituent represented by General Formula (IV), $L^1$ and $L^2$ are $-(CH_2-CH_2-O)_m-R^{21}$ described above or an alkyl group having, as a substituent, at least one of an alkoxy group, a carboxy group, a sulfo group, or a phosphono group, at least one of $R^1$ or $R^2$ and at least one of $R^3$ or $R^4$ includes a structure represented by $-(CH_2-CH_2-O)_m-$ described above, and at least one of $R^{41}$, ..., or $R^{44}$ (preferably $R^{43}$) is a sulfo group.

In this form, the substituents $R^{39}$ to $R^{43}$ in General Formula (IV) are preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance and is more preferably a hydrogen atom, an alkyl group, an alkoxy group, an amide group, a halogen atom, a sulfo group, a nitro group, a carboxy group, or a substituent capable of being bonded to a biological substance. Among the substituents represented by General Formula (IV), $R^{39}$ and $R^{43}$ are preferably an alkyl group, an alkoxy group, or a halogen atom from the viewpoint of further improving the fluorescence intensity and the viewpoint of obtaining excellent light resistance. In addition, it is particularly preferable that at least one of $R^{40}$, $R^{41}$, or $R^{42}$ has a carboxy group or a substituent capable of being bonded to a biological substance.

A second preferred form according to the present invention includes those having an excitation absorption wavelength at 620 to 700 nm, which are preferably a compound represented by General Formula (2-1) among the compounds represented by any of General Formulae (2-1) to (2-4) (provided that l is an integer of 2). This includes such

a substituent that one among $R^{11}$ to $R^{13}$ described above, which has a substituent having a carboxy group or a substituent capable of being bonded to a biological substance, is a substituent represented by any one of General Formulae (I) to (V), preferably a substituent represented by any one of General Formulae (I), (IV), or (V), and more preferably a substituent represented by General Formula (IV).

The substituents $R^{39}$ to $R^{43}$ in General Formula (IV) are preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance and is more preferably a hydrogen atom, an alkyl group, an alkoxy group, an amide group, a halogen atom, a sulfo group, a nitro group, a carboxy group, or a substituent capable of being bonded to a biological substance. Among the substituents represented by General Formula (IV), $R^{39}$ and $R^{43}$ are preferably an alkyl group, an alkoxy group, or a halogen atom from the viewpoint of further improving the fluorescence intensity and the viewpoint of obtaining excellent light resistance. In addition, it is particularly preferable that at least one of $R^{40}$, $R^{41}$, or $R^{42}$ has a carboxy group or a substituent capable of being bonded to a biological substance.

Among the compounds represented by General Formula (2-1) in the second preferred form according to the present invention (provided that l is an integer of 2), preferred examples of the substituent other than the above-described substituents include a form in which $L^1$ and $L^2$ are an alkyl group having, as a substituent, at least one of an alkoxy group, a carboxy group, a sulfo group, or a phosphono group, at least one of $R^1$ or $R^2$ and at least one of $R^3$ or $R^4$ includes a structure represented by $-(CH_2-CH_2-O)_m-$ described above, and at least one of $R^{41}$, ..., or $R^{44}$ (preferably $R^{43}$) is a sulfo group.

A third preferred form according to the present invention includes those having an excitation absorption wavelength at 620 to 720 nm, which are preferably a compound represented by General Formula (2-4) among the compounds represented by any of General Formulae (2-1) to (2-4) (provided that l is an integer of 2). This includes such a substituent that one among $R^{11}$ to $R^{13}$ described above, which has a substituent having a carboxy group or a substituent capable of being bonded to a biological substance, is a substituent represented by any one of General Formulae (I) to (V), preferably a substituent represented by any one of General Formulae (I), (IV), or (V), and more preferably a substituent represented by General Formula (IV).

The substituents $R^{39}$ to $R^{43}$ in General Formula (IV) are preferably, among the groups described above, a hydrogen atom, an alkyl group, an alkoxy group, an alkylthio group, an amino group, an amide group, a halogen atom, a sulfo group, a nitro group, a cyano group, a carboxy group, or a substituent capable of being bonded to a biological substance and is more preferably a hydrogen atom, an alkyl group, an alkoxy group, an amide group, a halogen atom, a sulfo group, a nitro group, a carboxy group, or a substituent capable of being bonded to a biological substance. From the viewpoint of further improving the fluorescence intensity and the viewpoint of obtaining excellent light resistance, among the substituents represented by General Formula (IV), it is preferable that at least one of $R^{39}$ or $R^{43}$ is an alkyl group, an alkoxy group, or a halogen atom, and it is more preferable that $R^{39}$ and $R^{43}$ are an alkyl group, an alkoxy group, or a halogen atom. In addition, it is preferable that at least one of $R^{40}$, $R^{41}$, or $R^{42}$ has a carboxy group or a substituent capable of being bonded to a biological substance, and it is particularly preferable that $R^{41}$ has a carboxy group or a substituent capable of being bonded to a biological substance.

Among the compounds represented by General Formula (2-4) in the third preferred form according to the present invention (provided that l is an integer of 2), preferred examples of the substituent other than the above-described substituents include a form in which $L^1$ and $L^2$ are $-(CH_2-CH_2-O)_m-R^{21}$ described above or an alkyl group having, as a substituent, at least one of an alkoxy group, a carboxy group, a sulfo group, or a phosphono group, at least one of $R^1$ or $R^2$ and at least one of $R^3$ or $R^4$ includes a structure represented by $-(CH_2-CH_2-O)_m-$described above, and at least one of $R^{83}$, $R^{84}$, or $R^{85}$ (preferably $R^{83}$ and $R^{85}$, or $R^{84}$; and more preferably $R^{83}$ and $R^{85}$) is a sulfo group.

The compound according to the embodiment of the present invention, which is represented by General Formula (1), can be bonded to a biological substance such as a protein, a peptide, an amino acid, a nucleic acid, a sugar chain, or a lipid, with a substituent capable of being bonded to a biological substance, where the substituent is contained in at least one of $R^{11}$, $R^{12}$, or $R^{13}$, and it can be used as a labeled biological substance.

The substituent capable of being bonded to a biological substance can be used without particular limitation as long as it is a group for acting (including adhering) or bonding to a biological substance, and examples thereof include the substituents described in WO2002/026891A. Among them, preferred examples thereof include an N-hydroxysuccinimide ester (NHS) structure, a succinimide structure, a maleimide structure, an azido group, an acetylene group, a peptide structure (a polyamino acid structure), a long-chain alkyl group (preferably having 12 to 30 carbon atoms), and a quaternary ammonium group.

Among the compounds according to the embodiment of the present invention, which are represented by General Formula (1), specific examples of the compound in which at least any one of $R^{11}$, $R^{12}$, or $R^{13}$ has at least a substituent capable of being bonded to a biological substance include an exemplary compound of a fluorescently labeled biological substance described later, in addition to the above-described compounds according to the embodiment of the present invention which are represented by General Formula (1). In addition, specific examples thereof also include a form of

an exemplary compound of the compound according to the embodiment of the present invention represented by General Formula (1), in which a substituent capable of being bonded to a biological substance is contained and which is shown as an exemplary compound of the labeled biological substance described later. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention, which is represented by General Formula (1), can be synthesized by a known method except that the compound structure is the structure regulated by General Formula (1). For example, the methods described in US2021/0093737A, WO2009/078970A, and the like can be mentioned.

A compound having a substituent capable of being bonded to a biological substance can be synthesized by a known method except that the compound structure is the structure regulated by General Formula (1). For example, Bioconjugate Techniques (Third Edition, written by Greg T. Hermanson) can be referred to.

«Labeled biological substance»

The labeled biological substance according to the embodiment of the present invention is a substance in which the compound according to the embodiment of the present invention, which is represented by General Formula (1), is bonded to a biological substance. Since the compound according to the embodiment of the present invention, which is represented by General Formula (1), has fluorescence and exhibits an absorption wavelength peak suitable for color development in the near infrared range and an excellent fluorescence intensity, it can be preferably used for a labeled biological substance. The bond between the compound represented by General Formula (1) and a biological substance may have a form in which the compound represented by General Formula (1) and the biological substance are directly bonded or a form in which they are linked through a linking group.

Preferred examples of the biological substance include a protein (including a peptide), an amino acid, a nucleic acid, a sugar chain, and a lipid. Preferred examples of the protein include an antibody, and preferred examples of the lipid include a phospholipid, a fatty acid, sterol, where a phospholipid is more preferable.

Among the above biological substances, the clinically useful substance is not particularly limited, but examples thereof include immunoglobulins such as immunoglobulin (Ig) G, IgM, IgE, IgA, and IgD; plasma proteins such as complement, C-reactive protein (CRP), ferritin, $\alpha_1$ microglobulin, $\beta_2$ microglobulin, and antibodies thereof; tumor markers such as $\alpha$-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotropin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphtheriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELMS, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, styrelidine O), and the like, and antibodies thereof. In addition, antibody fragments such as Fab 2, Fab, and Fv can also be used.

Examples of the specific form in which the compound (hereinafter, also abbreviated as the compound (1)) according to the embodiment of the present invention, which is represented by General Formula (1), and the biological substance interact with each other to be bonded include the forms described below;

i) non-covalent bond (for example, hydrogen bond, ionic bond including chelate formation) or covalent bond between a peptide in the compound (1) and a peptide in the biological substance,
ii) van der Waals force between a long-chain alkyl group in the compound (1) and a lipid bilayer, a lipid, or the like in the biological substance,
iii) an amide bond formed by reacting an N-hydroxysuccinimide ester (NHS ester) in the compound (1) with an amino group in the biological substance,
iv) a thioether bond formed by reacting a maleimide group in the compound (1) with a sulfanyl group (-SH) in the biological substance, and
v) a formation of a triazole ring, which is formed by the Click reaction between an azido group in the compound (1) and an acetylene group in the biological substance, or the Click reaction between an acetylene group in the compound (1) and an azido group in the biological substance.

In addition to the forms of the i) to v), the bond can be formed according to another form, for example, which is described in "Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, p. 62-83". Further, the method described in the same document can be appropriately referred to for the preparation of the labeled biological substance according to the embodiment of the present invention.

Hereinafter, among the compounds according to the embodiment of the present invention, which are represented by General Formula (1), a compound in which at least any one of $R^{11}$, $R^{12}$, or $R^{13}$ has a substituent capable of being bonded to a biological substance, and specific examples of the labeled biological substance according to the embodiment of the present invention, which is obtained from the biological substance that is bonded to the above compound with interaction; however, the present invention is not limited to these compounds. In the following specific examples, a group having a dissociative hydrogen atom such as a sulfo group may adopt a salt structure by a hydrogen atom being dissociated therefrom.

| Compound example | Product (bonding mode) |
|---|---|
| 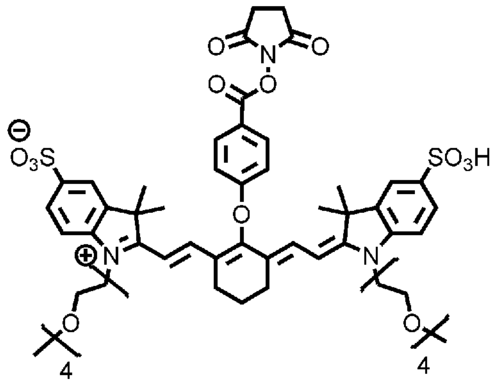<br>NHS ester structure | 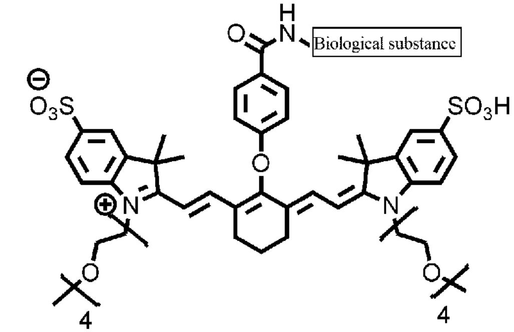<br>Bonding through amino group of biological substance |
| 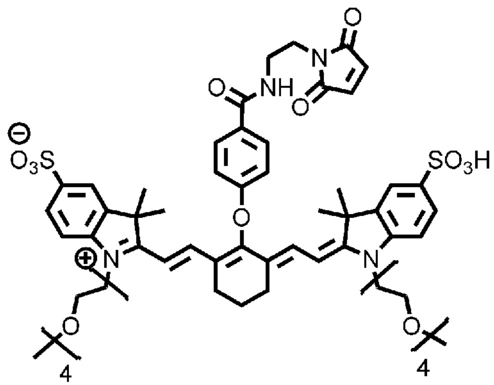<br>Maleimide structure | 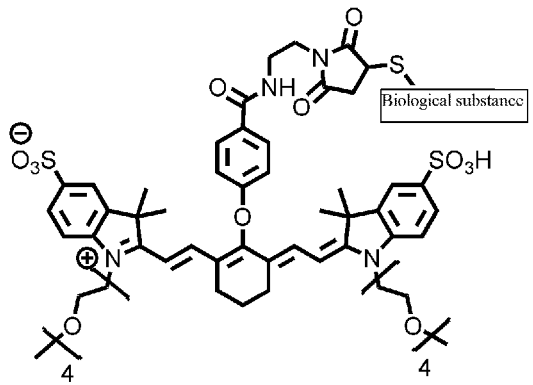<br>Bonding through sulfanyl group of biological substance |

(continued)

| Compound example | Product (bonding mode) |
| --- | --- |
| 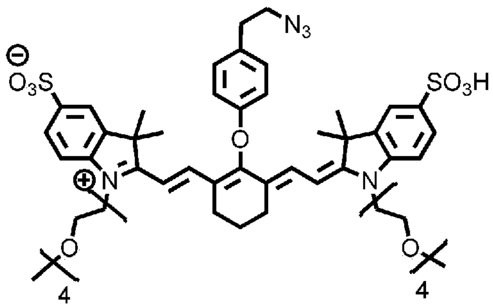<br>Azide structure | 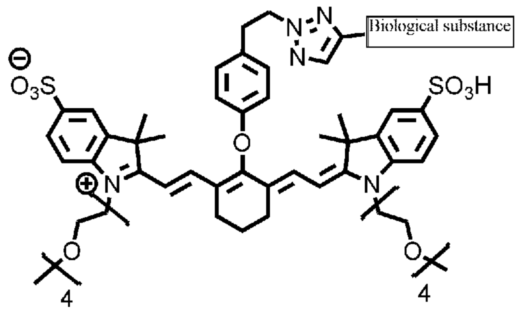<br>Click reaction through acetylene group of biological substance |
| 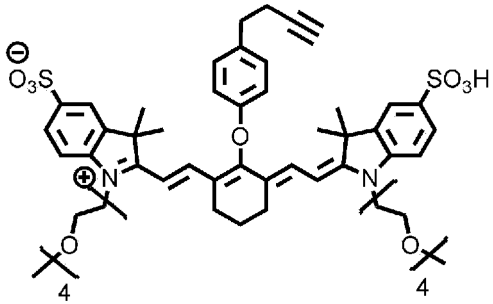<br>Acetylene structure | 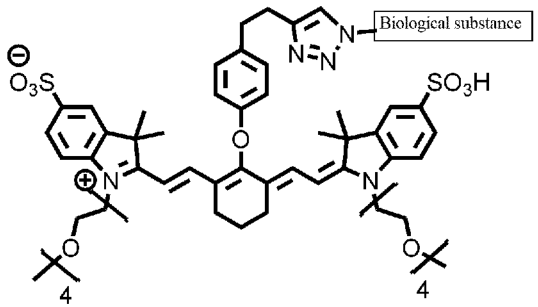<br>Click reaction through azide group of biological substance |

| Compound example | Product (bonding mode) |
|---|---|
| 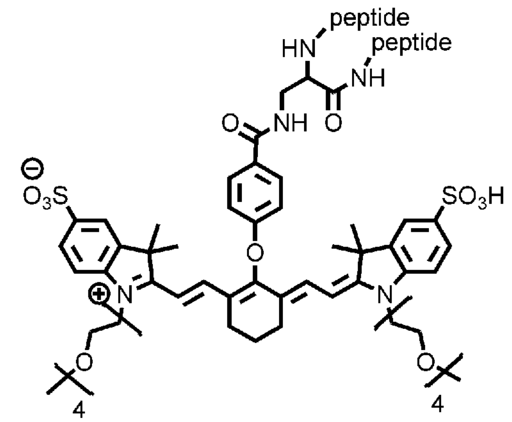 Polypeptide structure (polyamino acid structure) | 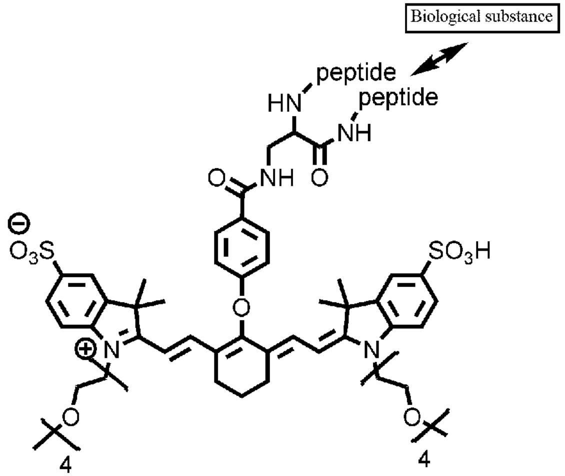 Bonding through peptide of biological substance |
| 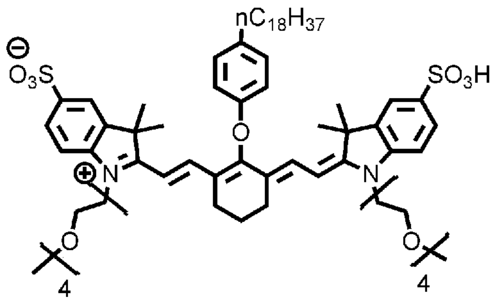 Long-chain alkyl group | Van der Waals force through lipid bilayer, phospholipid, or the like of biological substance |

<Reagent containing labeled biological substance>

In the reagent containing the labeled biological substance according to the embodiment of the present invention, the form of the labeled biological substance according to the embodiment of the present invention, for example, a solution form dissolved in an aqueous medium such as physiological saline and a phosphate buffer solution, and a solid form such as a fine particle powder or a lyophilized powder, is not particularly limited and can be appropriately selected depending on the purpose of use.

For example, in a case where the labeled biological substance according to the embodiment of the present invention is used as a fluorescence labeling reagent, it can be used as a reagent containing the labeled biological substance having any one of the forms described above.

<Use application of labeled biological substance>

The labeled biological substance according to the embodiment of the present invention, obtained from the compound according to the embodiment of the present invention, which is represented by General Formula (1), can exhibit an excellent fluorescence intensity and stably detect fluorescence emitted from the labeled biological substance excited by light irradiation. As a result, the labeled biological substance according to the embodiment of the present invention can be applied to various techniques using the fluorescence labeling, and it can be suitably used, for example, as a fluorescence labeling reagent in a multicolor WB or a reagent for in vivo fluorescence imaging.

The fluorescence detection carried out using the labeled biological substance according to the embodiment of the present invention usually includes the following processes (i) to (iii) or (iv) to (vii). The fluorescence detection including the processes (i) to (iii) corresponds to the direct method using a primary antibody fluorescently labeled with the compound according to the embodiment of the present invention, and the fluorescence detection including the processes (iv) to (vii) corresponds to the indirect method using a secondary antibody fluorescently labeled with the compound according to the embodiment of the present invention.

(i) The process of preparing each of the following (a) and (b)

(a) A sample containing a targeted biological substance (hereinafter, also referred to as a "target biological substance")
(b) A labeled biological substance according to the embodiment of the present invention (hereinafter, also referred to as a "labeled biological substance A according to the embodiment of the present invention") obtained by bonding the biological substance (hereinafter, also referred to as a "primary biological substance") capable of binding to the target biological substance in the above (a) to the compound according to the embodiment of the present invention

(ii) The process of preparing a conjugate (hereinafter, also referred to as a "fluorescently labeled conjugate A") in which the target biological substance in the above (a) is bonded to the primary biological substance in the labeled biological substance A according to the embodiment of the present invention in the above (b)
(iii) The process of irradiating the fluorescently labeled conjugate A with light having the range of the wavelength which is absorbed by the labeled biological substance A according to the embodiment of the present invention, and detecting the fluorescence emitted by the labeled biological substance A according to the embodiment of the present invention
(iv) The process of preparing each of the following (c) to (e)

(c) A sample containing a target biological substance
(d) A biological substance capable of binding to the target biological substance in the above (c) (hereinafter, also referred to as a "primary biological substance")
(e) A labeled biological substance according to the embodiment of the present invention (hereinafter, also referred to as a "labeled biological substance B according to the embodiment of the present invention") obtained by bonding the biological substance (hereinafter, also referred to as a "secondary biological substance") capable of binding to the primary biological substance in the above (d) to the compound according to the embodiment of the present invention (hereinafter, also referred to as a "labeled biological substance B according to the embodiment of the present invention")

(v) The process of preparing a conjugate (hereinafter, also referred to as a "conjugate b") in which the target biological substance in the above (c) is bonded to the primary biological substance of the above (d)
(vi) The process of preparing a conjugate (hereinafter, also referred to as a "fluorescently labeled conjugate B2")

in which the primary biological substance in the conjugate b is bonded to the secondary biological substance in the labeled biological substance B according to the embodiment of the present invention

(vii) The process of irradiating the fluorescently labeled conjugate B2 with light having the range of the wavelength which is absorbed by the labeled biological substance B according to the embodiment of the present invention, and detecting the fluorescence emitted by the labeled biological substance B according to the embodiment of the present invention

Examples of the biological substance (the primary biological substance) capable of binding to the target biological substance and the biological substance (the secondary biological substance) capable of binding to the primary biological substance include the biological substances in the labeled biological substance according to the embodiment of the present invention. The above biological substance can be appropriately selected in accordance with the target biological substance (a biological substance in the test object) or the primary biological substance, and a biological substance capable of specifically binding to the biological substance in the test object or to the primary biological substance can be selected.

Examples of the protein among the target biological substances include a protein, which is a so-called disease marker. The disease marker is not particularly limited, and examples thereof include α-fetoprotein (AFP), protein induced by vitamin K absence or antagonist II (PIVKA-II), breast carcinoma-associated antigen (BCA) 225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, γ-seminoprotein (γ-Sm), prostate specific antigen (PSA), prostatic acid phosphatase (PAP), nerve specific enolase (NSE), Iba1, amyloid β, tau, flotillin, squamous cell carcinoma associated antigen (SCC antigen), sialyl LeX-i antigen (SLX), SPan-1, tissue polypeptide antigen (TPA), serial Tn antigen (STN), cytokeratin (CYFRA) pepsinogen (PG), C-reactive protein (CRP), serum amyloid A protein (SAA), myoglobin, creatine kinase (CK), troponin T, and ventricular muscle myosin light chain I.

The target biological substance may be a bacterium. Examples of the bacterium include a bacterium to be subjected to a cellular and microbiological test, which is not particularly limited. Specific examples thereof include Escherichia coli, Salmonella, Legionella, and bacteria causing problems in public health.

The target biological substance may be a virus. Although the virus is not particularly limited, examples of the virus include hepatitis virus antigens such as hepatitis C and B virus antigens, p24 protein antigen of HIV virus, and pp65 protein antigen of cytomegalovirus (CMV), and E6 and E7 proteins of human papillomavirus (HPV).

The target biological substance may be a cell, and the cell is not limited to a living cell and may be a dead cell.

In the above (i) or (iv), the sample containing the target biological substance is not particularly limited and can be prepared according to a conventional method.

In addition, the labeled biological substance according to the embodiment of the present invention is not particularly limited and can be prepared by bonding a biological substance capable of binding to a target biological substance to the compound according to the embodiment of the present invention, according to a conventional method. The form of the bond and the reaction to form the bond are as described above in the labeled biological substance according to the embodiment of the present invention.

In the above (v), the target biological substance may be directly bonded to the primary biological substance or may be bonded through another biological substance which is different from the target biological substance and the primary biological substance. Further, in the above (vi), the primary biological substance in the conjugate b may be directly bonded to the secondary biological substance in the labeled biological substance B according to the embodiment of the present invention or may be bonded through another biological substance which is different from the primary biological substance and the secondary biological substance.

The labeled biological substance according to the embodiment of the present invention can be used as a fluorescently labeled antibody in both the direct method and the indirect method but is preferably used as a fluorescently labeled antibody in the indirect method.

In the above (ii) or (v) and the above (vi), the binding of the labeled biological substance or the like according to the embodiment of the present invention to the target biological substance is not particularly limited and can be carried out according to a conventional method.

In the above (iii) or (vii), the wavelength for exciting the labeled biological substance according to the embodiment of the present invention is not particularly limited as long as the wavelength is a luminescence wavelength (wavelength light) capable of exciting the labeled biological substance according to the embodiment of the present invention.

Since the labeled biological substance using a compound in which n is 1 among the compounds (1) according to the embodiment of the present invention has an absorption maximum wavelength in the vicinity of 585 nm (specifically, a wavelength range of 560 to 600 nm), the range of the wavelength of light to be emitted is preferably 530 to 650 nm and more preferably 550 to 630 nm. The labeled biological substance using this compound can be suitably used as a labeled biological substance that exhibits an excellent fluorescence intensity with respect to an excitation light source for light in the vicinity of 600 nm in the visible range.

Since the labeled biological substance using a compound in which n is 2 among the compounds (1) according to the embodiment of the present invention has an absorption maximum wavelength in the vicinity of 685 nm (specifically, a wavelength range of 620 to 700 nm), the range of the wavelength of light to be emitted is preferably 630 to 750 nm and more preferably 650 to 730 nm. The labeled biological substance using this compound can be suitably used as a labeled biological substance that exhibits an excellent fluorescence intensity with respect to an excitation light source for light in the vicinity of 700 nm in the near infrared range of the multicolor WB.

Since the labeled biological substance using a compound in which n is 3 among the compounds (1) according to the embodiment of the present invention has an absorption maximum wavelength in the vicinity of 785 nm (specifically, a wavelength range of 740 to 830 nm), the range of the wavelength of light to be emitted is preferably 700 to 850 nm and more preferably 730 to 830 nm. The labeled biological substance using this compound can be suitably used as a labeled biological substance that exhibits an excellent fluorescence intensity with respect to an excitation light source for light in the vicinity of 800 nm in the near infrared range of the multicolor WB.

The fluorescence excitation light source used in the present invention is not particularly limited as long as it emits light having an luminescence wavelength (wavelength light) capable of exciting the labeled biological substance according to the embodiment of the present invention, and for example, various laser light sources can be used. In addition, various optical filters can be used to obtain a preferred excitation wavelength or detect only fluorescence.

Other matters in the above (i) to (vii) are not particularly limited, and conditions of a method, a reagent, a device, and the like, which are generally used in the fluorescence detection using fluorescence labeling, can be appropriately selected.

Further, regarding the processes other than the above (i) to (vii) as well, conditions of a method, a reagent, a device, and the like, which are generally used, can be appropriately selected in accordance with various methods using fluorescence labeling.

For example, in the multicolor WB using the labeled biological substance according to the embodiment of the present invention, it is possible to detect a target biological substance with excellent fluorescence intensity by preparing a blotted membrane according to a method generally used for a target biological substance (protein separation by electrophoresis, blotting to a membrane, and blocking of a membrane) and using the labeled biological substance according to the embodiment of the present invention as a labeled antibody (preferably, as a secondary antibody).

- Substituent group T -

In the present invention, the preferred substituents include those selected from the following substituent group T.

In addition, in the present invention, in a case where it is simply described as a substituent, the substituent refers to the substituent group T, and in a case where an individual group, for example, an alkyl group is only described, a corresponding group in the substituent group T is preferably applied.

Further, in the present specification, in a case where an alkyl group is described separately from a cyclic (cyclo)alkyl group, the alkyl group is used to include a linear alkyl group and a branched alkyl group. On the other hand, in a case where an alkyl group is not described separately from a cyclic alkyl group, and unless otherwise specified, the alkyl group is used to include a linear alkyl group, a branched alkyl group, and a cycloalkyl group. This also applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure,

In the following description for the substituent group T, a group having a linear or branched structure and a group having a cyclic structure, such as an alkyl group and a cycloalkyl group, are sometimes described separately for clarity.

The groups included in the substituent group T include the following groups.

An alkyl group (preferably having 1 to 30 carbon atoms, more preferably 1 to 20 carbon atoms, still more preferably 1 to 12 carbon atoms, still more preferably 1 to 8 carbon atoms, still more preferably 1 to 6 carbon atoms, and particularly preferably 1 to 3 carbon atoms), an alkenyl group (preferably having 2 to 30 carbon atoms, more preferably 2 to 20 carbon atoms, still more preferably 2 to 12 carbon atoms, still more preferably 2 to 6 carbon atoms, and even still more preferably 2 to 4 carbon atoms), an alkynyl group (preferably having 2 to 30 carbon atoms, still more preferably 2 to 20 carbon atoms, still more preferably 2 to 12 carbon atoms, still more preferably 2 to 6 carbon atoms, and even still more preferably 2 to 4 carbon atoms), a cycloalkyl group (preferably having 3 to 20 carbon atoms), a cycloalkenyl group (preferably having 5 to 20 carbon atoms), an aryl group (it may be a monocyclic group or may be a fused ring group (preferably a fused ring group of 2 to 6 rings); in a case of a fused ring group, it consists of a 5- to 7-membered ring; and the aryl group preferably has 6 to 40 carbon atoms, more preferably 6 to 30 carbon atoms, still more preferably 6 to 26 carbon atoms, and particularly preferably 6 to 10 carbon atoms), a heterocyclic group (it has, as a ring-constituting

atom, at least one of a nitrogen atom, oxygen atom, a sulfur atom, a phosphorus atom, a silicon atom, or selenium atom, may be a monocyclic ring, or may be a fused ring group (preferably a fused ring group of 2 to 6 rings); in a case of a monocyclic group, the monocyclic ring is preferably a 5- to 7-membered ring and more preferably a 5-membered or 6-membered ring; the heterocyclic group preferably has 2 to 40 carbon atoms and more preferably 2 to 20 carbon atoms; and the heterocyclic group includes an aromatic heterocyclic group (a heteroaryl group) and an aliphatic heterocyclic group (an aliphatic heterocyclic group), an alkoxy group (preferably having 1 to 20 carbon atoms, and more preferably having 1 to 12 carbon atoms), an alkenyloxy group (preferably having 2 to 20 carbon atoms, and more preferably having 2 to 12 carbon atoms), and an alkynyloxy group (preferably having 2 to 20 carbon atoms, and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms), a polyalkyleneoxy group (preferably having 2 to 40 carbon atoms and more preferably 2 to 20 carbon atoms).

An alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; the amino group includes an unsubstituted amino group ($-NH_2$), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, or a (mono- or di-) heterocyclic amino group, where each of the above groups substituting an unsubstituted amino group has the same definition as the corresponding group in the substituent group T), a sulfamoyl group (preferably having 0 to 20 carbon atoms; the sulfamoyl group is preferably an alkyl, cycloalkyl, or aryl sulfamoyl group), an acyl group (preferably having 1 to 20 carbon atoms, and more preferably having 2 to 15 carbon atoms), an acyloxy group (preferably having 1 to 20 carbon atoms), a carbamoyl group (preferably having 1 to 20 carbon atoms; the carbamoyl group is preferably an alkyl, cycloalkyl, or aryl carbamoyl group).

An acylamino group (preferably having 1 to 20 carbon atoms, for example, acetylamino, cyclohexylcarbonylamino, or benzoylamino), an amide group (an aminocarbonyl group), a sulfonamide group (it may be any one of a sulfonylamino group or a sulfamoyl group; and it is preferably an alkyl, cycloalkyl, or aryl sulfonamide group which preferably having 0 to 20 carbon atoms), an alkylthio group (preferably having 1 to 20 carbon atoms and more preferably 1 to 12 carbon atoms), a cycloalkylthio group (preferably having 3 to 20 carbon atoms), an arylthio group (preferably having 6 to 40 carbon atoms, more preferably 6 to 26 carbon atoms, and still more preferably 6 to 14 carbon atoms), a heterocyclic thio group (preferably having 2 to 20 carbon atoms), an alkyl, cycloalkyl, or aryl sulfonyl group (preferably having 1 to 20 carbon atoms).

A silyl group (preferably having 1 to 30 carbon atoms and more preferably 1 to 20 carbon atoms, and preferably substituted with an alkyl, aryl, alkoxy, or aryloxy), a silyloxy group (preferably having 1 to 20 carbon atoms and preferably substituted with an alkyl, aryl, alkoxy, or aryloxy), a hydroxy group, a cyano group, a nitro group, a halogen atom (for example, a fluorine atom, a chlorine atom, a bromine atom or an iodine atom), an oxygen atom (specifically replacing $>CH_2$ which constitutes a ring with >C=O), a carboxy group ($-CO_2H$), a phosphono group [$-PO(OH)_2$], a phosphoryl group [$-O-PO(OH)_2$], a sulfo group ($-SO_3H$), a boric acid group [$-B(OH)_2$], an onio group (an ammonio group including a cyclic ammonio group, which contains a sulfonio group or a phosphonio group, and preferably has 0 to 30 carbon atoms and more preferably 1 to 20 carbon atoms), a sulfanyl group (-SH), an amino acid residue, or a polyamino acid residue.

In addition, examples thereof include the above-described alkyl group, alkenyl group, alkynyl group, cycloalkyl group, cycloalkenyl group, aryl group, heterocyclic group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and an alkyl, cycloalkyl, and aryl sulfonyl group, which have, as a substituent, a carboxy group, a phosphono group, a sulfo group, an onio group, an amino acid residue, a polyamino acid residue, or a $-(CH_2-CH_2-O)_m$-alkyl group (m has the same meaning as m in $R^1$ to $R^6$).

The substituent selected from the substituent group T is more preferably an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a cyano group or a halogen atom, and particularly preferably an alkyl group, an alkenyl group, an aryl group, a heterocyclic group, an alkoxy group, an alkoxycarbonyl group, an amino group, an acylamino group, or a cyano group.

The substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring moiety, and may be substituted or unsubstituted.

## Examples

Hereinafter, the present invention will be described in more detail based on Examples, but the present invention is not limited thereto. It is noted that room temperature means 25°C.

Compounds (1) to (20) represented by General Formula (1), comparative compounds (1) to (5), and Alexa Fluor 647 are shown below.

The compounds (1) to (4) and (17) to (20) are compounds of the first preferred form according to the present invention, which have an excitation absorption wavelength at 740 to 830 nm, the compounds (5) to (7) are compounds of the second preferred form according to the present invention, which have an excitation absorption wavelength at 620 to 700 nm, and the compounds (8) to (16) are compounds of the third preferred form according to the present invention, which have an excitation absorption wavelength at 620 to 700 nm.

It is noted that the sulfo group represented by $-SO_3H$ in the compounds of Examples and the comparative compounds may partially adopt, in the purification step or the like, a structure including a salt structure (for example, a potassium salt, a sodium salt, a triethylamine (TEA) salt, or an N,N-diisopropylethylamine (DIPEA) salt). In addition, in Examples and Comparative Compounds, a number attached to the parentheses enclosing an ethyleneoxy structure means an average repetition number. All of the compounds were synthesized using a compound in which the first decimal place of the average repetition number was 0 as a raw material.

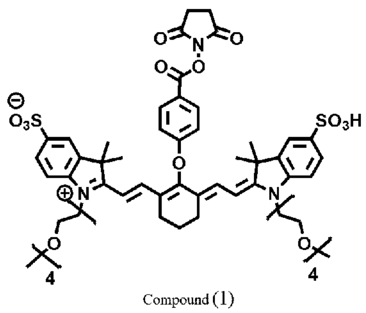

Compound (1)

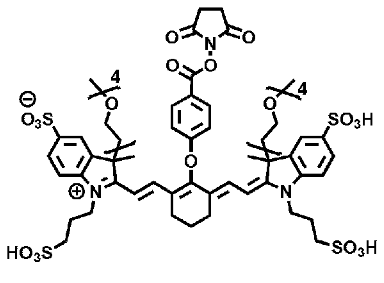

Compound (2)

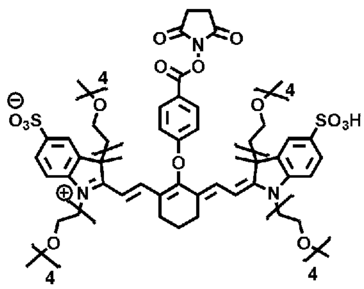

Compound (3)

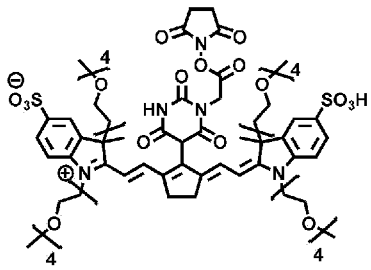

Compound (4)

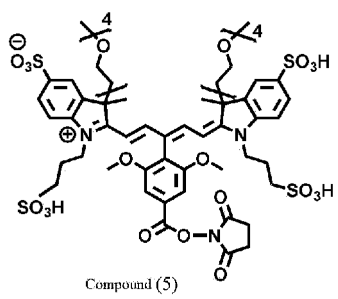

Compound (5)

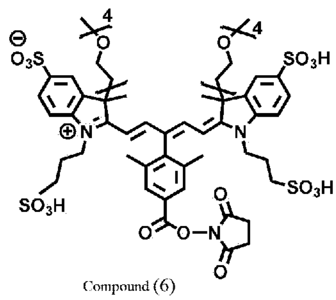

Compound (6)

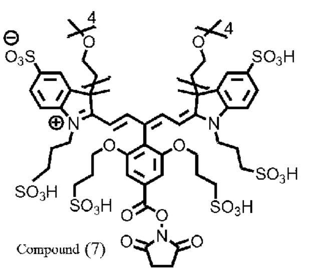

Compound (7)

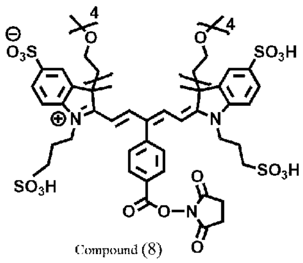

Compound (8)

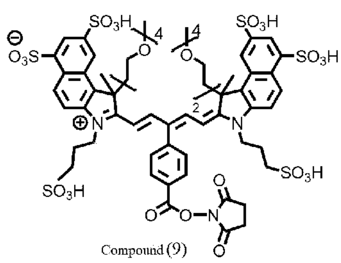

Compound (9)

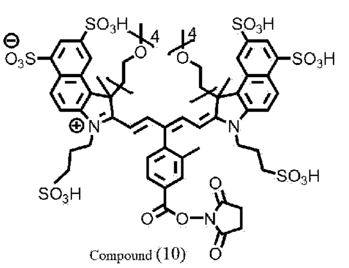
Compound (10)

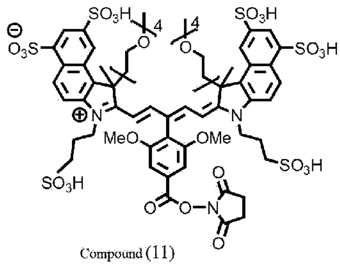
Compound (11)

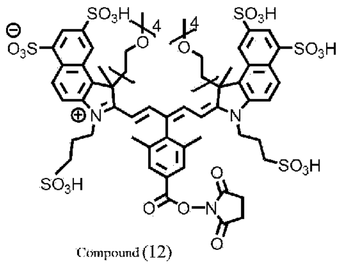
Compound (12)

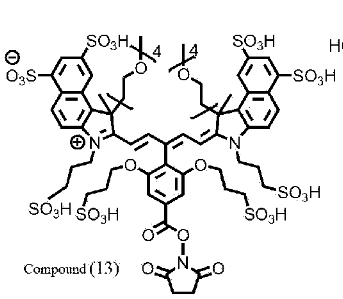
Compound (13)

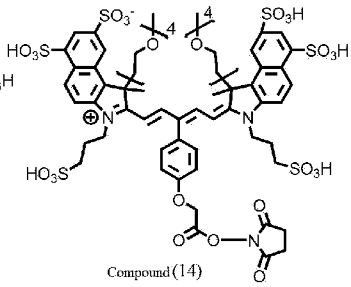
Compound (14)

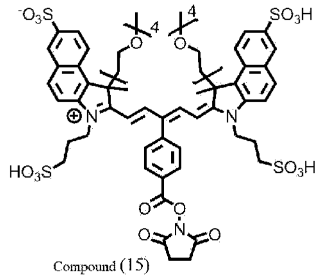
Compound (15)

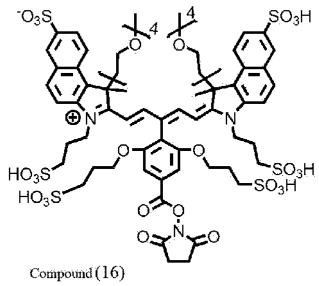
Compound (16)

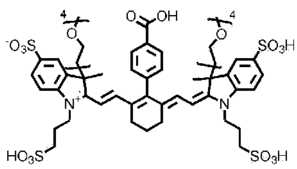
Compound (17)

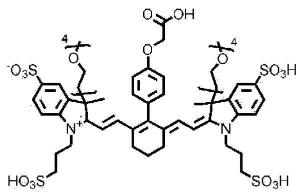
Compound (18)

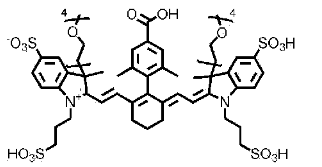
Compound (19)

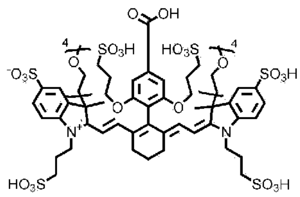
Compound (20)

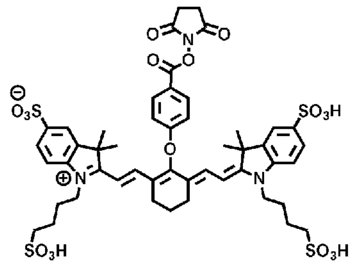

Comparative compound (1)

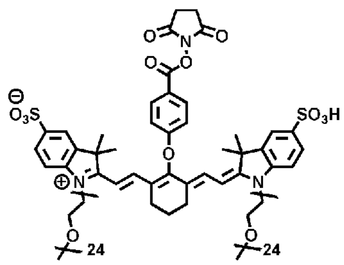

Comparative compound (2)

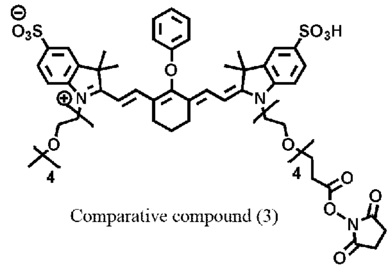

Comparative compound (3)

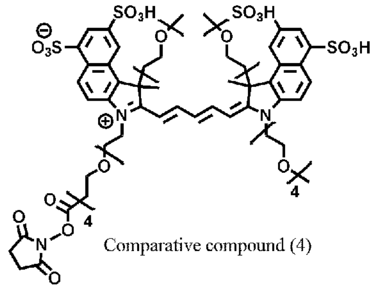

Comparative compound (4)

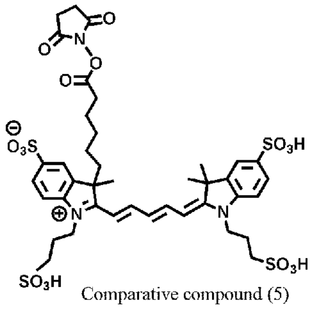

Comparative compound (5)

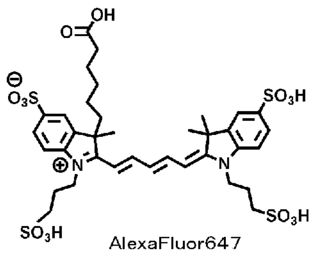

AlexaFluor647

The comparative compound (1) is the compound described in the specification of US2021/0093737A.

The comparative compound (2) is an NHS ester derivative of the compound described in WO2009/078970A.

The comparative compound (5) is an NHS ester derivative of Alexa Fluor 647 (product name, product number: A33084, manufactured by Thermo Fisher Scientific, Inc.), which is presumed to have the above-described chemical structure.

The methods for synthesizing the compounds (1) to (16) and the comparative compounds (1) to (4) used in each Example are described in detail below, but the starting materials, the dye intermediates, and the synthetic routes are not limited thereto.

In the following synthetic route, room temperature means 25°C.

Unless otherwise specified, SNAP Ultra C18 (product name, manufactured by Biotage, LLC) or Sfar C18 (product name, manufactured by Biotage, LLC) was used as the carrier in the reverse phase column chromatography, and Hi-Flash Column (product name, manufactured by Yamazen Corporation) was used as a carrier in the normal phase column chromatography.

The mixing ratio in the eluent used in the reverse phase column chromatography or the normal phase column chromatography is in terms of the volume ratio. For example, "acetonitrile:water = from 0:100 to 20:80" means that the eluent of "acetonitrile:water = 0:100" was changed to an eluent of "acetonitrile:water = 20:80".

For the preparative high performance liquid chromatography (HPLC), 2767 (product name, manufactured by Waters Corporation) was used.

The MS spectrum was measured by ACQUITY SQD LC/MS System [product name, manufactured by Waters Corporation, ionization method: electrospray Ionization (ESI)] or LCMS-2010EV [product name, manufactured by Shimadzu Corporation, ionization method: an ionization method simultaneously carrying out ESI and atomospheric pressure chemical ionization (APCI)].

<Synthesis of compound (1)>

A compound (1) was synthesized based on the following scheme.

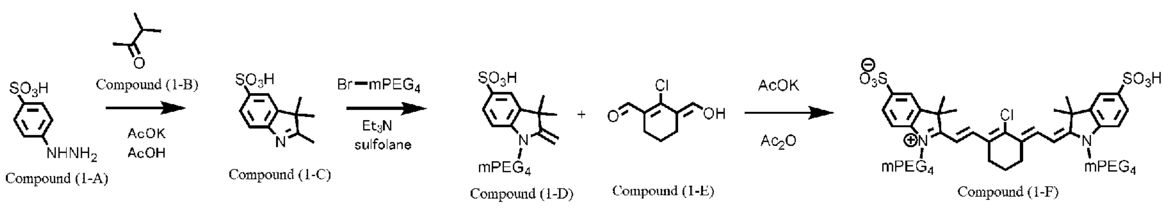

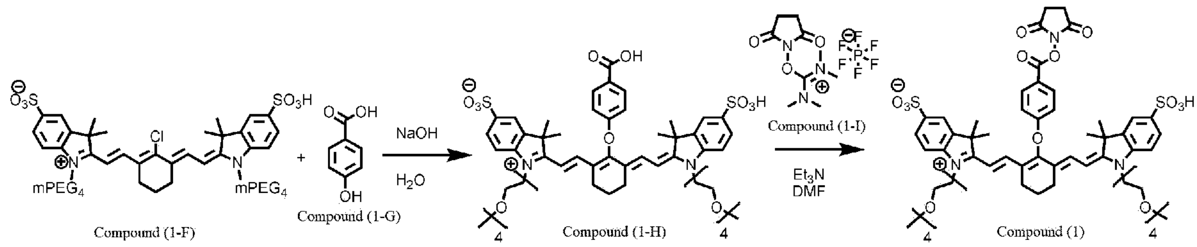

Compound (1-F) Compound (1-G) Compound (1-H) Compound (1-I) Compound (1)

1) Synthesis of compound (1-C)

1.9 g of the compound (1-A), 20 ml of acetic acid (AcOH), 1.3 g of the compound (1-B), and 0.98 g of potassium acetate (AcOK) were placed in an eggplant flask having a capacity of 200 ml and stirred at 140°C for 1 hour in a nitrogen atmosphere. The solvent was distilled off under reduced pressure, the purification was carried out by reverse phase column chromatography (eluent: acetonitrile/water = from 0/100 to 35/65) to obtain 0.99 g of a compound (1-C).

2) Synthesis of compound (1-D)

478 mg of the compound (1-C), 1.08 g of $mPEG_4$-Br, 2 mL of sulfolane, and 0.132 ml of triethylamine ($Et_3N$) were added in an eggplant flask having a capacity of 50 ml and heated and stirred at 120°C for 6 hours. 20 mL of ethyl acetate/hexane (1/1) was added to the reaction solution to cause precipitation. The precipitate was purified by reverse phase column chromatography (eluent: acetonitrile/water = from 0/100 to 20/100) to obtain 369 mg of a compound (1-D).

3) Synthesis of compound (1-F)

214 mg of the compound (1-D), 43 mg of the compound (1-E), 25 mg of potassium acetate (AcOK), and 1 mL of acetic anhydride (AczO) were added in a test tube, and stirring was carried out at 60°C for 2 hours in a nitrogen atmosphere. After the reaction was settled, distilled water was added, and purification was carried out by reverse phase column chromatography (eluent: acetonitrile/water = from 0/100 to 25/75) to obtain 170 mg of a compound (1-F).

4) Synthesis of compound (1-H)

10 mg of the compound (1-F) and 300 μL of distilled water were added in a test tube, and stirring was carried out at 95°C. A solution obtained by mixing 3 mg of a compound (1-G) and 5 mg of sodium hydroxide in 200 μL of distilled water was added dropwise to this solution, and stirring was carried out at 95°C for 30 minutes. The reaction solution was cooled to room temperature and purified by preparative HPLC, and freeze drying was followed to obtain 6.0 mg of a compound (1-H). The results of the MS measurement of the compound (1-H) were as follows.
MS (ESI m/z):$(M + H^+)^+$ = 1,097, $(M - H^+)^-$ = 1,095

5) Synthesis of compound (1)

0.30 ml of N,N-dimethylformamide (DMF) and 1 mg of the compound (1-I) were dissolved in 3.0 mg of the compound (1-H), and then stirring was carried out for 1 hour. Then, the solvent was distilled off under reduced pressure, 1.5 mL of ethyl acetate was added, the supernatant was removed, and vacuum drying was carried out to obtain a compound (1).

<Synthesis of compound (2)>

A compound (2) was synthesized in the same manner as the compound (1), based on the following scheme. The results of the MS measurement of the compound (2-G) were as follows.
MS (ESI m/z):$(M + H^+)^+$ = 1,313

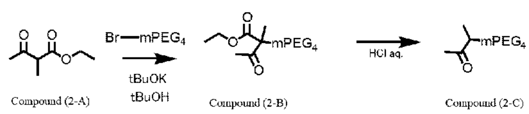

Compound (2-A) Compound (2-B) Compound (2-C)

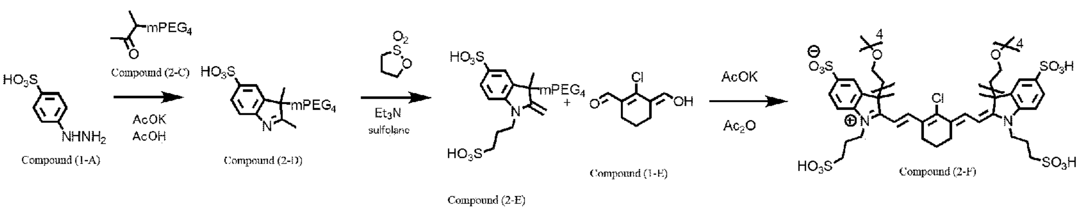


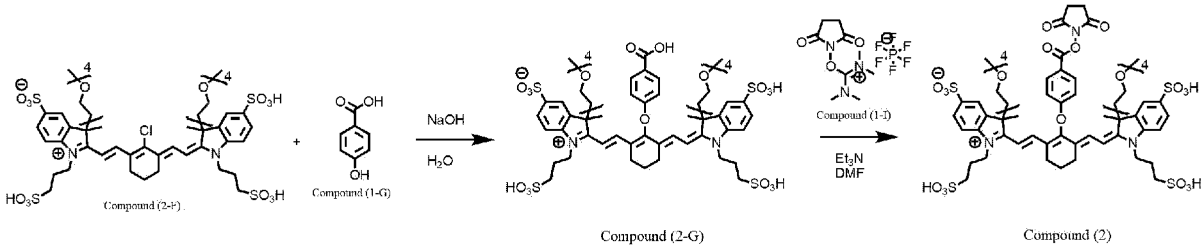


A compound (2-C) was synthesized as follows.

200 ml of tert-butanol (tBuOH) and 12 g of potassium tert-butoxide (tBuOK) were placed in a nitrogen-substituted three-necked flask having a capacity of 500 ml, and while stirring the resultant mixture, 14.4 g of the compound (2-A) was added dropwise thereto, and stirring was carried out for a while. Next, 32.5 g of triethylene glycol 2-bromoethyl methyl ether (Br-mPEG$_4$) was added dropwise thereto, and heating and stirring were carried out. After stirring at 80°C for 1 hour, the solvent was distilled off under reduced pressure, a liquid separation operation was carried out with ethyl acetate and distilled water, and a crude product was extracted with distilled water. 30 ml of a 30% hydrochloric acid aqueous solution was added to the obtained crude product, and stirring was carried out at 100°C for 3 hours. Then, the solvent was distilled off under reduced pressure, and purification was carried out by normal phase column chromatography (eluent: hexane/ethyl acetate = from 50/50 to 30/70) to obtain 11.0 g of a compound (2-C).

<Synthesis of compound (3)>

A compound (3) was synthesized in the same manner as the compound (1), based on the following scheme. The results of the MS measurement of the compound (3-C) were as follows.
MS (ESI m/z):$(M + H^+)^+$ = 1,450, $(M - H^+)^-$ = 1,448

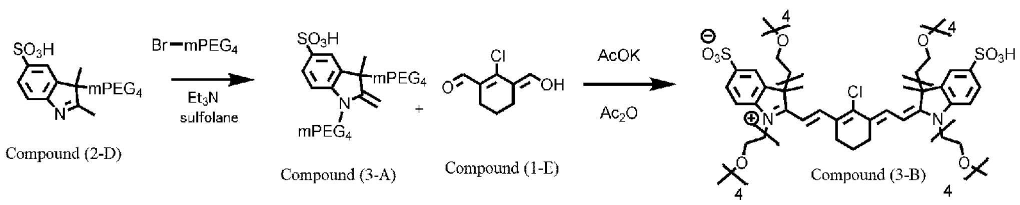


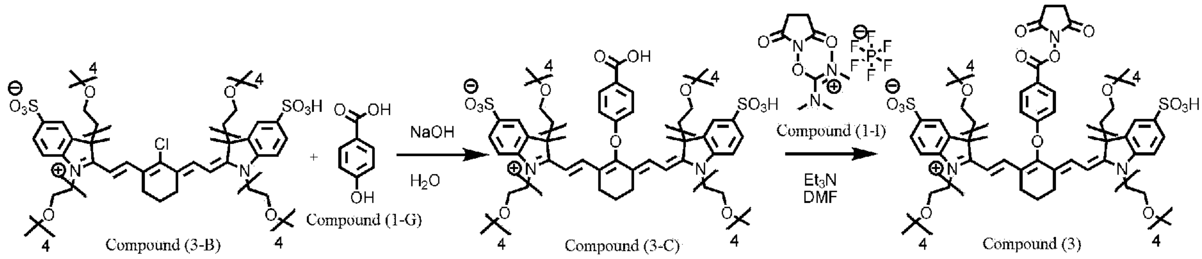


<Synthesis of compound (4)>

A compound (4) was synthesized in the same manner as the compound (1), based on the following scheme. The results of the MS measurement of the compound (4-F) were as follows.

MS (ESI m/z):$(M + H^+)^+$ = 1,484, $(M - H^+)^-$ = 1,482

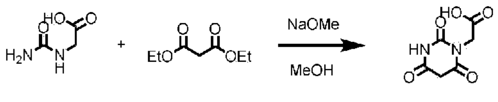


Compound (4-A) Compound (4-B) Compound (4-C)

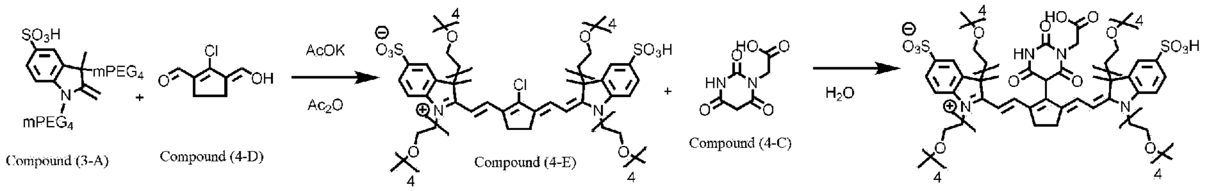


Compound (3-A) Compound (4-D) Compound (4-E) Compound (4-C) Compound (4-F)

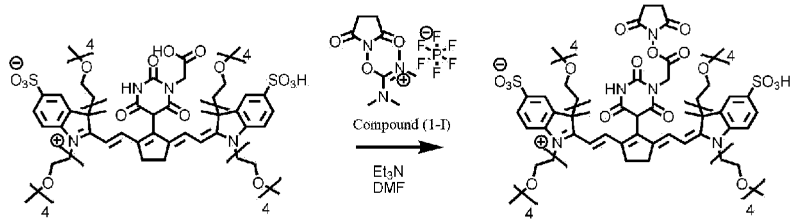


Compound (4-F) Compound (4)

A compound (4-C) was synthesized as follows.

500 mg of the compound (4-A), 1.28 mL of the compound (4-B), 0.8 g of sodium methoxide, and 10 mL of methanol were placed in a nitrogen-substituted flask having a capacity of 50 ml, and the resultant mixture was heated and refluxed for 3 hours. After cooling to room temperature, 0.52 g of the compound (4-C) was obtained by filtration.

<Synthesis of compound (5)>

A compound (5) was synthesized based on the following scheme.

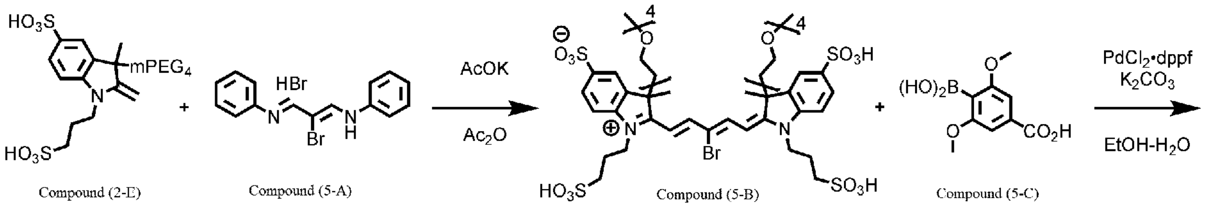


Compound (2-E) Compound (5-A) Compound (5-B) Compound (5-C)

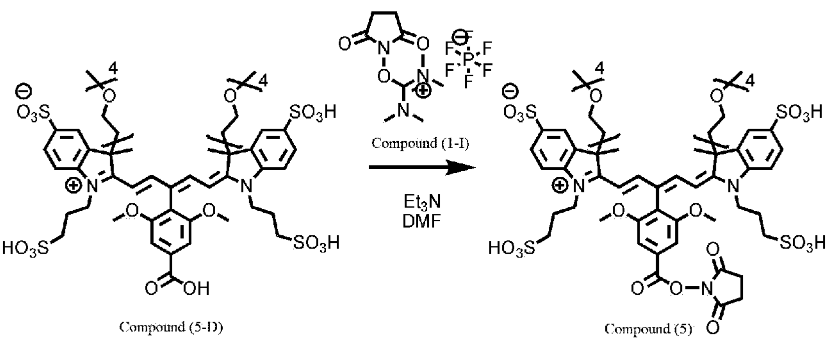


Compound (5-D) Compound (5)

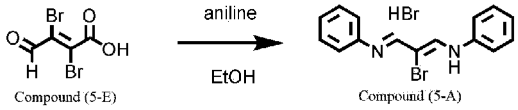


Compound (5-E) Compound (5-A)

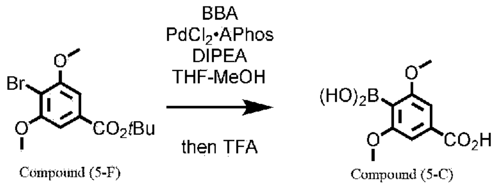


Compound (5-F) Compound (5-C)

1) Synthesis of compound (5-B)

94 mg of the compound (2-E), 34 mg of the compound (5-A), 80 mg of potassium acetate (AcOK), and 4.5 mL of acetic anhydride (AczO) were added in a 5 ml eggplant flask, and stirring was carried out at 60°C for 2.5 hours in a nitrogen atmosphere. The reaction solution was added to ethyl acetate to cause precipitation. The precipitate was purified by reverse phase column chromatography (eluent: acetonitrile/water = from 0/100 to 10/90) to obtain 46 mg of a compound (5-B). The results of the MS measurement of the compound (5-B) were as follows.
MS (ESI m/z):(M + H$^+$)$^+$ = 1,190, 1,192

2) Synthesis of compound (5-D)

30 mg of the compound (5-B), 28 mg of the compound (5-C), 21 mg of [1,1'-bis(diphenylphosphino)ferrocene]palladium (II) dichloride dichloromethane adduct, 35 mg of potassium carbonate, 1.26 ml of ethanol, and 0.126 ml of distilled water were added in a test tube, and the resultant mixture was heated and stirred at 55°C for 30 minutes in a nitrogen atmosphere. The solvent was distilled off under reduced pressure, the purification was carried out by reverse phase column chromatography (eluent: acetonitrile/water = from 0/100 to 20/100) and preparative HPLC, and freeze drying was followed to obtain 2.6 mg of a compound (5-D). The results of the MS measurement of the compound (5-D) were as follows.
MS (ESI m/z):(M + H$^+$)$^+$ = 1,291

3) Synthesis of compound (5)

0.10 ml of N,N-dimethylformamide (DMF), 0.001 ml of triethylamine, and 1 mg of the compound (1-I) were dissolved in 0.9 mg of the compound (5-D), and then stirring was carried out for 1 hour. Then, the solvent was distilled off under reduced pressure, 1 mL of ethyl acetate/hexane (1/1) was added, the supernatant was removed, and vacuum drying was carried out to obtain a compound (5).
A compound (5-A) was synthesized as follows.
2.89 g of the compound (5-E) and 15 ml of ethanol were added in a three-necked eggplant flask having a capacity of 100 ml. A mixture of 2.05 ml of aniline and 15 ml of ethanol was added dropwise thereto under ice-cooling, and then the mixture was heated and stirred at 60°C for 4 hours. After the reaction was settled, the solvent was distilled off under reduced pressure until the amount thereof was reduced to half, and then followed by transferring to an ice-saline bath and stirring. The resulting solid was filtered and dried to obtain 2.55 g of the compound (5-A). The results of the MS measurement of the compound (5-A) were as follows.
MS (ESI m/z):(M + H$^+$)$^+$ = 301, 303
A compound (5-C) was synthesized as follows.
316 mg of the compound (5-F), 141 mg of tetrahydroxydiboron, 1.5 mg of dichlorobis[di-t-butyl(p-dimethylaminophenyl)phosphino] palladium (II), 3.2 ml of tetrahydrofuran, 1.6 ml of methanol, and 0.348 ml of N,N-diisopropylethylamine were added in a three-necked eggplant flask having a capacity of 50 ml. After heating and stirring at 55°C for 2 hours, the solvent was distilled off under reduced pressure. 3.2 ml of trifluoroacetic acid was added to the residue, and stirring was carried out at room temperature for 15 minutes. After the reaction was settled, the solvent was distilled off under reduced pressure, and then the purification was carried out by reverse phase column chromatography (eluent: acetonitrile/water = from 0/100 to 10/90), and freeze drying was followed to obtain 93 mg of a compound (5-C). The results of the MS measurement of the compound (5-C) were as follows.
MS (ESI m/z):(M + H$^+$)$^+$ = 227, (M - H$^+$)$^-$ = 225

<Synthesis of compound (6)>

A compound (6) was synthesized in the same manner as the compound (5), based on the following scheme. The results of the MS measurement of the compound (6-B) were as follows.
MS (ESI m/z):(M + H$^+$)$^+$ = 1,259

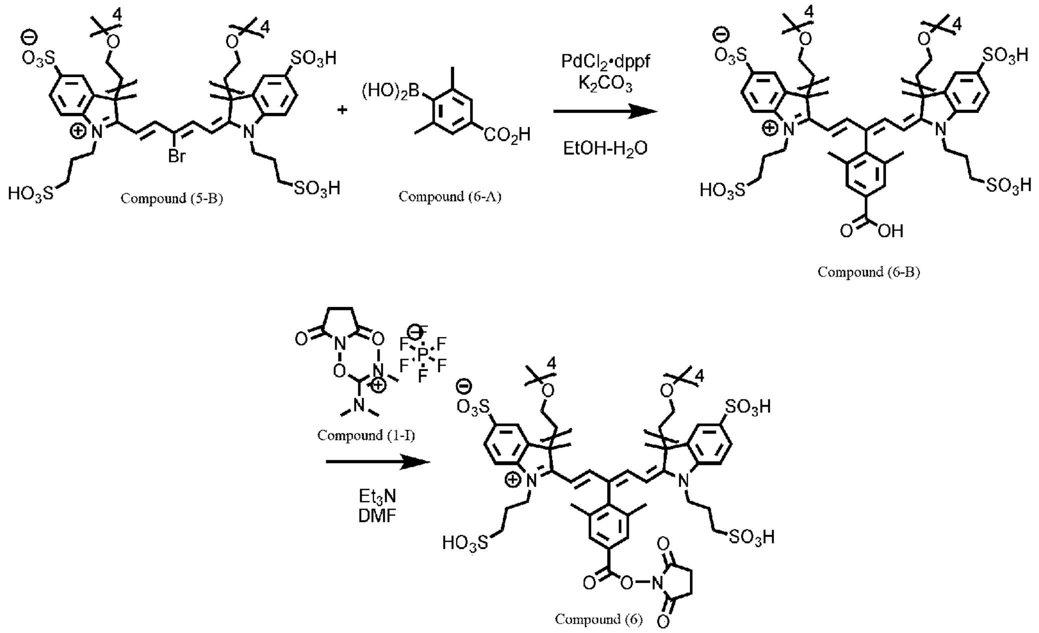


Compound (5-B) Compound (6-A) Compound (6-B) Compound (1-I) Compound (6)

<Synthesis of compound (7)>

A compound (7) was synthesized in the same manner as the compound (5), based on the following scheme.

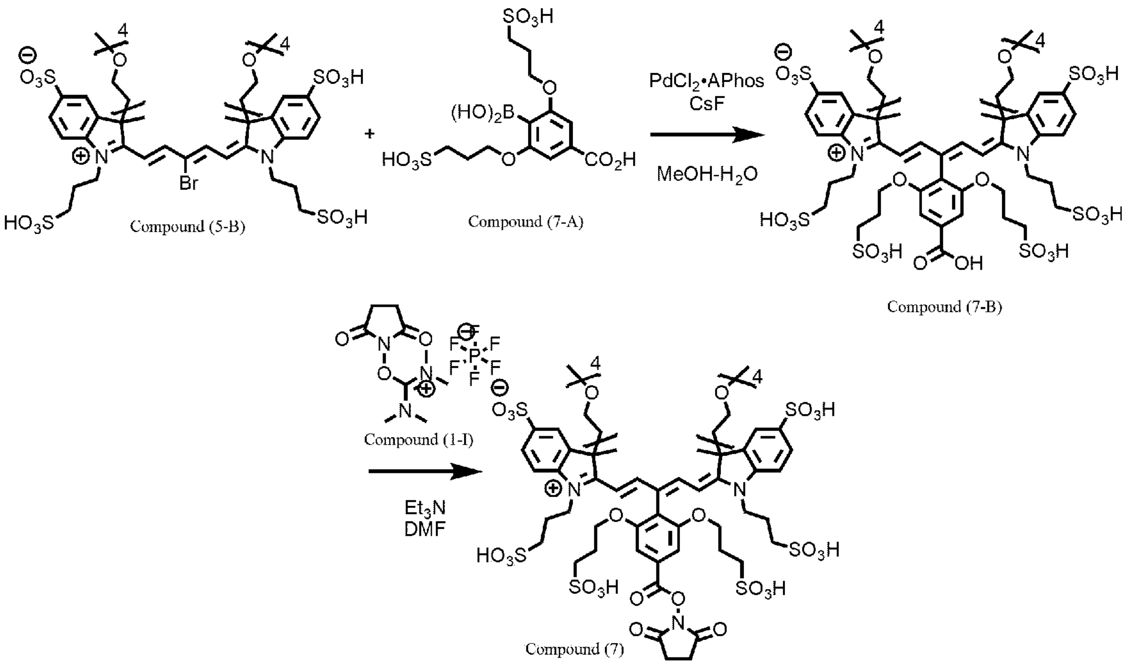


Compound (5-B) Compound (7-A) Compound (7-B) Compound (1-I) Compound (7)

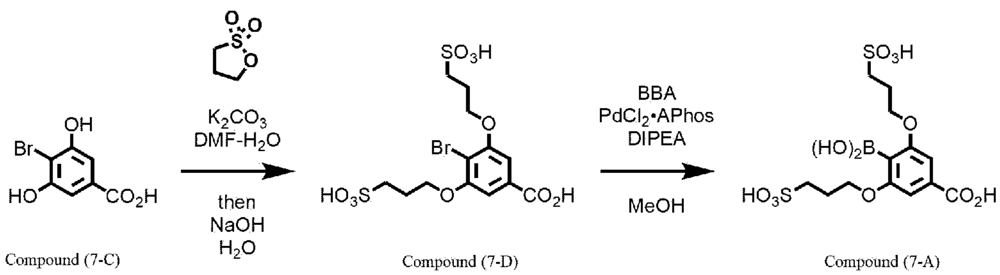


Compound (7-C) Compound (7-D) Compound (7-A)

A compound (7-D) was synthesized as follows.

500 mg of the compound (7-C), 1.20 g of potassium carbonate ($K_2CO_3$), 0.95 ml of 1,3-propane sultone, 20 ml of N,N-dimethylformamide (DMF), and 10 ml of distilled water added in an eggplant flask having a capacity of 200 ml. After heating and stirring at 80°C for 1 hour, 0.57 ml of 1,3-propane sultone was added thereto. After heating and stirring at 80°C for 1 hour, the solvent was distilled off under reduced pressure. 30 ml of ethyl acetate was added to the residue, and heating and stirring were carried out at 60°C for 15 minutes, followed by air cooling to 0°C. The solid was filtered and dried under reduced pressure to obtain a white solid. The obtained solid was dissolved in 5 ml of distilled water, and 400 mg of sodium hydroxide (NaOH) was added thereto. After heating and stirring at 80°C for 1 hour, cooling was carried out to room temperature, and 1.2 ml of a 30% aqueous hydrogen chloride solution was added thereto. The reaction solution was purified by reverse phase column chromatography (eluent: acetonitrile/water = from 0/100 to 10/90), and freeze drying was followed to obtain 333 mg of a compound (7-D). The results of the MS measurement of the compound (7-D) were as follows.
MS (ESI m/z):$(M - H^+)^-$ = 475, 477

A compound (7-A) was synthesized as follows.

A colorless solid compound (7-A) (118 mg, yield: 30%) was obtained in the same manner, except that in the synthesis method for the compound (5-C), the compound (7-D) was used instead of the compound (5-F).
$[M + H^+]^+$: 443

<Synthesis of compound (8)>

A compound (8) was synthesized in the same manner as the compound (5), based on the following scheme. The results of the MS measurement of the compound (8-B) were as follows.
MS (ESI m/z):$(M + H^+)^+$ = 1,231

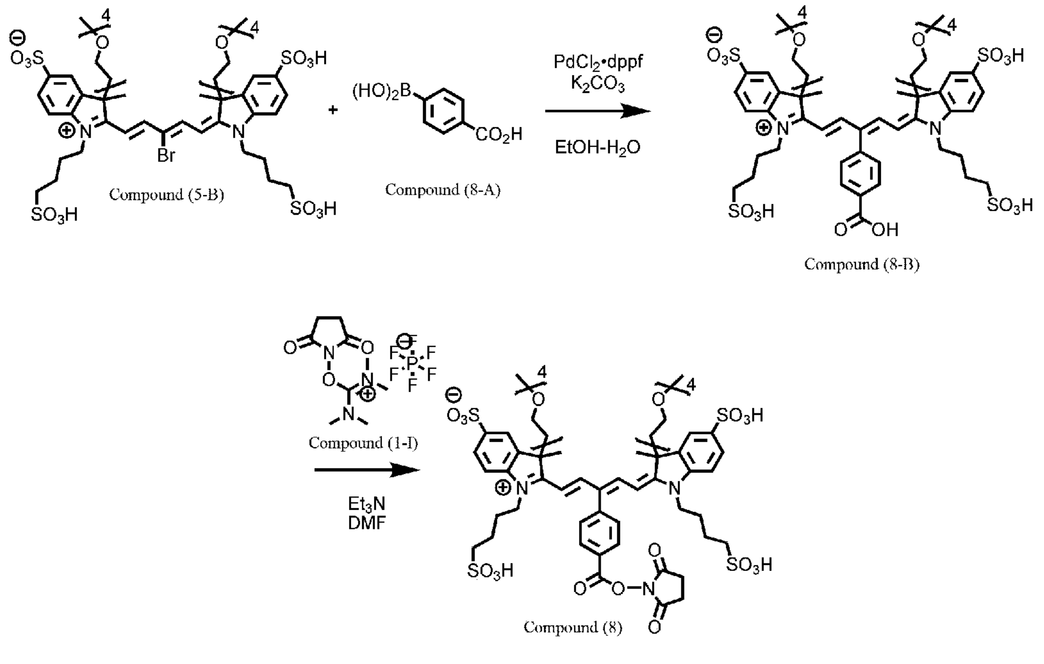

Compound (5-B) Compound (8-A) Compound (8-B) Compound (1-I) Compound (8)

<Synthesis of compound (9)>

The compound (9) was synthesized based on the following scheme.

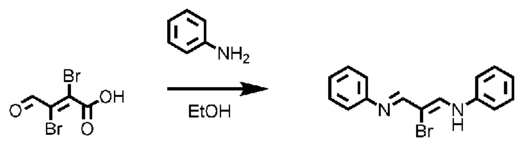

Compound (9-A) Compound (9-B)

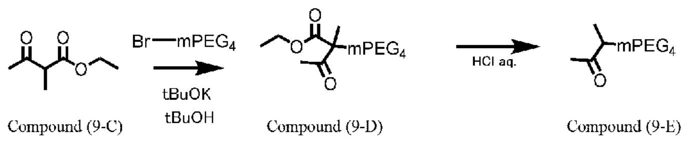


Compound (9-C) Compound (9-D) Compound (9-E)

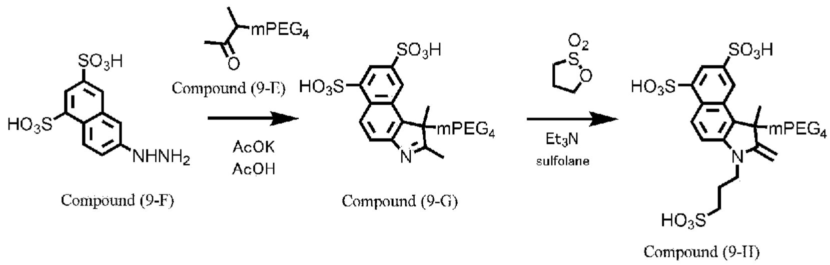


Compound (9-F) Compound (9-E) Compound (9-G) Compound (9-H)

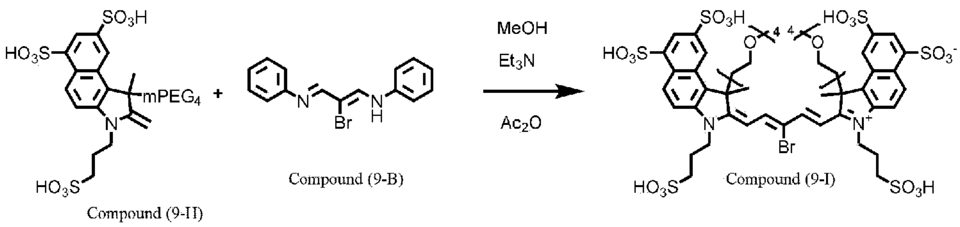


Compound (9-H) Compound (9-B) Compound (9-I)

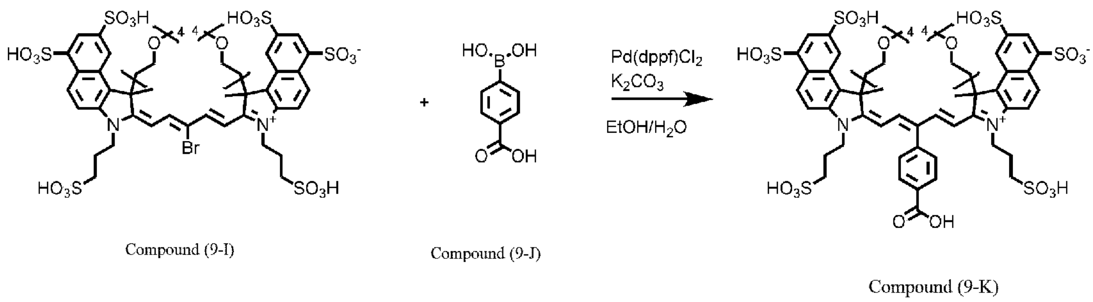


Compound (9-I) Compound (9-J) Compound (9-K)

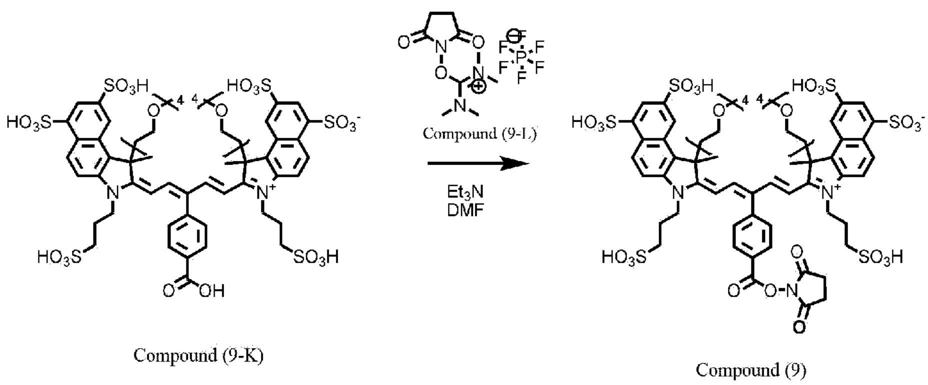


Compound (9-L) Compound (9-K) Compound (9)

Synthesis of compound (9-B)

2.9 g of the compound (9-A) and 15 ml of ethanol were placed in an eggplant flask having a capacity of 100 ml, and 15 mL of ethanol in which 2.1 mL of aniline had been dissolved was added dropwise thereto at 0°C, followed by stirring at 60°C for 4 hours in a nitrogen atmosphere. The solvent was distilled off under reduced pressure to adjust the solvent amount to 15 mL, and air cooling was carried out to 0°C. The precipitated yellow crystals were filtered to obtain 2.6 g of a compound (9-B).

Synthesis of compound (9-E)

200 ml of tert-butanol (tBuOH) and 12 g of potassium tert-butoxide (tBuOK) were placed in a nitrogen-substituted three-necked flask having a capacity of 500 ml, and while stirring the resultant mixture, 14.4 g of the compound (9-C) was added dropwise thereto, and stirring was carried out for a while. Next, 32.5 g of triethylene glycol 2-bromoethyl methyl ether (Br-mPEG4) was added dropwise thereto, and heating and stirring were carried out. After stirring at 80°C for 1 hour, the solvent was distilled off under reduced pressure, a liquid separation operation was carried out with ethyl acetate and distilled water, and a crude product was extracted with distilled water. 30 ml of a 30% hydrochloric acid aqueous solution was added to the obtained crude product, and stirring was carried out at 100°C for 3 hours. Then, the solvent was distilled off under reduced pressure, and purification was carried out by normal phase column chromatography (eluent: hexane/ethyl acetate = from 50/50 to 30/70) to obtain 11.0 g of a compound (9-E).

Synthesis of compound (9-G)

100 mg of the compound (9-F), 0.3 mL of acetic acid (AcOH), 124 mg of the compound (9-E), and 31 mg of potassium acetate (AcOK) were placed in an eggplant flask having a capacity of 100 ml, and stirring was carried out at 130°C for 1 hour in a nitrogen atmosphere. The solvent was distilled off under reduced pressure, the purification was carried out by reverse phase column chromatography (eluent: acetonitrile/water = from 0/100 to 20/80) to obtain 58 mg of a compound (9-G).

Synthesis of compound (9-H)

470 mg of the compound (9-G), 0.15 mL of 1,3-propane sultone, 2 mL of sulfolane, and 254 mg of potassium acetate (AcOK) were placed in an eggplant flask having a capacity of 50 ml, and stirring was at 110°C for 1 hour. 20 mL of ethyl acetate was added to the reaction solution to cause precipitation. The precipitate was purified by reverse phase column chromatography (eluent: acetonitrile/water = from 0/100 to 30/70) to obtain 475 mg of a compound (9-H).

Synthesis of compound (9-I)

171 mg of the compound (9-H), 48 mg of the compound (9-B), 71 μL of triethylamine ($Et_3N$), 5 μL of methanol (MeOH), and 0.48 mL of acetic anhydride (AczO) were added in a test tube, and stirring was carried out at 50°C for 2 hours in a nitrogen atmosphere. After the reaction was settled, distilled water was added, and purification was carried out by reverse phase column chromatography (eluent: acetonitrile/water = from 0/100 to 25/75) to obtain 150 mg of a compound (9-I).

Synthesis of compound (9-K)

10 mg of the compound (9-I), 10 mg of potassium carbonate ($K_2CO_3$), 300 μL of ethanol (EtOH), and 100 μL of distilled water were added in a test tube, and stirring was carried out at 50°C. To this solution, a solution obtained by mixing 6 mg of the compound (9-J) and [1,1'-bis(diphenylphosphino)ferrocene]palladium (II) dichloride ($Pd(dppf)Cl_2$) in 200 μL of distilled water was added dropwise, and then stirring was carried out at 50°C for 30 minutes. The reaction solution was cooled to room temperature and purified by preparative HPLC, and freeze drying was followed to obtain 3.6 mg of a compound (9-K). The results of the MS measurement of the compound (9-K) were as follows.
MS (ESI m/z):$(M + H^+)^+$ = 1,491, $(M - H^+)^-$ = 1,489

Synthesis of compound (9)

0.30 ml of N,N-dimethylformamide (DMF) and 1 mg of the compound (9-L) were dissolved in 3.6 mg of the compound (9-K), and then stirring was carried out for 1 hour. Then, the solvent was distilled off under reduced pressure, 1.5 mL of ethyl acetate was added, the supernatant was removed, and vacuum drying was carried out to obtain a compound (9).

<Synthesis of compound (10)>

A compound (10) was synthesized in the same manner as the compound (9), based on the following scheme. The results of the MS measurement of the compound (10-C) were as follows.
MS (ESI m/z):$(M + H^+)^+$ = 1,505, $(M - H^+)^-$ = 1,503

A compound (10-B) was synthesized as follows. 500 mg of the compound (10-A), 309 mg of tetrahydroxydiboron,

3 mg of bis(di-tert-butyl(4-dimethylaminophenyl)phosphine)dichloropalladium (II), 760 μL of N,N-diisopropylethylamine (DIPEA), 5 mL of tetrahydrofuran (THF), and 2.5 mL of methanol (MeOH) were placed in a three-necked eggplant flask having a capacity of 100 ml, and stirring was carried out at 55°C for 3 hours in a nitrogen atmosphere. The solvent was distilled off under reduced pressure, the purification was carried out by reverse phase column chromatography (eluent: acetonitrile/water = from 0/100 to 30/70) to obtain 220 mg of a compound (10-B).

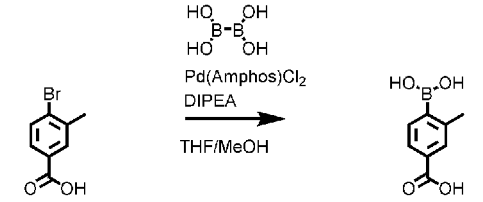


Compound (10-A) Compound (10-B)

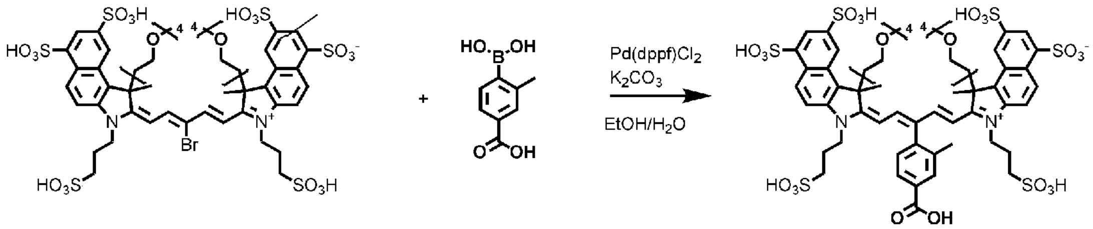


Compound (9-I) Compound (10-B)

Compound (10-C)

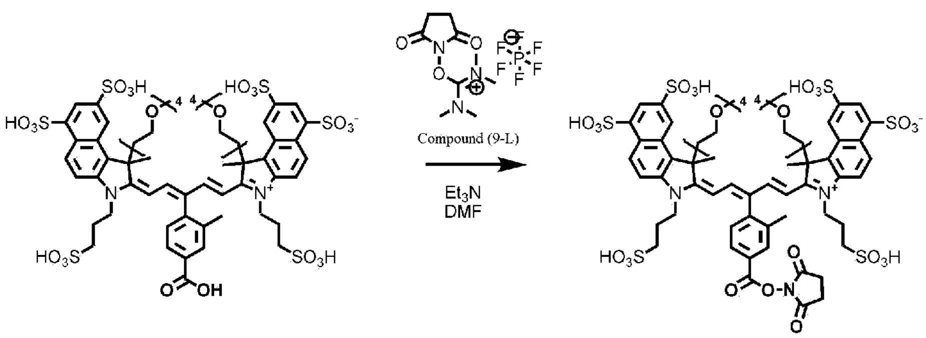


Compound (10-C) Compound (10)

<Synthesis of compound (11)>

A compound (11) was synthesized in the same manner as the compound (10), based on the following scheme. The results of the MS measurement of the compound (11-C) were as follows.

MS (ESI m/z):$(M + H^+)^+ = 1{,}551$, $(M - H^+)^- = 1{,}549$

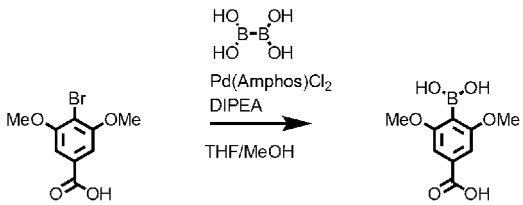


Compound (11-A) Compound (11-B)

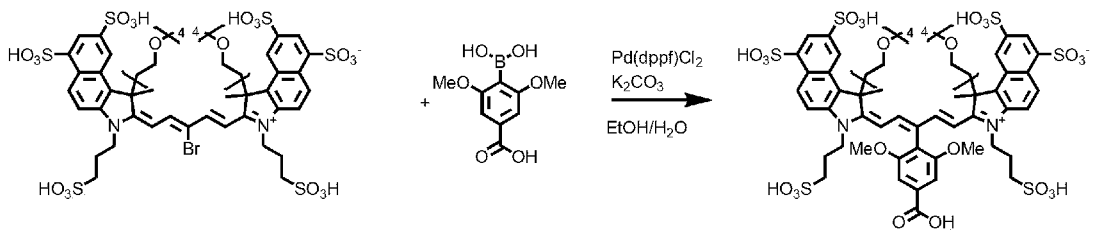


Compound (9-I) Compound (11-B) Compound (11-C)

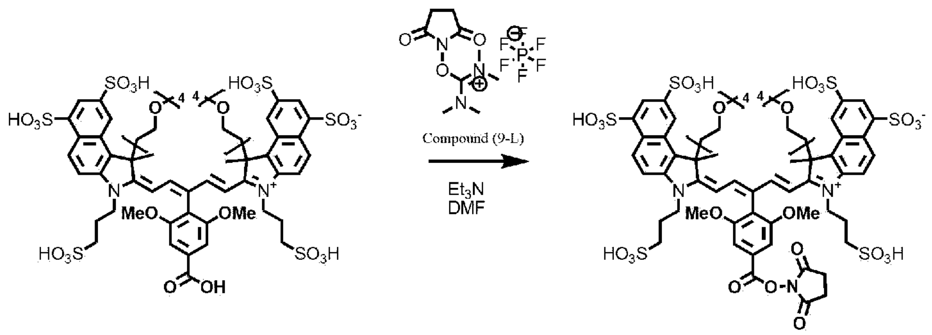


Compound (11-C) Compound (11)

<Synthesis of compound (12)>

A compound (12) was synthesized in the same manner as the compound (10), based on the following scheme. The results of the MS measurement of the compound (12-C) were as follows.
MS (ESI m/z):$(M + H^+)^+$ = 1,519, $(M - H^+)^-$ = 1,517

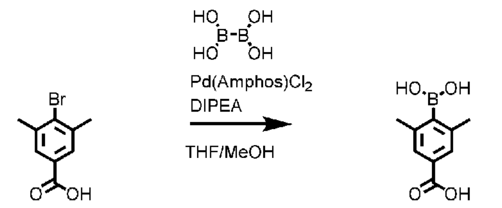


Compound (12-A) Compound (12-B)

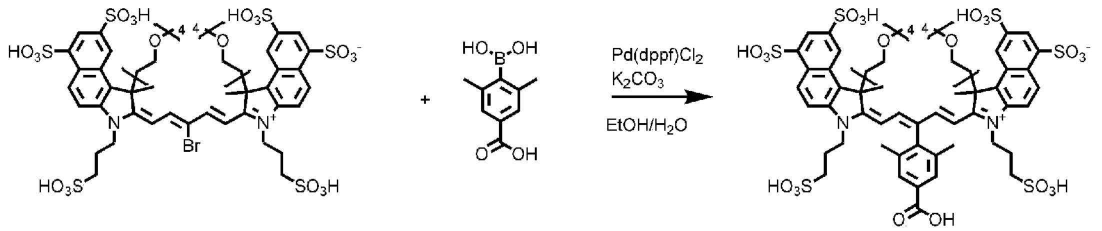


Compound (9-I) Compound (12-B) Compound (12-C)

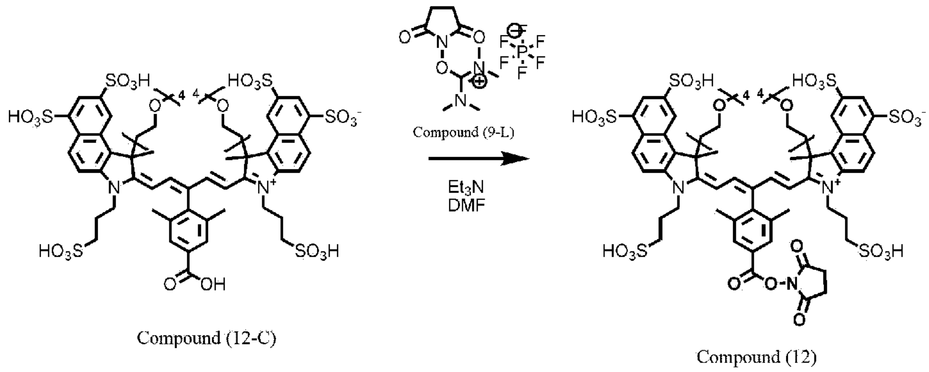


Compound (12-C)

Compound (12)

<Synthesis of compound (13)>

A compound (13) was synthesized in the same manner as the compound (10), based on the following scheme. The results of the MS measurement of the compound (13-C) were as follows.
MS (ESI m/z):$(M + H^+)^+$ = 1,767, $(M - H^+)^-$ = 1,765

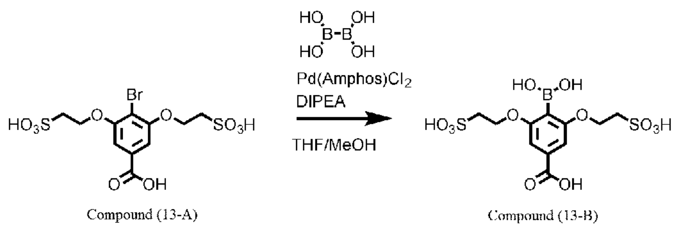


Compound (13-A)

Compound (13-B)

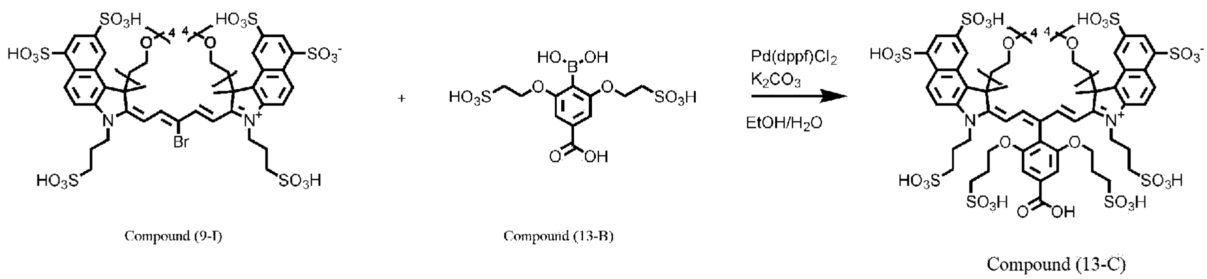


Compound (9-I)

Compound (13-B)

Compound (13-C)

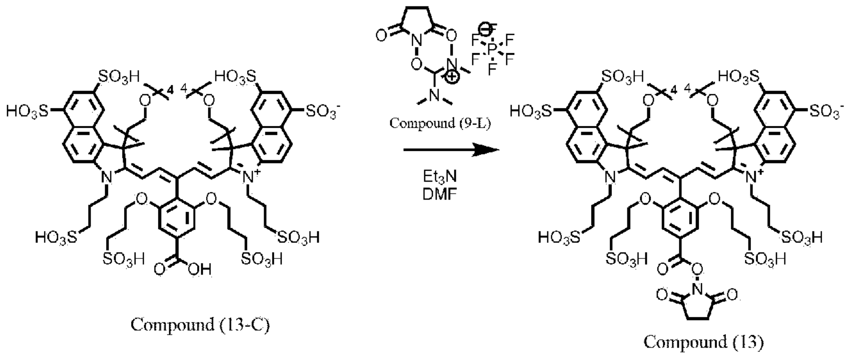


Compound (13-C)

Compound (13)

<Synthesis of compound (14)>

A compound (14) was synthesized in the same manner as the compound (10), based on the following scheme. The results of the MS measurement of the compound (14-C) were as follows.
MS (ESI m/z):$(M + H^+)^+$ = 1,521, $(M - H^+)^-$ = 1,519

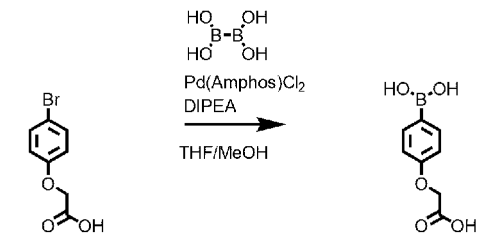

Compound (14-A) Compound (14-B)

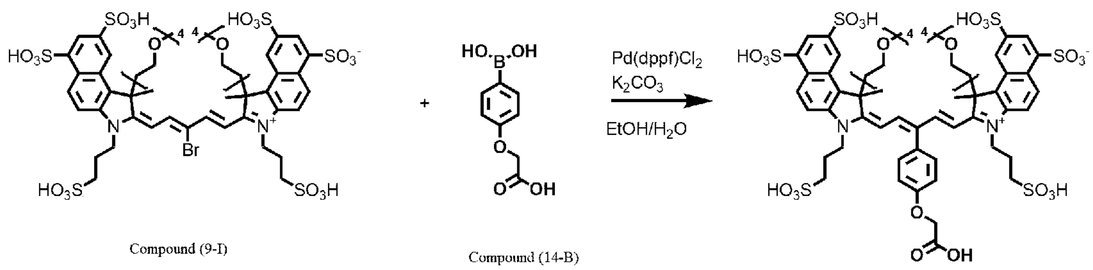

Compound (9-I) Compound (14-B) Compound (14-C)

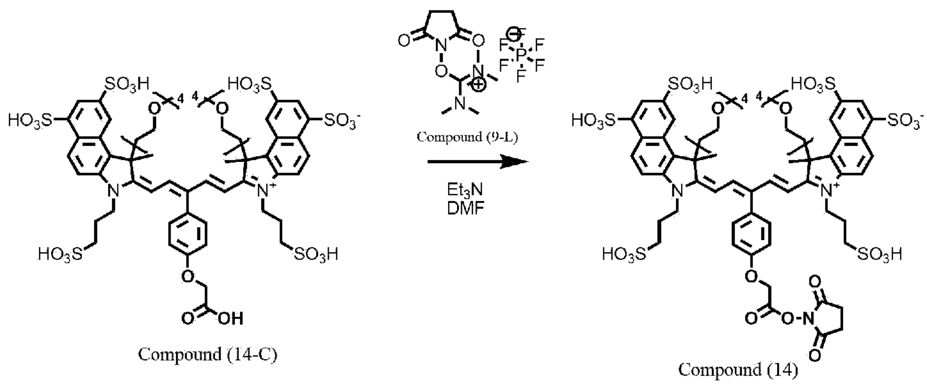

Compound (14-C) Compound (14)

<Synthesis of compound (15)>

A compound (15) was synthesized in the same manner as the compound (9), based on the following scheme. The results of the MS measurement of the compound (15-K) were as follows.
MS (ESI m/z):$(M + H^+)^+$ = 1,331, $(M - H^+)^-$ = 1,329

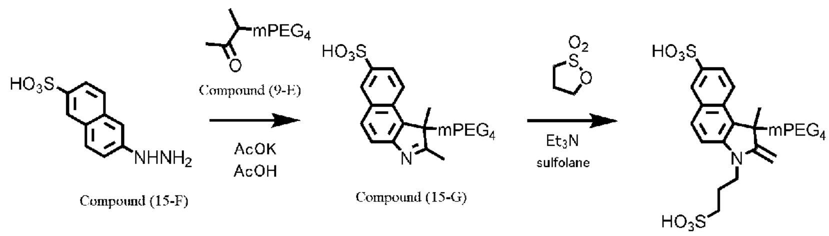

Compound (15-F) Compound (15-G) Compound (15-H)

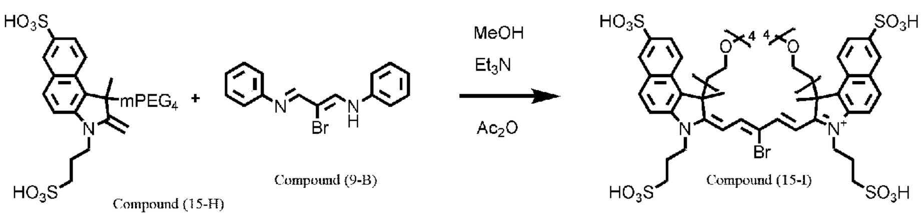

Compound (15-H) Compound (9-B) Compound (15-I)

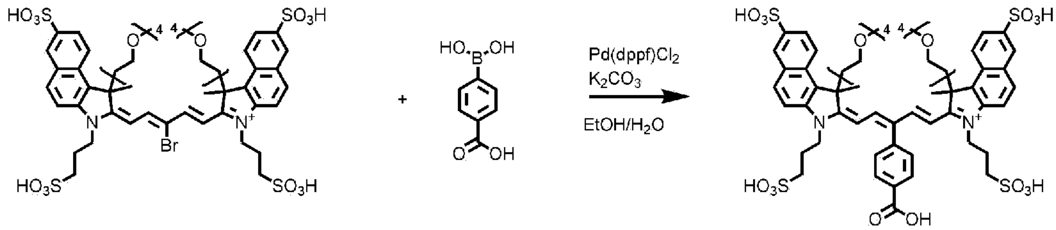


Compound (15-I) Compound (15-J) Compound (15-K)

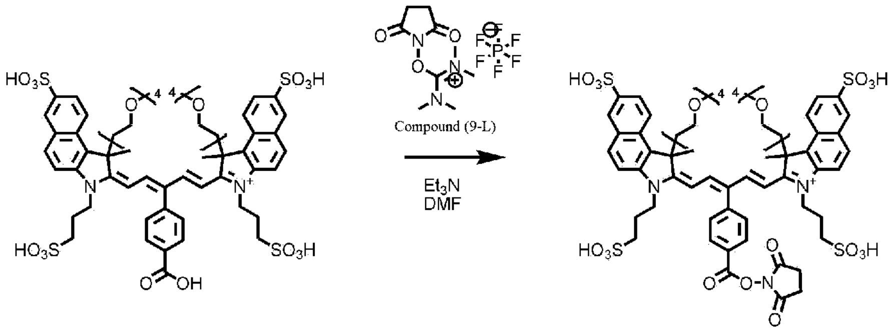


Compound (15-K) Compound (15)

<Synthesis of compound (16)>

A compound (16) was synthesized in the same manner as the compound (13), based on the following scheme. The results of the MS measurement of the compound (16-A) were as follows.
MS (ESI m/z):$(M + H^+)^+$ = 1,608, $(M - H^+)^-$ = 1,606

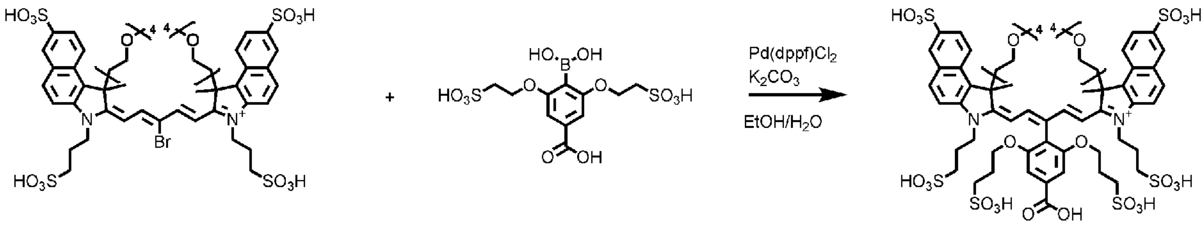


Compound (15-I) Compound (13-B) Compound (16-A)

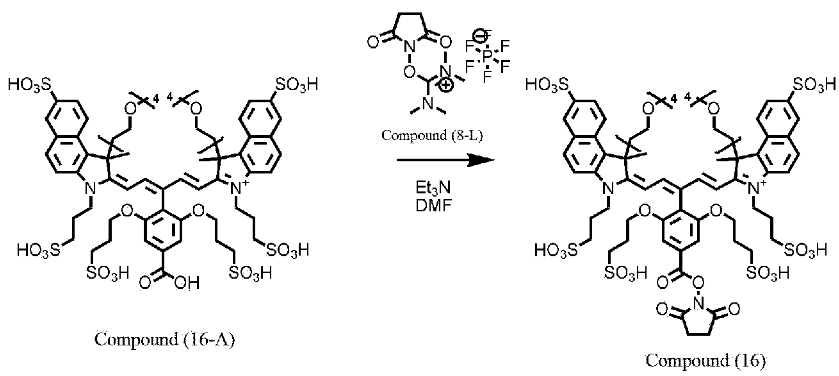


Compound (16-A) Compound (16)

<Synthesis of compound (17)>

Synthesis of compound (17)

5 mg of the compound (2-F), 8 $\mu$L of triethylamine (TEA), and 100 $\mu$L of distilled water were added in a test tube, and stirring was carried out at 80°C. To this solution, a solution obtained by mixing 2 mg of the compound (9-J) and tetrakis(triphenylphosphine)palladium (0) ($Pd(PPh_3)_4$) in 100 $\mu$L of distilled water was added dropwise, and stirring was carried out at 80°C for 1 hour. The reaction solution was cooled to room temperature and purified by preparative HPLC, and freeze drying was followed to obtain 2.8 mg of a compound (17). The results of the MS measurement and the absorption fluorescence measurement of the compound (17) were as follows. It is noted that $\lambda_{ex}$ means an excitation wavelength, and $\lambda_{em}$ means a fluorescence wavelength, each of which has the same meaning hereinafter.

MS (ESI m/z):$(M + H^+)^+$ = 1,297, $(M - H^+)^-$ = 1,295
$\lambda_{ex}$ = 768 nm, $\lambda_{em}$ = 787 nm

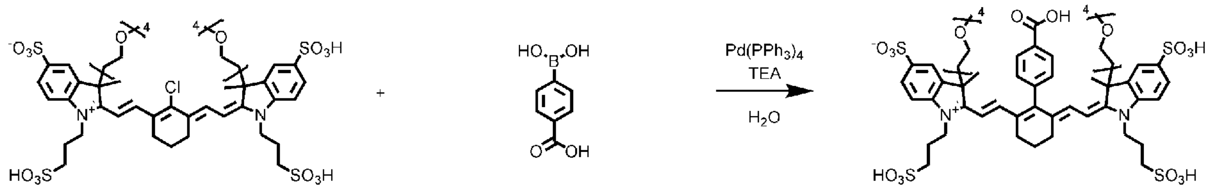


Compound (2-F) Compound (9-J) Compound (17)

<Synthesis of compound (18)>

A compound (18) was synthesized in the same manner as the compound (17), based on the following scheme. The results of the MS measurement and the absorption fluorescence measurement of the compound (18) were as follows.

MS (ESI m/z):$(M + H^+)^+$ = 1,327, $(M - H^+)^-$ = 1,325
$\lambda_{ex}$ = 765 nm, $\lambda_{em}$ = 784 nm

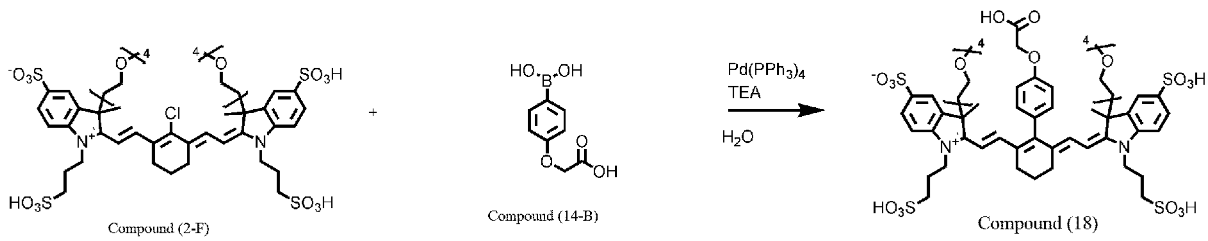


Compound (2-F) Compound (14-B) Compound (18)

<Synthesis of compound (19)>

A compound (19) was synthesized in the same manner as the compound (17), based on the following scheme. The results of the MS measurement and the absorption fluorescence measurement of the compound (19) were as follows.

MS (ESI m/z):$(M + H^+)^+$ = 1,325, $(M - H^+)^-$ = 1,323
$\lambda_{ex}$ = 770 nm, $\lambda_{em}$ = 789 nm

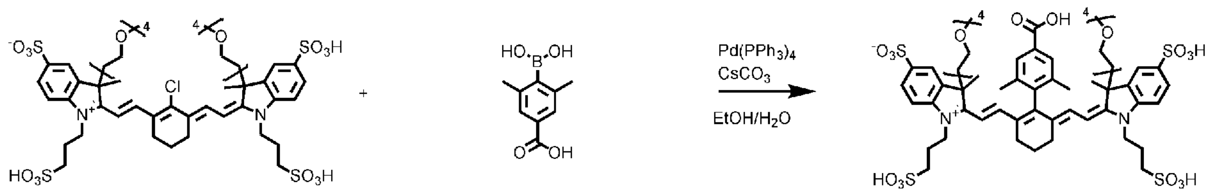


Compound (2-F) Compound (12-B) Compound (19)

<Synthesis of compound (20)>

A compound (20) was synthesized in the same manner as the compound (17), based on the following scheme. The results of the MS measurement and the absorption fluorescence measurement of the compound (20) were as follows.

MS (ESI m/z):$(M + H^+)^+$ = 1,573, $(M - H^+)^-$ = 1,571
$\lambda_{ex}$ = 777 nm, $\lambda_{em}$ = 794 nm

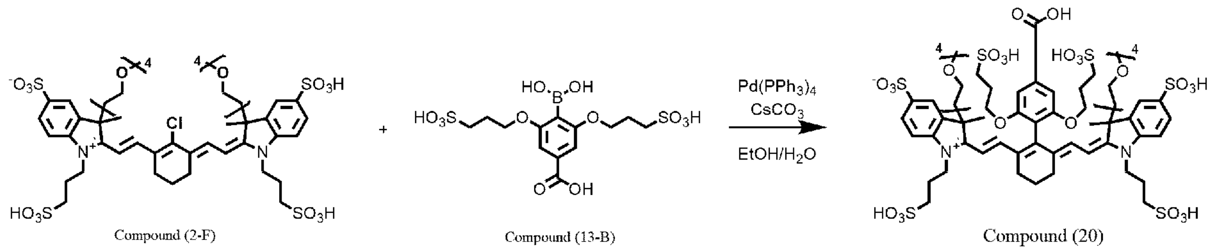


Compound (2-F) Compound (13-B) Compound (20)

<Synthesis of comparative compound (1)>

A comparative compound (1) was synthesized in the same manner as the compound (1), based on the following scheme. The results of the MS measurement of the compound (c1-C) were as follows.
MS (ESI m/z):$(M + H^+)^+$ = 989, $(M - H^+)^-$ = 987

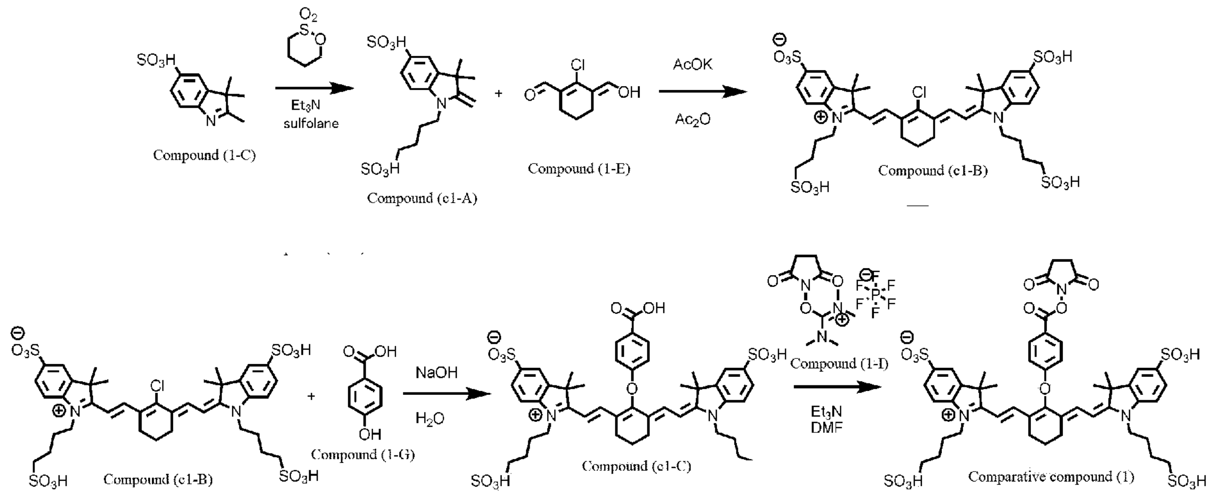


Compound (1-C) Compound (c1-A) Compound (1-E) Compound (c1-B)

Compound (c1-B) Compound (1-G) Compound (c1-C) Compound (1-I) Comparative compound (1)

<Synthesis of comparative compound (2)>

A comparative compound (2) was synthesized in the same manner as the compound (1), based on the following scheme. The results of the MS measurement of the compound (c2-C) were as follows.
MS (ESI m/z):$(M + H^+)^+$ = 2,858, $(M - H^+)^-$ = 2,856

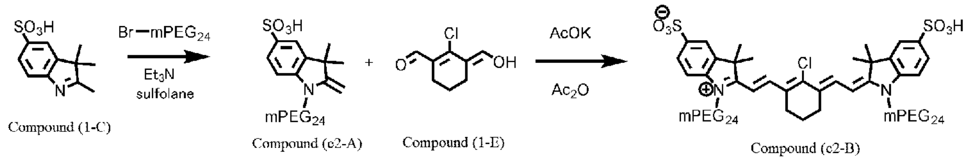


Compound (1-C) Compound (c2-A) Compound (1-E) Compound (c2-B)

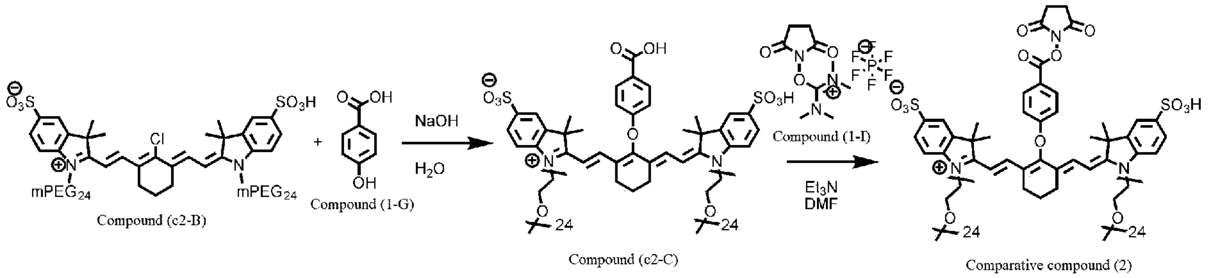


<Synthesis of comparative compound (3)>

A comparative compound (3) was synthesized in the same manner as the compound (1), based on the following scheme. The results of the MS measurement of the compound (c3-D) were as follows.
MS (ESI m/z): $(M + H^+)^+$= 1,111, $(M - H^+)^-$ = 1,109

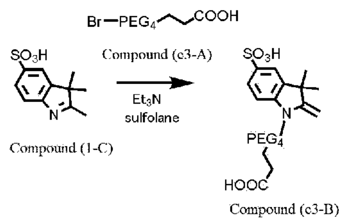


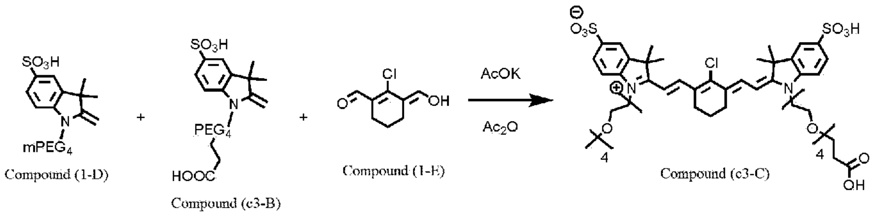


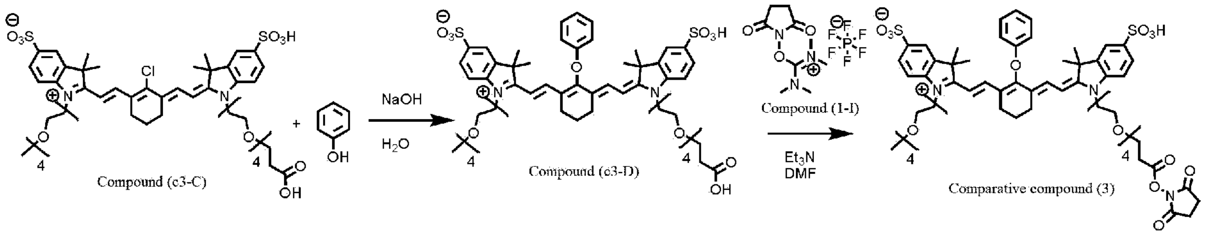


<Synthesis of comparative compound (4)>

A comparative compound (4) was synthesized in the same manner as the compound (8), based on the following scheme. The results of the MS measurement of the comparative compound (4-G) were as follows.
MS (ESI m/z):$(M + H^+)^+$ = 1,565, $(M - H^+)^-$ = 1,563

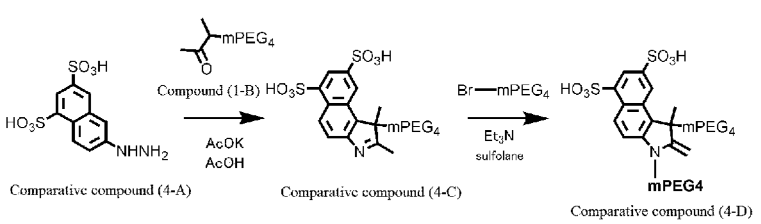

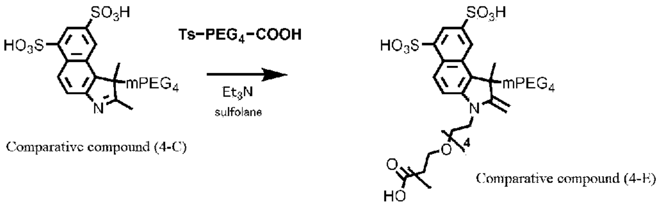


Comparative compound (4-C)

Comparative compound (4-E)

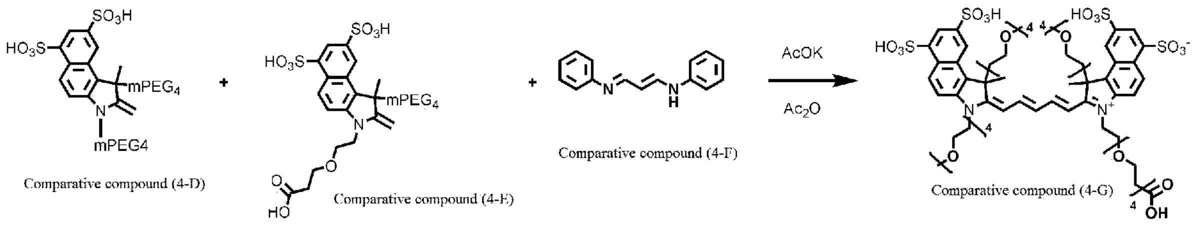


Comparative compound (4-D)

Comparative compound (4-E)

Comparative compound (4-F)

Comparative compound (4-G)

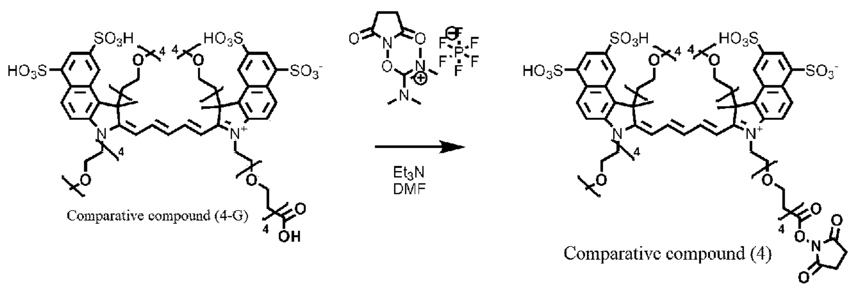


Comparative compound (4-G)

Comparative compound (4)

<Example 1: Evaluation at excitation light wavelength of 785 nm>

Regarding each of the above-described compounds (1) to (4) and the comparative compounds (1) to (3), the fluorescence labeling rate, the solution fluorescence intensity, and the fluorescence intensity on the membrane were evaluated.

[1] Evaluation of fluorescence labeling rate

217 μL of an anti-rabbit IgG antibody (2.3 mg/ml) and 21.7 μL of a carbonate buffer were added to a microtube, the resultant mixture was shaken and stirred. Then, a dimethyl sulfoxide solution of the compound (1) was added thereto so that the molar equivalent ratio of the compound (1) to the antibody was as shown in Table 1, and the resultant mixture further was shaken and stirred. After being allowed to stand at room temperature for 1 hour, the reaction solution was purified using a gel filtration chromatography column PD10 (manufactured by GE Healthcare Life Sciences) and a PBS solution (a phosphate-buffered saline solution) to obtain a labeled anti-rabbit IgG antibody labeled with the compound (1). In the same manner, a labeled anti-rabbit IgG antibody labeled with each compound or a comparative compound was obtained. The fluorescence labeling rate (DOL) of the obtained labeled antibody was calculated according to the method described below. The results are summarized in Table 1.

As the method for calculating the fluorescence labeling rate, a general method as described below was used. The description in [ ] indicates a unit, and [-] means that there is no unit. In this study, protein means an anti-rabbit IgG antibody.

$$\text{fluorescence labeling rate} = \text{fluorescent dye concentration}/\text{protein concentration}$$

The fluorescent dye concentration means the total molar concentration [M] of the labeled fluorescent dye, and the protein concentration means the molar concentration [M] of the fluorescently labeled protein. They are respectively calculated according to the following expressions.

$$\text{Fluorescent dye concentration} = \text{Dye}_{\text{max}}/\varepsilon_{\text{dye}}$$

$$\text{Protein concentration} = (IgG_{280} - (Dye_{max} \times CF))/\varepsilon_{protein}$$

Each symbol in the above expressions is as follows.

$Dye_{max}$: Absorption [-] of fluorescent dye at maximum absorption wavelength
$\varepsilon_{dye}$: Molar absorption coefficient [$M^{-1}cm^{-1}$] of fluorescent dye
$IgG_{280}$: Absorption [-] of fluorescently labeled protein at 280 nm
$Dye_{280}$: Absorption [-] of fluorescent dye at 280 nm
$\varepsilon_{protein}$: Molar absorption coefficient [$M^{-1}cm^{-1}$] of protein
Correction Factor (CF): $Dye_{280}/Dye_{max}$ [-]

[Table 1]

| No. | Labeled antibody | 3 equivalents | 5 equivalents | 7 equivalents | 10 equivalents |
|---|---|---|---|---|---|
| 101 | Compound (1) - IgG | 2.3 | 3.6 | 4.5 | 6.2 |
| 102 | Compound (2) - IgG | 2.2 | 3.5 | 4.5 | 6.1 |
| 103 | Compound (3) - IgG | 2.3 | 3.7 | 4.4 | 6.5 |
| 104 | Compound (4) - IgG | 2.2 | 3.4 | 4.3 | 6.0 |
| c11 | Comparative compound (1) - IgG | 2.2 | 3.2 | 4.2 | 5.9 |
| c12 | Comparative compound (2) - IgG | 1.2 | 1.8 | 2.4 | 3.3 |
| cl3 | Comparative compound (3) - IgG | 2.3 | 3.3 | 4.2 | 6.1 |
| (Note in table) | | | | | |

In the column of the labeled antibody, the notation of the compound (Z) - IgG or the comparative compound (Z) - IgG means a labeled anti-rabbit IgG antibody labeled with the compound (Z) or a labeled anti-rabbit IgG antibody labeled with the comparative compound (Z). Here, Z means the number of each compound. The same applies to the following tables.

From the results in Table 1 above, the following points can be seen.

Even in a case of being added at any molar equivalent of 3 equivalents, 5 equivalents, 7 equivalents, or 10 equivalents, with respect to 1 equivalent of the antibody, the compounds (1) to (4), which are the compounds according to the embodiment of the present invention, exhibit a fluorescence labeling rate comparable to that obtained in a case where the comparative compound (1) which is a labeling compound described in US2021/0093737A is used, where the fluorescence labeling rate exceeds that obtained in a case where the comparative compound (2) which is the labeling compound described in WO2009/078970A is used, and the binding property to the antibody was at a sufficient level without any problem in practical use. This fact can be read from the comparison between No. c11 or c12 and Nos. 101 to 104.

On the other hand, the comparative compound (3) is not the compound defined in the present invention in that $L^1$ in General Formula (1) according to the embodiment of the present invention has a substituent capable of being bonded to a biological substance. It can be seen that the compounds (1) to (4), which are the compounds according to the embodiment of the present invention, and the comparative compound (3) exhibit a similar level of the fluorescence labeling rate, and there is no difference in the fluorescence labeling rate.

[2] Evaluation of solution fluorescence intensity

A solution of each labeled antibody, which had been prepared in "[1] Evaluation of fluorescence labeling rate" described above, was adjusted to a protein concentration of 0.005 mg/mL, and the integrated value of the fluorescence intensity in the fluorescence wavelength range of 810 to 840 nm was calculated by using a spectroscopic fluorescence intensity meter (product name: RF-5300 or RF-6000, manufactured by Shimadzu Corporation) with excitation light of 785 nm and unified the exposure conditions. Using the integrated value of the fluorescence intensity of the comparative compound (1) - IgG having DOL of 2.2 (3 equivalents of dye had been added) in the fluorescence wavelength range of 810 nm to 840 nm as the reference value, the ratio to this reference value (the integrated value of the fluorescence intensity of the labeled antibody in the fluorescence wavelength range of 810 nm to 840 nm/the reference value) was

calculated, and then, the evaluation was made based on the following evaluation standards. The results are summarized in Table 2.

- Evaluation standards for fluorescence intensity (integrated value) -

A: The ratio of fluorescence intensity to the reference value is 2.0 times or more.

B: The ratio of fluorescence intensity to the reference value is 1.7 times or more and less than 2.0 times.

C: The ratio of fluorescence intensity to the reference value is 1.4 times or more and less than 1.7 times.

D: The ratio of fluorescence intensity to the reference value is 1.2 times or more and less than 1.4 times.

E: The ratio of fluorescence intensity to the reference value is 1.1 times or more and less than 1.2 times.

F: The ratio of fluorescence intensity to the reference value is 0.9 times or more and less than 1.1 times.

G: The ratio of fluorescence intensity to the reference value is less than 0.9 times.

[Table 2]

| No. | Labeled antibody | Molar equivalent ratio of compound to antibody | DOL | Fluorescence intensity (solution) |
|---|---|---|---|---|
| 101 | Compound (1) - IgG | 3 equivalents | 2.3 | D |
| | | 5 equivalents | 3.6 | D |
| | | 7 equivalents | 4.5 | D |
| | | 10 equivalents | 6.2 | E |
| 102 | Compound (2) - IgG | 3 equivalents | 2.2 | C |
| | | 5 equivalents | 3.5 | B |
| | | 7 equivalents | 4.5 | B |
| | | 10 equivalents | 6.1 | B |
| 103 | Compound (3) - IgG | 3 equivalents | 2.3 | B |
| | | 5 equivalents | 3.7 | A |
| | | 7 equivalents | 4.4 | A |
| | | 10 equivalents | 6.5 | A |
| 104 | Compound (4) - IgG | 3 equivalents | 2.2 | B |
| | | 5 equivalents | 3.4 | A |
| | | 7 equivalents | 4.3 | A |
| | | 10 equivalents | 6.0 | A |
| c11 | Comparative compound (1) - IgG | 3 equivalents | 2.2 | 1.0 (reference value) |
| | | 5 equivalents | 3.2 | F |
| | | 7 equivalents | 4.2 | G |
| | | 10 equivalents | 5.9 | G |

(continued)

| No. | Labeled antibody | Molar equivalent ratio of compound to antibody | DOL | Fluorescence intensity (solution) |
|---|---|---|---|---|
| c12 | Comparative compound (2) - IgG | 3 equivalents | 1.2 | G |
| | | 5 equivalents | 1.8 | G |
| | | 7 equivalents | 2.4 | F |
| | | 10 equivalents | 3.3 | E |
| c13 | Comparative compound (3) - IgG | 3 equivalents | 2.3 | F |
| | | 5 equivalents | 3.3 | F |
| | | 7 equivalents | 4.2 | G |
| | | 10 equivalents | 6.1 | G |

From the results in Table 2 above, the following points can be seen.

The comparative compound (1) is not a compound having the structure defined in the present invention in that the comparative compound (1) does not have a structure including a PEG group. In the solution, the fluorescence intensity of the labeled antibody labeled with this comparative compound (1) is low at any of the DOL values (No. c11).

The comparative compound (2) has a repetition number of PEG which is larger than the repetition number defined in the present invention, in that the repetition number (m defined in the present invention) of PEG is 24. In the solution, the fluorescence intensity of the labeled antibody labeled with this comparative compound (2) is low as compared with the labeled antibody labeled with each of the compounds (1) to (4), which had the same DOL (No. c12).

The comparative compound (3) is not a compound having the structure defined in the present invention in that $L^1$, instead of $R^{11}$ to $R^{13}$ in General Formula (1), has a carboxy group or a substituent capable of being bonded to a biological substance. In the solution, the fluorescence intensity of the labeled antibody labeled with this comparative compound (3) is low at any of the DOL values (No. c13).

On the other hand, all of the labeled antibodies labeled with the compounds (1) to (4), which are the compounds according to the embodiment of the present invention, exhibit obviously high fluorescence intensity as compared with the fluorescence intensity of the labeled antibody labeled with the comparative compound (1) (Nos. 101 to 104 with respect to No. c11).

In addition, it can be seen that among the labeled antibodies labeled with the compounds (1) to (4), which are the compounds according to the embodiment of the present invention, the labeled antibody labeled with each of the compounds (2) to (4) according to the embodiment of the present invention, in which the position having a structure including a PEG group is set to at least one of $R^1$ or $R^2$ and at least one of $R^3$ or $R^4$, exhibits a more excellent fluorescence intensity, and the labeled antibody labeled with each of the compounds (3) and (4) according to the embodiment of the present invention, which further has a structure including two PEG groups in the compound, exhibit a more excellent fluorescence intensity.

In addition, From the comparison between No. 101 in which the compound (1) having the repetition number of PEG of 4, shown in Table 1 and Table 2, is used, and No. c12 in which the comparative compound (2) having a structure differing only in that the repetition number of PEG is 24 is used, it can be seen that it is possible to sufficiently label an antibody with a smaller using amount of the fluorescent compound by setting the repetition number of PEG within the range defined in the present invention (Table 1). In addition, from the comparison between the solutions of the labeled antibodies showing a similar level of DOL, it can be also seen that it is possible to realize a more excellent fluorescence intensity in a case of setting the repetition number of PEG within the range defined in the present invention (Table 2).

[3] Fluorescence intensity on membrane

The anti-rabbit IgG solution was adjusted to a protein concentration of 5.0 ng/mL, and 2 μL thereof was carefully spotted on a nitrocellulose membrane. The membrane was dried and then blocked with a Fish Gelatin blocking buffer solution in a Tris Buffered Saline with Tween 20 (TBS-T). The membrane was incubated at room temperature for 1 hour with stirring. The blocking solution was removed, and the PBS solution of the labeled antibody (the solution of the labeled antibody prepared in the section of [1] described above, concentration before dilution: 1 mg·mL) was diluted 20,000 times with a TBS buffer solution. The membrane was immersed in the diluted solution and incubated for 1 hour with stirring. The membrane was washed 3 times with a TBS-T buffer solution for 10 minutes and finally washed with a TBS buffer solution for 10 minutes. The obtained membrane was dried on a hot plate at 40°C for 1 hour and imaged using

an Amersham Typhoon scanner (manufactured by GEHC), and with excitation light of 785 nm under the uniform exposure conditions, the fluorescence intensity of a fraction of 6.48 $mm^2$ in a fluorescence wavelength range of 810 to 840 nm was measured and used as a signal fluorescence intensity. Using the integrated value of the signal fluorescence intensity of the comparative compound (1) - IgG having DOL of 2.2 in the fluorescence wavelength range of 810 nm to 840 nm as the reference value, the ratio to this reference value (the integrated value of the signal fluorescence intensity of the labeled antibody in the fluorescence wavelength range of 810 nm to 840 nm/the reference value) was calculated, and then, the evaluation was made based on the following evaluation standards. The results are summarized in Table 3.

- Evaluation standards for fluorescence intensity (integrated value) -

A: The ratio of signal fluorescence intensity to the reference value is 2.0 times or more.

B: The ratio of signal fluorescence intensity to the reference value is 1.7 times or more and less than 2.0 times.

C: The ratio of signal fluorescence intensity to the reference value is 1.4 times or more and less than 1.7 times.

D: The ratio of signal fluorescence intensity to the reference value is 1.2 times or more and less than 1.4 times.

E: The ratio of signal fluorescence intensity to the reference value is 1.1 times or more and less than 1.2 times.

F: The ratio of signal fluorescence intensity to the reference value is 0.9 times or more and less than 1.1 times.

G: The ratio of signal fluorescence intensity to the reference value is less than 0.9 times.

[Table 3]

| No. | Labeled antibody | Molar equivalent ratio of compound to antibody | DOL | Signal fluorescence intensity (on membrane) |
|---|---|---|---|---|
| 201 | Compound (1) - IgG | 3 equivalents | 2.3 | D |
| | | 5 equivalents | 3.6 | D |
| | | 7 equivalents | 4.5 | D |
| | | 10 equivalents | 6.2 | E |
| 202 | Compound (2) - IgG | 3 equivalents | 2.2 | B |
| | | 5 equivalents | 3.5 | B |
| | | 7 equivalents | 4.5 | A |
| | | 10 equivalents | 6.1 | B |
| 203 | Compound (3) - IgG | 3 equivalents | 2.3 | A |
| | | 5 equivalents | 3.7 | A |
| | | 7 equivalents | 4.4 | A |
| | | 10 equivalents | 6.5 | A |
| 204 | Compound (4) - IgG | 3 equivalents | 2.2 | A |
| | | 5 equivalents | 3.4 | A |
| | | 7 equivalents | 4.3 | A |
| | | 10 equivalents | 6.0 | A |

(continued)

| No. | Labeled antibody | Molar equivalent ratio of compound to antibody | DOL | Signal fluorescence intensity (on membrane) |
|---|---|---|---|---|
| c21 | Comparative compound (1) - IgG | 3 equivalents | 2.2 | 1.0 (reference value) |
| | | 5 equivalents | 3.2 | G |
| | | 7 equivalents | 4.2 | G |
| | | 10 equivalents | 5.9 | G |
| c22 | Comparative compound (2) - IgG | 3 equivalents | 1.2 | G |
| | | 5 equivalents | 1.8 | G |
| | | 7 equivalents | 2.4 | F |
| | | 10 equivalents | 3.3 | E |
| c23 | Comparative compound (3) - IgG | 3 equivalents | 2.3 | F |
| | | 5 equivalents | 3.3 | F |
| | | 7 equivalents | 4.2 | G |
| | | 10 equivalents | 6.1 | G |

Regarding the signal fluorescence intensity of the labeled antibody on the membrane, shown in Table 3, the same results as those of the fluorescence intensity of the labeled antibody in the solution, shown in Table 2, were obtained.

<Example 2: Evaluation at excitation light wavelength of 633 nm>

Regarding each of the above-described compounds (5) to (8) and the comparative compound (5), the fluorescence intensity in the stained cells was evaluated. In addition, the fluorescence quantum yield and the light resistance of the compound and the labeled antibody were evaluated.

[4] Evaluation of fluorescence intensity of labeled antibody in stained cells

In the preparation of the solution of each labeled antibody in "[1] Evaluation of fluorescence labeling rate" described above, a solution of each labeled antibody was prepared in the same manner, except that the compound that was used for the labeling of the antibody and the molar equivalent ratio of the compound to 1 equivalent of the antibody were changed as shown in Table 4 below.

Cell staining was carried out as follows using the labeled antibody and the comparative labeled antibody synthesized as described above. The following characteristics were evaluated for the prepared stained cells. The results are summarized in Table 4.

[Preparation of stained cell sample]

HeLa cells [European Collection of Authenticated Cell Cultures, Inc.] were seeded on a 96-well plate [Thermo Fisher Scientific, Inc.] and cultured in an incubator for 20 hours in a D-MEM medium containing 10% fetal bovine serum, 1% Penicilline/streptomycin, and 1% MEM non-essential amino acids [all from FUJIFILM Wako Pure Chemical Corporation].

Subsequently, the culture medium was removed, methanol was used to carry out treatment at -20°C for 5 minutes, thereby carrying out fixation, washing was subsequently carried out with PBS [Thermo Fisher Scientific, Inc.], and a PBS solution containing, as a blocking agent and a membrane permeating agent, Triton X-100 (polyethylene glycol mono-p-isooctylphenyl ether) [Sigma-Aldrich Co., LLC] having a concentration of 0.2% and bovine serum albumin (BSA) [Biological Industries, Inc.] having a concentration of 2%, was added to the washed cells, which were subsequently allowed to stand for 1 hour. The blocking and the membrane permeating agent were removed, a diluted solution of an anti-α-Tubulin antibody [rabbit polyclonal, GeneTex Inc.] was added to the cells as a primary antibody, and the cells were allowed to stand at 4°C for 15 hours at a final antibody concentration of 1 μg/mL. After washing with PBST, 2 μg/mL of the aqueous solution of each labeled antibody prepared as described above was added to the cells as a secondary antibody, the cells were allowed to stand at room temperature for 1 hour while being shielded from light and

then washed again with PBST. Further, after washing with PBS, one drop of Prolong Gold [Thermo Fisher Scientific, Inc.] was added as an antifading agent to each well with a dropper to obtain each stained cell sample. Immediately after the preparation of the stained cell sample, the following evaluation of the fluorescence intensity was carried out.

[Evaluation of fluorescence intensity in stained cells]

Using a plate reader EnSight manufactured by PerkinElmer, Inc., the fluorescence intensity of the obtained stained cells was measured under the following conditions.
(Measurement condition) Well scan mode, excitation wavelength: 633 nm, fluorescence wavelength: 670 to 720 nm (data interval: 5 nm), band width: 8 nm, 100 times of integration, 3 × 3 points, measurement height: 3 mm, point distance: 1 mm)

The sum (integrated value) of the fluorescence intensities in the fluorescence wavelength range of 670 to 720 nm was defined as the fluorescence intensity of the sample. Using the integrated value of the fluorescence intensity of the comparative compound (5) - IgG having DOL of 3.7 in the fluorescence wavelength range of 670 nm to 720 nm as the reference value, the ratio to this reference value (the integrated value of the fluorescence intensity of the labeled antibody in the fluorescence wavelength range of 670 nm to 720 nm/the reference value) was calculated, and then, the evaluation was made based on the following evaluation standards. The results are summarized in Table 4.

- Evaluation standards for fluorescence intensity (integrated value) -

A: The ratio of fluorescence intensity to the reference value is 2.5 times or more.
B: The ratio of fluorescence intensity to the reference value is 2.0 times or more and less than 2.5 times.
C: The ratio of fluorescence intensity to the reference value is 1.2 times or more and less than 2.0 times.
D: The ratio of fluorescence intensity to the reference value is 1.0 times or more and less than 1.2 times.
E: The ratio of fluorescence intensity to the reference value is less than 1.0 times.

[Table 4]

| No. | Labeled antibody | Molar equivalent ratio of compound to antibody | DOL | Fluorescence intensity in stained cell |
|---|---|---|---|---|
| 105 | Compound (5) - IgG | 20 equivalents | 4.7 | B |
| 106 | Compound (6) - IgG | 40 equivalents | 6.2 | A |
| 107 | Compound (7) - IgG | 80 equivalents | 8.2 | A |
| 108 | Compound (8) - IgG | 20 equivalents | 4.8 | C |
| c15 | Comparative compound (5) - IgG | 5 equivalents | 5.5 | (Reference value) |

From the results in Table 4 above, the following points can be seen.

The compounds (5) to (8), which are the compounds according to the embodiment of the present invention, exhibit a fluorescence labeling rate (DOL) of 4.7 to 8.2, and the binding property to the antibody is at a sufficient level without any problem in practical use.

The comparative compound (5) is not the compound defined in the present invention in that $R^3$, instead of $R^{11}$ to $R^{13}$ in General Formula (1), has a carboxy group or a substituent capable of being bonded to a biological substance, and a structure represented by $-(CH_2-CH_2-O)_m-R^{21}$ described above is not contained.

All of the labeled antibodies using the compounds (5) to (8), which are the compounds according to the embodiment of the present invention, exhibit obviously high fluorescence as compared with the fluorescence intensity of the labeled antibody using the comparative compound (5) (Nos. 105 to 108 with respect to No. c15).

[5] Evaluation of fluorescence quantum yield

A PBS solution (pH 7.4) of the above-described compound or labeled antibody was evaluated according to the method described in the following reference document.

"A Guide to Recording Fluorescence Quantum Yields" (a HORIBA Scientific document, available from https://www.horiba.com/fileadmin/uploads/Scientific/Documents/Fluorescence/quantumyields trad.pdf)
Regarding the fluorescence quantum yield, a high evaluation rank is highly preferable since the fluorescence quantum yield is excellent.

- Evaluation standards for fluorescence quantum yield -

A: 0.5 or more
B: 0.4 or more and less than 0.5
C: 0.3 or more and less than 0.4
D: 0.2 or more and less than 0.3
E: Less than 0.2

[6] Evaluation of light resistance

The compound synthesized above or the labeled antibody was dissolved in a PBS solution (pH 7.4) so that the absorbance at the absorption peak wavelength was 0.095 to 0.105. In a state where this solution was exposed using a merry-go-round type light irradiator [xenon lamp UXL-500D-O manufactured by Ushio Inc., HA-50 filter, Y44 filter, exposure intensity: 22 mW/cm$^2$ (in terms of 500 nm)], the absorbance at the absorption peak wavelength of each compound or labeled antibody was measured over time with a spectrometer (Agilent 8453, manufactured by Hewlett-Packard Company). The absorbance at the absorption peak wavelength before light exposure was set to 100% and the light exposure time until the absorbance at this absorption peak wavelength decreases by 50% (the absorbance at the absorption peak wavelength reaches 50%) for the first time was measured. The results were evaluated based on the following evaluation standards.
A high evaluation rank is preferable since stability is kept for a long time.

- Evaluation standards for light resistance -

A: 40 hours or more
B: 30 hours or more and less than 40 hours
C: 20 hours or more and less than 30 hours
D: 10 hours or more and less than 20 hours
E: Less than 10 hours

[Table 5]

| No. | Fluorescent compound or fluorescent labeled antibody | DOL | Fluorescence quantum yield | Light resistance |
|---|---|---|---|---|
| 301 | Compound (5-D) | - | B | A |
| 302 | Compound (6-B) | - | A | A |
| 303 | Compound (7-B) | - | B | A |
| 304 | Compound (8-B) | - | D | B |
| c31 | Alexa Fluor 647 | - | C | E |
| 305 | Compound (5) - IgG | 4.7 | B | B |
| 306 | Compound (6) - IgG | 6.2 | C | C |
| 307 | Compound (7) - IgG | 8.2 | B | A |
| 308 | Compound (8) - IgG | 4.8 | C | D |
| c32 | Comparative compound (5) - IgG | 5.5 | E | E |
| (Note in table) | | | | |

Alexa Fluor 647 is as described above, and the comparative compound (5) - IgG means a labeled antibody prepared by using an NHS ester form of Alexa Fluor 647.

From the results of Tables 4 and 5, it can be seen that the compound of the second preferred form according to the present invention, which has an excitation absorption wavelength in a range of 620 to 700 nm, and the labeled antibody thereof exhibit an excellent fluorescence quantum yield in addition to exhibiting an excellent fluorescence intensity of the obtained labeled biological substance, and moreover, they also have an excellent light resistance as compared with Alexa Fluor 647, which is a commercially available fluorescent compound, and a labeled antibody thereof.

<Example 3: Evaluation at excitation light wavelength of 685 nm>

Regarding each of the above-described compounds (9) to (13) and the comparative compound (4), the solution fluorescence intensity and the fluorescence intensity on the membrane were evaluated.

[7] Evaluation of solution fluorescence intensity

A solution of each labeled antibody was prepared in the same manner, except that in the preparation of the solution of each labeled antibody in "[1] Evaluation of fluorescence labeling rate" described above, the compound that was used for the labeling of the antibody and the molar equivalent ratio of the compound to 1 equivalent of the antibody were changed as shown in Table 6 below. A solution of the obtained each labeled antibody was adjusted to a protein concentration of 0.005 mg/mL, and the integrated value of the fluorescence intensity in the fluorescence wavelength range of 710 to 730 nm was calculated by using a spectroscopic fluorescence intensity meter (product name: RF-6000, manufactured by Shimadzu Corporation) with excitation light of 685 nm and unified the exposure conditions. Using the integrated value of the fluorescence intensity of the comparative compound (4) - IgG having DOL of 3.4 (10 equivalents of dye had been added) in the fluorescence wavelength range of 710 nm to 730 nm as the reference value, the ratio to this reference value (the integrated value of the fluorescence intensity of the labeled antibody in the fluorescence wavelength range of 710 nm to 730 nm/the reference value) was calculated, and then, the evaluation was made based on the following evaluation standards. The results are summarized in Table 6.

- Evaluation standards for fluorescence intensity (integrated value) -

A: The ratio of fluorescence intensity to the reference value is 2.0 times or more.
B: The ratio of fluorescence intensity to the reference value is 1.7 times or more and less than 2.0 times.
C: The ratio of fluorescence intensity to the reference value is 1.4 times or more and less than 1.7 times.
D: The ratio of fluorescence intensity to the reference value is 1.2 times or more and less than 1.4 times.
E: The ratio of fluorescence intensity to the reference value is 1.1 times or more and less than 1.2 times.
F: The ratio of fluorescence intensity to the reference value is 0.9 times or more and less than 1.1 times.
G: The ratio of fluorescence intensity to the reference value is less than 0.9 times.

[Table 6]

| No. | Labeled antibody | Molar equivalent ratio of compound to antibody | DOL | Fluorescence intensity (solution) |
|---|---|---|---|---|
| 109 | Compound (9) - IgG | 15 equivalents | 4.4 | E |
| | | 25 equivalents | 6.4 | D |
| | | 40 equivalents | 7.8 | A |
| 110 | Compound (10) - IgG | 10 equivalents | 3.6 | D |
| | | 15 equivalents | 4.9 | C |
| | | 25 equivalents | 6.3 | B |
| 111 | Compound (11) - IgG | 25 equivalents | 3.9 | C |
| | | 40 equivalents | 5.9 | A |
| | | 60 equivalents | 7.4 | A |

(continued)

| No. | Labeled antibody | Molar equivalent ratio of compound to antibody | DOL | Fluorescence intensity (solution) |
|---|---|---|---|---|
| 112 | Compound (12) - IgG | 20 equivalents | 3.7 | B |
| | | 40 equivalents | 5.2 | A |
| | | 60 equivalents | 6.7 | A |
| 113 | Compound (13) - IgG | 15 equivalents | 2.1 | E |
| | | 60 equivalents | 3.6 | D |
| cl4 | Comparative compound (4) - IgG | 7 equivalents | 2.0 | F |
| | | 10 equivalents | 3.4 | 1.0 (reference value) |
| | | 15 equivalents | 6.2 | F |

From the results in Table 6 above, the following points can be seen.

The compounds (9) to (13), which are the compounds according to the embodiment of the present invention, exhibit a fluorescence labeling rate (DOL) of 2.1 to 7.8, and the binding property to the antibody is at a sufficient level without any problem in practical use.

The comparative compound (4) is not a compound having the structure defined in the present invention in that $L^1$, instead of $R^{11}$ to $R^{13}$ in General Formula (1), has a carboxy group or a substituent capable of being bonded to a biological substance. In the solution, the fluorescence intensity of the labeled antibody using this comparative compound (4) is low at any of the DOL values (No. c14).

On the other hand, all of the labeled antibodies using the compounds (9) to (13), which are the compounds according to the embodiment of the present invention, exhibit obviously high fluorescence as compared with the fluorescence intensity of the labeled antibody using the comparative compound (4) (Nos. 109 to 113 with respect to No. c14).

In addition, it can be seen that among the labeled antibodies of the compounds (9) to (13) according to the embodiment of the present invention in which $R^{13}$ having a substituent capable of binding to a biological substance is a group represented by General Formula (IV), the compounds (10) to (13) according to the embodiment of the present invention which have at least one substituent in $R^{39}$ or $R^{43}$ in General Formula (IV) exhibit more excellent fluorescence intensity, and the compounds (11) to (13) according to the embodiment of the present invention which have substituents in both $R^{39}$ and $R^{43}$ exhibit a more excellent fluorescence intensity.

[8] Evaluation of fluorescence intensity in Western blotting

Transferrin (manufactured by Merck KGaA) was diluted with a Fluorescent Compatible Sample Buffer (4X, non-reducing) (manufactured by Thermo Fisher Scientific, Inc.) to 1 ng/uL, 0.3 ng/uL, or 0.1 ng/μL, followed by heating treatment at 95°C for 5 minutes. The above-described transferrin sample and a PageRuler Prestained NIR Protein Ladder (manufactured by Thermo Fisher Scientific, Inc.) were loaded on Novex 4-20% Tris-Glycine Mini Gels (manufactured by Thermo Fisher Scientific, Inc.), and then electrophoresis was carried out at a constant voltage of 225 V for 45 minutes. The gel after electrophoresis and a nitrocellulose membrane (manufactured by Cytiva) were superimposed, protein transfer was carried out at a constant voltage of 20 V for 1 hour, and then the membrane was immersed in a Western Blot Blocking Buffer (Fish Gelatin) (manufactured by TAKARA Bio Inc.) and allowed to stand at 4°C for 12 hours. After washing with a TBS-T buffer, the membrane was immersed in a solution containing a primary antibody for transferrin (manufactured by Dako Corporation) (diluted 5,000 times), shaking was carried out for 1 hour, and the membrane was washed with the TBS-T buffer. A solution of the comparative compound (4) - IgG (diluted 25,000 times) was adjusted, and the membrane was immersed and shaken for 1 hour in a light-shielded state, followed by washing with TBS-T. The membrane was shielded from light for 1 hour in a constant-temperature tank at 40°C and then dried. Imaging was carried out using an Amersham Typhoon scanner (manufactured by Cytiva), and with excitation light of 685 nm under the uniform measurement conditions, the fluorescence intensity of a fraction of 3.24 $mm^2$ in a fluorescence wavelength range of 710 to 730 nm was measured and used as a signal fluorescence intensity. The fluorescence intensity of the labeled antibodies of other compounds was measured in the same manner as described above.

Using the integrated value of the signal fluorescence intensity of the comparative compound (4) - IgG having DOL of 3.4 in the fluorescence wavelength range of 710 nm to 730 nm as the reference value, the ratio to this reference value (the integrated value of the signal fluorescence intensity of the labeled antibody in the fluorescence wavelength range of 710 nm to 730 nm/the reference value) was calculated, and then, the evaluation was made based on the

following evaluation standards. The results are summarized in Table 7.

- Evaluation standards for fluorescence intensity (integrated value) -

A: The ratio of signal fluorescence intensity to the reference value is 3.0 times or more.
B: The ratio of signal fluorescence intensity to the reference value is 2.5 times or more and less than 3.0 times.
C: The ratio of signal fluorescence intensity to the reference value is 2.0 times or more and less than 2.5 times.
D: The ratio of signal fluorescence intensity to the reference value is 1.5 times or more and less than 2.0 times.
E: The ratio of signal fluorescence intensity to the reference value is 1.1 times or more and less than 1.5 times.
F: The ratio of signal fluorescence intensity to the reference value is 0.9 times or more and less than 1.1 times.
G: The ratio of signal fluorescence intensity to the reference value is less than 0.9 times.

[Table 7]

| No. | Labeled antibody | Molar equivalent ratio of compound to antibody | DOL | Fluorescence intensity in western blotting |
|---|---|---|---|---|
| 209 | Compound (9) - IgG | 15 equivalents | 4.4 | C |
| | | 25 equivalents | 6.4 | C |
| | | 40 equivalents | 7.8 | D |
| 210 | Compound (11) - IgG | 25 equivalents | 3.9 | B |
| | | 40 equivalents | 5.9 | B |
| | | 60 equivalents | 7.4 | B |
| 211 | Compound (12) - IgG | 20 equivalents | 3.7 | B |
| | | 40 equivalents | 5.2 | A |
| | | 60 equivalents | 6.7 | A |
| 212 | Compound (13) - IgG | 15 equivalents | 2.1 | D |
| | | 60 equivalents | 3.6 | C |
| c24 | Comparative compound (4) - IgG | 10 equivalents | 3.4 | 1.0 (reference value) |

Regarding the fluorescence intensity of the labeled antibody shown in Table 7 in Western blotting, the same results as those of the fluorescence intensity of the labeled antibody in the solution, shown in Table 7, were obtained.

<Example 4>

The light resistance of each of the above compounds (19) and (20) and a commercially available product (Alexa Fluor 750) was evaluated.

[9] Evaluation of light resistance

The compound synthesized above or a commercially available product was dissolved in a PBS solution (pH 7.4) so that the absorbance at the absorption peak wavelength was 0.095 to 0.105. In a state where this solution was exposed using a merry-go-round type light irradiator [xenon lamp UXL-500D-O manufactured by Ushio Inc., HA-50 filter, Y44 filter, exposure intensity: 22 mW/$cm^2$ (in terms of 500 nm)], the absorbance at the absorption peak wavelength of each compound or a commercially available product was measured over time with a spectrometer (Agilent 8453, manufactured by Hewlett-Packard Company). The absorbance at the absorption peak wavelength before light exposure was set to 100% and the light exposure time until the absorbance at this absorption peak wavelength decreases by 50% (the absorbance at the absorption peak wavelength reaches 50%) for the first time was measured. The results were evaluated based on the following evaluation standards.

A high evaluation rank is preferable since stability is kept for a long time.

- Evaluation standards for light resistance -

A: 9 hours or more
B: 7 hours or more and less than 9 hours
C: 5 hours or more and less than 7 hours
D: 3 hours or more and less than 5 hours
E: Less than 3 hours

[Table 8]

| No. | Fluorescent compound | Light resistance |
|---|---|---|
| 119 | Compound (19) | A |
| 120 | Compound (20) | A |
| c15 | Alexa Fluor 750 | E |
| (Note in table) | | |

Alexa Fluor 750: Alexa Fluor 750 (product name, manufactured by Thermo Fisher Scientific, Inc.). This is a fluorescent dye with $\lambda_{ex}$ = 749 nm and $\lambda_{em}$ = 775 nm and is known as a dye having excellent light resistance.
From the results in Table 8 above, it can be seen that the compound of the first preferred form according to the present invention, which has an excitation absorption wavelength at 740 to 830 nm, is excellent in light resistance as compared with Alexa Fluor 750, which is a commercially available fluorescent compound.
As described above, the labeled biological substance using the compound according to the embodiment of the present invention has an excellent fluorescence intensity in at least any form to be used in a solution, on a membrane, on a stained cell, or in Western blotting.
The present invention has been described together with the embodiments of the present invention. However, the inventors of the present invention do not intend to limit the present invention in any part of the details of the description unless otherwise specified, and it is conceived that the present invention should be broadly construed without departing from the spirit and scope of the invention shown in the attached "WHAT IS CLAIMED IS".
This application claims priority based on JP2021-101305 filed in Japan on June 18, 2021, and JP2022-044427 filed in Japan on March 18, 2022, which are incorporated herein by reference as a part of the description of the present specification.

## Claims

1. A compound represented by General Formula (1),

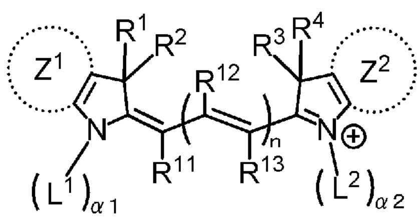

General Formula (1)

in the formula, $R^1$ to $R^4$ represent an alkyl group, an aryl group, a heteroaryl group, or $-(CH_2-CH_2-O)_m-R^{21}$, where m is 1 to 10, and $R^{21}$ represents an alkyl group,
$R^{11}$ to $R^{13}$ represent a hydrogen atom, an alkyl group, an aryl group, a heteroaryl group, an alkoxy group, an aryloxy group, an alkylthio group, an arylthio group, an amino group, an amide group, or a halogen atom, where adjacent groups may be bonded to each other to form a 5- or 6-membered ring, provide that at least one of $R^{11}$, $R^{12}$, or $R^{13}$ contains a carboxy group or a substituent capable of being bonded to a biological substance,

n is an integer of 1 to 3,

$L^1$ and $L^2$ represent an alkyl group or $-(CH_2-CH_2-O)_m-R^{21}$, where $R^{21}$ and m respectively have the same meanings as $R^{21}$ and m described above, and $L^1$ and $L^2$ may be bonded to each other to form a ring,

$\alpha 1$ and $\alpha 2$ are 0 or 1,

a ring $Z^1$ and a ring $Z^2$ represent a 6-membered ring formed of a ring-constituting atom selected from a carbon atom and a nitrogen atom, may have a substituent, and may form a fused ring,

in a case where the ring $Z^1$ and the ring $Z^2$ form a 6-membered ring having a ring-constituting nitrogen atom, the ring-constituting nitrogen atom may be substituted with a substituent, and

the compound represented by Formula (1) has at least one structure represented by $-(CH_2-CH_2-O)_m-R^{21}$, where $R^{21}$ and m respectively have the same meanings as $R^{21}$ and m described above,

provided that the compound represented by Formula (1) is a neutral compound.

**2.** The compound according to claim 1,

wherein the compound is represented by any one of General Formulae (2-1) to (2-4),

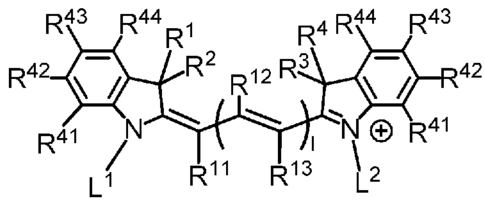

General Formula (2-1)

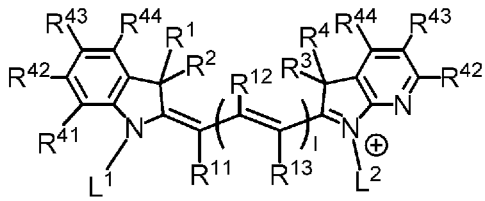

General Formula (2-2)

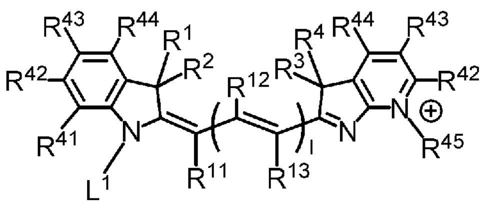

General Formula (2-3)

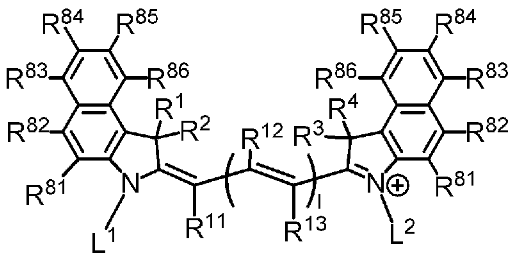

General Formula (2-4)

in the formulae, $R^{45}$ represent an alkyl group or $-(CH_2-CH_2-O)_m-R^{21}$,

$R^{41}$ to $R^{44}$ and $R^{81}$ to $R^{86}$ represent a hydrogen atom, an alkyl group, an alkoxy group, an aryl group, a sulfo group, a sulfonamide group, a nitro group, a carboxy group, an amide group, or a halogen atom, where adjacent groups may be bonded to each other to form a fused ring,

$R^1$ to $R^4$, $R^{11}$ to $R^{13}$, $L^1$, $L^2$, and m respectively have the same meanings as $R^1$ to $R^4$, $R^{11}$ to $R^{13}$, $L^1$, $L^2$, and m described above,

at least one of $R^1$ to $R^4$, $L^1$, $L^2$, or $R^{45}$ is $-(CH_2-CH_2-O)_m-R^{21}$, where $R^{21}$ and m respectively have the same meanings as $R^{21}$ and m described above, and

l represents an integer of 2 or 3,

provided that the compound represented by any one of Formulae (2-1) to (2-4) is a neutral compound.

**3.** The compound according to claim 1,
wherein at least one of $R^1$ or $R^2$ described above and at least one of $R^3$ or $R^4$ described above includes a structure represented by $-(CH_2-CH_2-O)_m-$, where m is 1 to 10.

**4.** The compound according to claim 3,
wherein the compound includes four structures, each of which is represented by $-(CH_2-CH_2-O)_m-$, where m is 1 to 10.

**5.** The compound according to claim 1,
wherein at least one of $L^1$ or $L^2$ described above is an alkyl group having, as a substituent, at least one of an alkoxy group, a carboxy group, a sulfo group, or a phosphono group.

**6.** The compound according to claim 5,
wherein $L^1$ and $L^2$ described above are an alkyl group having, as a substituent, at least one of an alkoxy group, a carboxy group, a sulfo group, or a phosphono group.

**7.** The compound according to claim 1,

wherein one among $R^{11}$ to $R^{13}$ described above, which has a substituent having a carboxy group or a substituent capable of being bonded to a biological substance, is a substituent represented by any one of General Formulae (I) to (V),

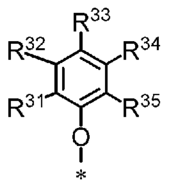

General Formula (I)

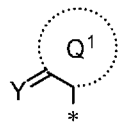

General Formula (II)

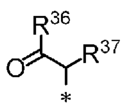

General Formula (III)

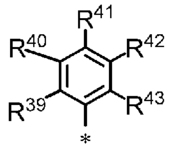

General Formula (IV)

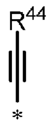

General Formula (V)

in the formulae, Y represents an oxygen atom or $=NR^{38}$, and a ring $Q^1$ represents a heterocyclic ring or hydrocarbon ring which is 5-membered or 6-membered,
$R^{31}$ to $R^{44}$ represent a hydrogen atom, an alkyl group, an aryl group, a heteroaryl group, an alkoxy group, an aryloxy group, an alkylthio group, an arylthio group, an amino group, an amide group, a halogen atom, a sulfo group, a nitro group, a cyano group, a carboxy group, or a substituent capable of being bonded to a biological substance,
provided that a group represented by any one of General Formulae (I) to (V) has at least one carboxy group or one substituent capable of being bonded to a biological substance, and
* represents a bonding site.

**8.** The compound according to claim 7,

wherein the substituent represented by General Formula (II) is a substituent represented by any one of Formulae (E-1) to (E-8),

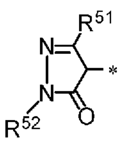

(E-1)

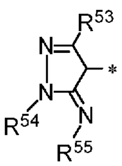

(E-2)

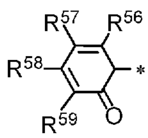

(E-3)

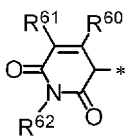

(E-4)

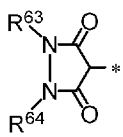

(E-5)

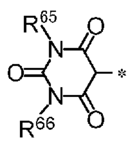

(E-6)

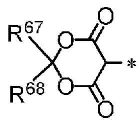

(E-7)

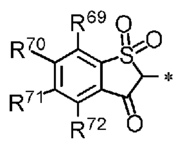

(E-8)

in the formulae, $R^{51}$ to $R^{72}$ represent a hydrogen atom, an alkyl group, an aryl group, a heteroaryl group, an alkoxy group, an aryloxy group, an alkylthio group, an arylthio group, an amino group, a halogen atom, a sulfo group, a nitro group, a cyano group, a carboxy group, or a substituent capable of being bonded to a biological substance,
provided that a substituent represented by any one of General Formulae (E-1) to (E-8) has at least one carboxy group or one substituent capable of being bonded to a biological substance, and
* represents a bonding site.

**9.** The compound according to claim 7,
wherein one among $R^{11}$ to $R^{13}$ described above, which has a substituent having a carboxy group or a substituent capable of being bonded to a biological substance, is a substituent represented by General Formula (IV).

**10.** The compound according to claim 9,
wherein at least one of $R^{39}$, ..., or $R^{43}$ described above is a carboxy group, an alkyloxy group having a carboxy group, an alkylcarbamoyl group having a carboxy group, or a poly(oxyalkylene) carbamoyl group having a carboxy group.

**11.** A labeled biological substance that is obtained by bonding the compound according to any one of claims 1 to 10 to a biological substance.

**12.** The labeled biological substance according to claim 11,
wherein the biological substance is any one of a protein, an amino acid, a nucleic acid, a sugar chain, or a phospholipid.

## INTERNATIONAL SEARCH REPORT

International application No.
**PCT/JP2022/023976**

**A. CLASSIFICATION OF SUBJECT MATTER**

***C09K 11/06***(2006.01)i; ***C09B 23/01***(2006.01)i; ***C09B 23/14***(2006.01)i; ***G01N 33/533***(2006.01)i; ***G01N 33/58***(2006.01)i; ***G01N 21/64***(2006.01)i
FI: C09B23/01 CSP; G01N33/58 A; G01N33/58 Z; G01N21/64 F; C09B23/14; C09K11/06; C09K11/06 645; G01N33/533

According to International Patent Classification (IPC) or to both national classification and IPC

**B. FIELDS SEARCHED**

Minimum documentation searched (classification system followed by classification symbols)

C09K11/06; C09B23/01; C09B23/14; G01N33/533; G01N33/58; G01N21/64

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | US 2016/0168383 A1 (HERMANSON, Greg) 16 June 2016 (2016-06-16) claims, paragraphs [0512], [0528], [0802] | 1-8, 11-12 |
| Y | claims, paragraphs [0512], [0528], [0802] | 9-10 |
| X | JP 2021-513968 A (THE UNITED STATES OF AMERICA, AS REPRESENTED BY THE SECRETARY, DEPT. OF HEALTH AND HUMAN SERVICES) 03 June 2021 (2021-06-03) claims, paragraph [0150], etc. | 1-2, 11-12 |
| X | JP 2006-513293 A (SCHERING AG.) 20 April 2006 (2006-04-20) claims, claim 20, etc. | 1-2, 5-8, 11-12 |
| X | JP 2012-517436 A (BETH ISRAEL DEACONESS MEDICAL CENTER, INC.) 02 August 2012 (2012-08-02) claims, fig. 1 | 1-2, 7-8, 11-12 |
| Y | JP 2012-524153 A (LI-COR, INC.) 11 October 2012 (2012-10-11) claims | 9-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2022** | **06 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/023976**

| Patent document cited in search report | Publication date (day/month/year) | Patent family member(s) | Publication date (day/month/year) |
|---|---|---|---|
| US 2016/0168383 A1 | 16 June 2016 | US 2014/0255312 A1 | |
| JP 2021-513968 A | 03 June 2021 | US 2021/0017132 A1<br>claims, page 19<br>WO 2019/161159 A1<br>EP 3752204 A1<br>KR 10-2020-0122338 A | |
| JP 2006-513293 A | 20 April 2006 | US 2004/0260072 A1<br>claim 20<br>WO 2004/065491 A1<br>EP 1585791 A1<br>KR 10-2005-0103907 A<br>CN 1742057 A | |
| JP 2012-517436 A | 02 August 2012 | US 2012/0028291 A1<br>fig. 1<br>WO 2010/091243 A1<br>EP 2393420 A1 | |
| JP 2012-524153 A | 11 October 2012 | US 2010/0323389 A1<br>claims<br>WO 2010/121163 A2<br>EP 2419478 A1<br>CN 102459469 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20210093737 A
- WO 2009078970 A
- WO 2002026891 A
- JP 2021101305 A
- JP 2022044427 A


### Non-patent literature cited in the description


- **GREG T. HERMANSON.** Bioconjugate Techniques
- **LUCAS C. D. DE REZENDE ; FLAVIO DA SILVA EMERY.** A Review of the Synthetic Strategies for the Development of BODIPY Dyes for Conjugation with Proteins. *Orbital: The Electronic Journal of Chemistry,* 2013, vol. 5 (1), 62-83